# EUROPEAN PATENT APPLICATION

(11) **EP 2 223 937 A1**
(43) Date of publication of application: **01.09.2010**
(21) Application number: 09290146.1
(22) Date of filing: 27.02.2009
(51) Int. Cl.: C07K 14/445, C07K 19/00, A61K 39/015

(54) **Polypeptides for the prevention or treatment of malaria**

(71) Applicant: Institut Pasteur, 75724 Paris Cédex 15 (FR)
(72) Inventor: Druilhe, Pierre, 75015 Paris (FR); Prieur, Eric, 92170 Vanves (FR); Bang, Gilles, 75016 Paris (FR)
(74) Representative: Desaix, Anne

(57) **Abstract**

The present invention relates to the protection against malaria and to polypeptides suitable in this respect.

More particularly, the invention relates to polypeptides, especially chimeric ones, which are suitable for the prevention or treatment of malaria. Said polypeptides are derived from particular motifs of the C-terminal region of *Plasmodium* proteins choosen among MSP3-1, MSP3-2, MSP3-3, MSP3-4, MSP3-7 and/or MSP3-8.

The invention also pertains to immunogenic compositions and vaccines against malaria, comprising as an immunogen a polypeptide of the invention, to a polynucleotide coding for a polypeptide of the invention as well as a cloning and/or expression vector comprising said polynucleotide, and their use in a medicament or a nucleic acid vaccine against *Plasmodium.*

SEQUENCE LISTING

<110>  INSTITUT PASTEUR

<120>  POLYPEPTIDES FOR THE PREVENTION OR TREATMENT OF MALARIA

<130>  B08068 - AD/CAL

<140>  EP Unknown
<141>  2009-02-27

<160>  71

<170>  PatentIn version 3.3

<210>  1
<211>  25
<212>  PRT
<213>  Plasmodium falciparum


<220>
<221>  MISC_FEATURE
<222>  (1)..(25)
<223>  Motif a of MSP3.1 (amino acids 167-191)

<400>  1

Tyr Glu Lys Ala Lys Asn Ala Tyr Gln Lys Ala Asn Gln Ala Val Leu
1               5                   10                  15


Lys Ala Lys Glu Ala Ser Ser Tyr Asp
            20                  25


<210>  2
<211>  27
<212>  PRT
<213>  Plasmodium falciparum


<220>
<221>  MISC_FEATURE
<222>  (1)..(27)
<223>  Motif b of MSP3.1 (amino acids 184-210)

<400>  2

Ala Lys Glu Ala Ser Ser Tyr Asp Tyr Ile Leu Gly Trp Glu Phe Gly
1               5                   10                  15


Gly Gly Val Pro Glu His Lys Lys Glu Glu Asn
            20                  25


<210>  3
<211>  28
<212>  PRT
<213>  Plasmodium falciparum


<220>
<221>  MISC_FEATURE
<222>  (1)..(28)
<223>  Motif c of MSP3.1 (amino acids 203-230)

<400>  3

Pro Glu His Lys Lys Glu Glu Asn Met Leu Ser His Leu Tyr Val Ser
1               5                   10                  15

Ser Lys Asp Lys Glu Asn Ile Ser Lys Glu Asn Asp
            20                  25

<210>  4
<211>  41
<212>  PRT
<213>  Plasmodium falciparum

<220>
<221>  MISC_FEATURE
<222>  (1)..(41)
<223>  Motif d of MSP3.1 (amino acids 211-252)

<400>  4

Met Leu Ser His Leu Tyr Val Ser Ser Lys Asp Lys Glu Asn Ile Ser
1               5                   10                  15

Lys Glu Asn Asp Asp Val Leu Asp Glu Lys Glu Glu Glu Ala Glu Glu
            20                  25                  30

Thr Glu Glu Glu Glu Leu Glu Glu Lys
        35                  40

<210>  5
<211>  34
<212>  PRT
<213>  Plasmodium falciparum

<220>
<221>  MISC_FEATURE
<222>  (1)..(34)
<223>  Motif e of MSP3.1 (amino acids 275-307)

<400>  5

Glu Glu Glu Asn Asp Lys Lys Lys Glu Gln Glu Lys Glu Gln Ser Asn
1               5                   10                  15

Glu Asn Asn Asp Gln Lys Lys Asp Met Glu Ala Gln Asn Leu Ile Ser
            20                  25                  30

Lys Asn

<210>  6
<211>  53
<212>  PRT
<213>  Plasmodium falciparum

<220>
<221>  MISC_FEATURE

<222>   (1)..(53)
<223>   Motif f of MSP3.1 (amino acids 302-354)

<400>   6

Asn Leu Ile Ser Lys Asn Gln Asn Asn Asn Glu Lys Asn Val Lys Glu
1               5                   10                  15

Ala Ala Glu Ser Ile Met Lys Thr Leu Ala Gly Leu Ile Lys Gly Asn
            20                  25                  30

Asn Gln Ile Asp Ser Thr Leu Lys Asp Leu Val Glu Glu Leu Ser Lys
        35                  40                  45

Tyr Phe Lys Asn His
    50

<210>   7
<211>   21
<212>   PRT
<213>   Plasmodium falciparum

<220>
<221>   MISC_FEATURE
<222>   (1)..(21)
<223>   Motif a of MSP3.2 (amino acids 161-182)

<400>   7

Ser Glu Thr Asn Lys Asn Pro Thr Ser His Ser Asn Ser Thr Thr Thr
1               5                   10                  15

Ser Leu Asn Asn Asn
            20

<210>   8
<211>   27
<212>   PRT
<213>   Plasmodium falciparum

<220>
<221>   MISC_FEATURE
<222>   (1)..(27)
<223>   Motif b of MSP3.2 (amino acids 179-204)

<400>   8

Leu Asn Asn Asn Ile Leu Gly Trp Glu Phe Gly Gly Gly Ala Pro Gln
1               5                   10                  15

Asn Gly Ala Ala Glu Asp Lys Lys Thr Glu Tyr
            20                  25

<210>   9
<211>   33
<212>   PRT
<213>   Plasmodium falciparum

<220>
<221>  MISC_FEATURE
<222>  (1)..(33)
<223>  Motif c of MSP3.2 (amino acids 192-224)

<400>  9

Pro Gln Asn Gly Ala Ala Glu Asp Lys Lys Thr Glu Tyr Leu Leu Glu
1               5                   10                  15

Gln Ile Lys Ile Pro Ser Trp Asp Arg Asn Asn Ile Pro Asp Glu Asn
            20                  25                  30

Glu

<210>  10
<211>  53
<212>  PRT
<213>  Plasmodium falciparum

<220>
<221>  MISC_FEATURE
<222>  (1)..(53)
<223>  Motif d of MSP3.2 (amino acids 205-257)

<400>  10

Leu Leu Glu Gln Ile Lys Ile Pro Ser Trp Asp Arg Asn Asn Ile Pro
1               5                   10                  15

Asp Glu Asn Glu Gln Val Ile Glu Asp Pro Gln Glu Asp Asn Lys Asp
            20                  25                  30

Glu Asp Glu Asp Glu Glu Thr Glu Thr Glu Asn Leu Glu Thr Glu Asp
        35                  40                  45

Asp Asn Asn Glu Glu
    50

<210>  11
<211>  42
<212>  PRT
<213>  Plasmodium falciparum

<220>
<221>  MISC_FEATURE
<222>  (1)..(42)
<223>  Motif e of MSP3.2 (amino acids 282-326)

<400>  11

Glu Glu Glu Lys Lys Glu Glu Lys Lys Glu Glu Lys Lys Pro Asp Asn
1               5                   10                  15

Glu Ile Thr Asn Glu Val Lys Glu Glu Gln Lys Tyr Ser Ser Pro Ser
            20                  25                  30

Asp Ile Asn Ala Gln Asn Leu Ile Ser Asn
        35                  40

<210>  12
<211>  52
<212>  PRT
<213>  Plasmodium falciparum

<220>
<221>  MISC_FEATURE
<222>  (1)..(52)
<223>  Motif f of MSP3.2 (amino acids 320-371)

<400>  12

Asn Leu Ile Ser Asn Lys Asn Lys Lys Asn Asp Glu Thr Lys Lys Thr
1               5                   10                  15

Ala Glu Asn Ile Val Lys Thr Leu Val Gly Leu Phe Asn Glu Lys Asn
            20                  25                  30

Glu Ile Asp Ser Thr Ile Asn Asn Leu Val Gln Glu Met Ile His Leu
        35                  40                  45

Phe Ser Asn Asn
    50

<210>  13
<211>  24
<212>  PRT
<213>  Plasmodium falciparum

<220>
<221>  MISC_FEATURE
<222>  (1)..(24)
<223>  Motif a of MSP3.3 (amino acids 228-252)

<400>  13

Tyr Glu Lys Lys Asn Glu Asn Lys Asn Val Ser Asn Val Asp Ser Lys
1               5                   10                  15

Thr Lys Ser Asn Glu Lys Gly Arg
            20

<210>  14
<211>  26
<212>  PRT
<213>  Plasmodium falciparum

<220>
<221>  MISC_FEATURE
<222>  (1)..(26)
<223>  Motif b of MSP3.3 (amino acids 246-272)

<400>  14

Ser Asn Glu Lys Gly Arg Pro Pro Thr Tyr Ser Pro Ile Leu Asp Asp
1               5                   10                  15

Gly Ile Glu Phe Ser Gly Gly Leu Tyr Phe
            20                  25

<210>  15
<211>  29
<212>  PRT
<213>  Plasmodium falciparum

<220>
<221>  MISC_FEATURE
<222>  (1)..(29)
<223>  Motif c of MSP3.3 (amino acids 262-291)

<400>  15

Gly Ile Glu Phe Ser Gly Gly Leu Tyr Phe Asn Glu Lys Lys Ser Thr
1               5                   10                  15

Glu Glu Asn Lys Gln Lys Asn Val Leu Glu Ser Val Asn
            20                  25

<210>  16
<211>  32
<212>  PRT
<213>  Plasmodium falciparum

<220>
<221>  MISC_FEATURE
<222>  (1)..(32)
<223>  Motif d of MSP3.3 (amino acids 276-308)

<400>  16

Ser Thr Glu Glu Asn Lys Gln Lys Asn Val Leu Glu Ser Val Asn Leu
1               5                   10                  15

Thr Ser Trp Asp Lys Glu Asp Ile Val Lys Glu Asn Glu Asp Val Lys
            20                  25                  30

<210>  17
<211>  33
<212>  PRT
<213>  Plasmodium falciparum

<220>
<221>  MISC_FEATURE
<222>  (1)..(33)
<223>  Motif e of MSP3.3 (amino acids 352-385)

<400>  17

Asp Thr Ser Asp Leu Glu Lys Lys Asn Ile Pro Asp Leu Ser Asn Asp
1               5                   10                  15

Asn Asn Tyr Tyr Ser Leu Ile Tyr Lys Asn Tyr Lys Asp Asn Asp Lys
            20                  25                  30

Ser

<210>  18
<211>  52
<212>  PRT
<213>  Plasmodium falciparum

<220>
<221>  MISC_FEATURE
<222>  (1)..(52)
<223>  Motif f of MSP3.3 (amino acids 372-424)

<400>  18

Ser Leu Ile Tyr Lys Asn Tyr Lys Asp Asn Asp Lys Ser Glu Lys Thr
1               5                   10                  15

Ala Gln Thr Leu Ile Thr Ala Leu Ile Ser Leu Leu Asn Gly Lys Asn
            20                  25                  30

Glu Leu Asp Ala Thr Ile Arg Arg Leu Lys His Arg Phe Met Glu Phe
        35                  40                  45

Phe Thr Tyr Asn
    50

<210>  19
<211>  25
<212>  PRT
<213>  Plasmodium falciparum

<220>
<221>  MISC_FEATURE
<222>  (1)..(25)
<223>  Motif a of MSP3.4 (amino acids 508-533)

<400>  19

Asp Asn Val Asn Ser Val Thr Gln Arg Gly Asn Asn Asn Tyr Asn Asn
1               5                   10                  15

Asn Leu Glu Arg Gly Leu Gly Ser Gly
            20                  25

<210>  20
<211>  27
<212>  PRT
<213>  Plasmodium falciparum

<220>
<221>  MISC_FEATURE
<222>  (1)..(27)
<223>  Motif b of MSP3.4 (amino acids 525-552)

<400>  20

Leu Glu Arg Gly Leu Gly Ser Gly Ala Leu Pro Gly Thr Asn Ile Ile
1               5                   10                  15

Thr Glu Glu Lys Tyr Ser Leu Glu Leu Ile Lys
            20                  25

<210>  21
<211>  28
<212>  PRT
<213>  Plasmodium falciparum

<220>
<221>  MISC_FEATURE
<222>  (1)..(28)
<223>  Motif c of MSP3.4 (amino acids 544-572)

<400>  21

Lys Tyr Ser Leu Glu Leu Ile Lys Leu Thr Ser Lys Asp Glu Glu Asp
1               5                   10                  15

Ile Ile Lys His Asn Glu Asp Val Arg Glu Glu Ile
            20                  25

<210>  22
<211>  28
<212>  PRT
<213>  Plasmodium falciparum

<220>
<221>  MISC_FEATURE
<222>  (1)..(28)
<223>  Motif d of MSP3.4 (amino acids 552-580)

<400>  22

Leu Thr Ser Lys Asp Glu Glu Asp Ile Ile Lys His Asn Glu Asp Val
1               5                   10                  15

Arg Glu Glu Ile Glu Glu Gln Gln Glu Asp Ile Glu
            20                  25

<210>  23
<211>  34
<212>  PRT
<213>  Plasmodium falciparum

<220>
<221>  MISC_FEATURE
<222>  (1)..(34)
<223>  Motif e of MSP3.4 (amino acids 605-639)

<400>  23

Glu Glu Asn Lys Glu Lys Glu Leu Ser Asn Gln Gln Gln Ser Glu Lys
1               5                   10                  15

Lys Ser Ile Ser Lys Val Asp Glu Asp Ser Tyr Arg Ile Leu Ser Val
            20                  25                  30

Ser Tyr

<210>  24
<211>  51
<212>  PRT
<213>  Plasmodium falciparum

<220>
<221>  MISC_FEATURE
<222>  (1)..(51)
<223>  Motif f of MSP3.4 (amino acids 646-697)

<400>  24

Arg Ile Leu Ser Val Ser Tyr Lys Asp Asn Asn Glu Val Lys Asn Val
1               5                   10                  15

Ala Glu Ser Ile Val Lys Lys Leu Phe Ser Leu Phe Asn Asp Asn Asn
            20                  25                  30

Asn Leu Glu Thr Ile Phe Lys Gly Leu Thr Glu Asp Met Thr Asp Leu
        35                  40                  45

Phe Gln Lys
    50

<210>  25
<211>  26
<212>  PRT
<213>  Plasmodium falciparum

<220>
<221>  MISC_FEATURE
<222>  (1)..(26)
<223>  Motif a of MSP3.7 (amino acids 214-240)

<400>  25

Pro Glu Gly Pro Arg Ala Asn Asn Arg Asn Glu Asn Asn Gln Asn Thr
1               5                   10                  15

Asp Pro Tyr Asn His Tyr Phe Ala Trp Glu
            20                  25

<210>  26
<211>  22
<212>  PRT
<213>  Plasmodium falciparum

<220>
<221>  MISC_FEATURE

<222>  (1)..(22)
<223>  Motif b of MSP3.7 (amino acids 232-254)

<400>  26

Tyr Asn His Tyr Phe Ala Trp Glu Ile Gly Gly Gly Ala Pro Thr Tyr
1               5                   10                  15

Lys Pro Glu Asn Asn Lys
            20

<210>  27
<211>  27
<212>  PRT
<213>  Plasmodium falciparum

<220>
<221>  MISC_FEATURE
<222>  (1)..(27)
<223>  Motif c of MSP3.7 (amino acids 245-272)

<400>  27

Pro Thr Tyr Lys Pro Glu Asn Asn Lys Asn Asp Asn Ile Leu Leu Glu
1               5                   10                  15

His Val Lys Ile Thr Ser Trp Asp Lys Glu Asp
            20                  25

<210>  28
<211>  29
<212>  PRT
<213>  Plasmodium falciparum

<220>
<221>  MISC_FEATURE
<222>  (1)..(29)
<223>  Motif d of MSP3.7 (amino acids 257-286)

<400>  28

Ile Leu Leu Glu His Val Lys Ile Thr Ser Trp Asp Lys Glu Asp Ile
1               5                   10                  15

Ile Lys Glu Asn Glu Asp Thr Lys Arg Glu Val Gln Glu
            20                  25

<210>  29
<211>  35
<212>  PRT
<213>  Plasmodium falciparum

<220>
<221>  MISC_FEATURE
<222>  (1)..(30)
<223>  Motif e of MSP3.7 (amino acids 324-359)

<400>  29

Val Asn Leu Glu Asp Ile Asn Lys Asn Thr Arg Asn Asp Ile Phe Glu
1               5                   10                  15

Glu Gln Ile Lys Leu Asp Ser Thr Gln Asp Asp Lys Ala Gln Lys Leu
            20                  25                  30

Ile Ser Asn
        35

<210>  30
<211>  52
<212>  PRT
<213>  Plasmodium falciparum

<220>
<221>  MISC_FEATURE
<222>  (1)..(52)
<223>  Motif f of MSP3.7 (amino acids 353-405)

<400>  30

Gln Lys Leu Ile Ser Asn Glu Tyr Lys Lys Thr Glu Glu Lys Lys Ser
1               5                   10                  15

Leu Glu Asp His Val Asn Leu Leu Phe Asn Phe Leu Gln Thr Asn Asn
            20                  25                  30

Gln Leu Asp Pro Ser Leu Lys Asp Leu Glu Asn Glu Leu Thr Phe Phe
        35                  40                  45

Leu Asn Asn Tyr
    50

<210>  31
<211>  33
<212>  PRT
<213>  Plasmodium falciparum

<220>
<221>  MISC_FEATURE
<222>  (1)..(33)
<223>  Motif a of MSP3.8 (amino acids 537-570)

<400>  31

His Glu Ser Asn Val Gly Ser Ile Gln Glu Val Asn Gln Gly Ser Val
1               5                   10                  15

Ser Glu Glu Ser His Ser Lys Thr Ile Asp Pro Ser Lys Ile Asp Asp
            20                  25                  30

Arg

<210>  32
<211>  29

<212>  PRT
<213>  Plasmodium falciparum

<220>
<221>  MISC_FEATURE
<222>  (1)..(29)
<223>  Motif b of MSP3.8 (amino acids 564-593)

<400>  32

Ser Lys Thr Ile Asp Pro Ser Lys Ile Asp Asp Arg Leu Glu Leu Ser
1               5                   10                  15

Ser Gly Ser Ser Ser Leu Glu Gln His Ser Lys Glu Asp
            20                  25

<210>  33
<211>  29
<212>  PRT
<213>  Plasmodium falciparum

<220>
<221>  MISC_FEATURE
<222>  (1)..(29)
<223>  Motif c of MSP3.8 (amino acids 586-615)

<400>  33

Glu Gln His Ser Lys Glu Asp Val Lys Lys Gly Cys Ala Leu Glu Leu
1               5                   10                  15

Val Pro Leu Ser Leu Ser Asp Ile Glu Gln Ile Ala Asn
            20                  25

<210>  34
<211>  33
<212>  PRT
<213>  Plasmodium falciparum

<220>
<221>  MISC_FEATURE
<222>  (1)..(33)
<223>  Motif d of MSP3.8 (amino acids 598-631)

<400>  34

Ala Leu Glu Leu Val Pro Leu Ser Leu Ser Asp Ile Glu Gln Ile Ala
1               5                   10                  15

Asn Glu Ser Glu Asp Val Leu Glu Glu Ile Glu Glu Glu Ile Asn Thr
            20                  25                  30

Asp

<210>  35
<211>  41
<212>  PRT

<213>  Plasmodium falciparum

<220>
<221>  MISC_FEATURE
<222>  (1)..(41)
<223>  Motif e of MSP3.8 (amino acids 669-710)

<400>  35

Glu Ile Glu Asp Lys Pro Glu Gln Glu Ile Lys Asn Lys Ser Leu Glu
1               5                   10                  15

Glu Lys Gln Ile Asp Lys Asn Thr Asp Thr Ser Glu Lys Lys Gly Phe
            20                  25                  30

Asn Asn Ser Glu Lys Asp Glu Lys Ala
        35                  40

<210>  36
<211>  61
<212>  PRT
<213>  Plasmodium falciparum

<220>
<221>  MISC_FEATURE
<222>  (1)..(61)
<223>  Motif f of MSP3.8 (amino acids 701-762)

<400>  36

Asn Asn Ser Glu Lys Asp Glu Lys Ala Arg Asn Leu Ile Ser Lys Asn
1               5                   10                  15

Tyr Lys Asn Tyr Asn Glu Leu Asp Lys Asn Val His Thr Leu Val Asn
            20                  25                  30

Ser Ile Ile Ser Leu Leu Glu Glu Gly Asn Gly Ser Asp Ser Thr Leu
        35                  40                  45

Asn Ser Leu Ser Lys Asp Ile Thr Asn Leu Phe Lys Asn
    50                  55                  60

<210>  37
<211>  204
<212>  DNA
<213>  Plasmodium falciparum

<220>
<221>  CDS
<222>  (1)..(204)

<220>
<221>  misc_feature
<222>  (1)..(204)
<223>  Motifs bcd of P. falciparum MSP3-1

<400>  37
gca aaa gaa gct tct agt tat gat tat att tta ggt tgg gaa ttt gga       48

Ala Lys Glu Ala Ser Ser Tyr Asp Tyr Ile Leu Gly Trp Glu Phe Gly
1               5                   10                  15

gga ggc gtt cca gaa cac aaa aaa gaa gaa aat atg tta tca cat tta         96
Gly Gly Val Pro Glu His Lys Lys Glu Glu Asn Met Leu Ser His Leu
            20                  25                  30

tat gtt tct tca aag gat aag gaa aat ata tct aag gaa aat gat gat        144
Tyr Val Ser Ser Lys Asp Lys Glu Asn Ile Ser Lys Glu Asn Asp Asp
        35                  40                  45

gta tta gat gag aag gaa gaa gag gca gaa gaa aca gaa gaa gaa gaa        192
Val Leu Asp Glu Lys Glu Glu Glu Ala Glu Glu Thr Glu Glu Glu Glu
    50                  55                  60

ctt gaa gaa aaa                                                        204
Leu Glu Glu Lys
65

<210>  38
<211>  68
<212>  PRT
<213>  Plasmodium falciparum

<400>  38

Ala Lys Glu Ala Ser Ser Tyr Asp Tyr Ile Leu Gly Trp Glu Phe Gly
1               5                   10                  15

Gly Gly Val Pro Glu His Lys Lys Glu Glu Asn Met Leu Ser His Leu
            20                  25                  30

Tyr Val Ser Ser Lys Asp Lys Glu Asn Ile Ser Lys Glu Asn Asp Asp
        35                  40                  45

Val Leu Asp Glu Lys Glu Glu Glu Ala Glu Glu Thr Glu Glu Glu Glu
    50                  55                  60

Leu Glu Glu Lys
65

<210>  39
<211>  291
<212>  DNA
<213>  Plasmodium falciparum

<220>
<221>  CDS
<222>  (1)..(291)

<220>
<221>  misc_feature
<222>  (1)..(291)
<223>  Motifs abcd of P. falciparum MSP3-2

<400>  39
tct gaa aca aat aaa aat cct act tct cat agt aat agt act aca act         48
Ser Glu Thr Asn Lys Asn Pro Thr Ser His Ser Asn Ser Thr Thr Thr
1               5                   10                  15

tct ctg aat aat aat ata ctt gga tgg gaa ttt gga gga ggt gct cct         96

Ser Leu Asn Asn Asn Ile Leu Gly Trp Glu Phe Gly Gly Gly Ala Pro
            20                  25                  30

caa aat gga gct gca gaa gat aaa aag aca gaa tat tta cta gaa caa      144
Gln Asn Gly Ala Ala Glu Asp Lys Lys Thr Glu Tyr Leu Leu Glu Gln
        35                  40                  45

ata aaa att cca tca tgg gat aga aat aac atc ccc gat gag aat gaa      192
Ile Lys Ile Pro Ser Trp Asp Arg Asn Asn Ile Pro Asp Glu Asn Glu
    50                  55                  60

caa gta ata gag gac cct caa gaa gat aat aaa gat gaa gat gaa gat      240
Gln Val Ile Glu Asp Pro Gln Glu Asp Asn Lys Asp Glu Asp Glu Asp
65                  70                  75                  80

gaa gaa aca gaa aca gaa aat ttg gaa aca gaa gat gat aat aat gaa      288
Glu Glu Thr Glu Thr Glu Asn Leu Glu Thr Glu Asp Asp Asn Asn Glu
                85                  90                  95

gag                                                                  291
Glu

<210>  40
<211>  97
<212>  PRT
<213>  Plasmodium falciparum

<400>  40

Ser Glu Thr Asn Lys Asn Pro Thr Ser His Ser Asn Ser Thr Thr Thr
1               5                   10                  15

Ser Leu Asn Asn Asn Ile Leu Gly Trp Glu Phe Gly Gly Gly Ala Pro
            20                  25                  30

Gln Asn Gly Ala Ala Glu Asp Lys Lys Thr Glu Tyr Leu Leu Glu Gln
        35                  40                  45

Ile Lys Ile Pro Ser Trp Asp Arg Asn Asn Ile Pro Asp Glu Asn Glu
    50                  55                  60

Gln Val Ile Glu Asp Pro Gln Glu Asp Asn Lys Asp Glu Asp Glu Asp
65                  70                  75                  80

Glu Glu Thr Glu Thr Glu Asn Leu Glu Thr Glu Asp Asp Asn Asn Glu
                85                  90                  95

Glu

<210>  41
<211>  240
<212>  DNA
<213>  Plasmodium falciparum

<220>
<221>  misc_feature
<222>  (1)..(240)

<223>  Motifs abcd of P. falciparum MSP3-3

<220>
<221>  CDS
<222>  (1)..(240)

<400>  41
tat gag aag aaa aat gaa aat aaa aat gta tca aat gta gat tca aaa        48
Tyr Glu Lys Lys Asn Glu Asn Lys Asn Val Ser Asn Val Asp Ser Lys
1               5                   10                  15

aca aaa tca aat gaa aaa gga aga cct cct aca tat tct cct att ctg        96
Thr Lys Ser Asn Glu Lys Gly Arg Pro Pro Thr Tyr Ser Pro Ile Leu
            20                  25                  30

gat gat ggg ata gaa ttt agt ggt ggt tta tat ttt aat gag aaa aag       144
Asp Asp Gly Ile Glu Phe Ser Gly Gly Leu Tyr Phe Asn Glu Lys Lys
        35                  40                  45

tcg act gaa gaa aat aaa caa aaa aat gtt tta gaa tca gta aat tta       192
Ser Thr Glu Glu Asn Lys Gln Lys Asn Val Leu Glu Ser Val Asn Leu
    50                  55                  60

aca tcg tgg gat aaa gag gat att gtt aag gaa aat gag gat gtt aaa       240
Thr Ser Trp Asp Lys Glu Asp Ile Val Lys Glu Asn Glu Asp Val Lys
65                  70                  75                  80


<210>  42
<211>  80
<212>  PRT
<213>  Plasmodium falciparum

<400>  42

Tyr Glu Lys Lys Asn Glu Asn Lys Asn Val Ser Asn Val Asp Ser Lys
1               5                   10                  15


Thr Lys Ser Asn Glu Lys Gly Arg Pro Pro Thr Tyr Ser Pro Ile Leu
            20                  25                  30


Asp Asp Gly Ile Glu Phe Ser Gly Gly Leu Tyr Phe Asn Glu Lys Lys
        35                  40                  45


Ser Thr Glu Glu Asn Lys Gln Lys Asn Val Leu Glu Ser Val Asn Leu
    50                  55                  60


Thr Ser Trp Asp Lys Glu Asp Ile Val Lys Glu Asn Glu Asp Val Lys
65                  70                  75                  80


<210>  43
<211>  216
<212>  DNA
<213>  Plasmodium falciparum


<220>
<221>  misc_feature
<222>  (1)..(216)
<223>  Motifs abcd of P. falciparum MSP3-4

<220>
<221>  CDS

<222>  (1)..(216)

<400>  43
gat aat gta aac tct gta acg caa aga gga aat aat aac tac aac aat        48
Asp Asn Val Asn Ser Val Thr Gln Arg Gly Asn Asn Asn Tyr Asn Asn
1               5                   10                  15

aat tta gag cgt gga ttg ggt tct ggt gct ctt cct ggt aca aat att        96
Asn Leu Glu Arg Gly Leu Gly Ser Gly Ala Leu Pro Gly Thr Asn Ile
            20                  25                  30

att act gaa gaa aaa tat tct cta gaa tta ata aaa tta aca tca aag       144
Ile Thr Glu Glu Lys Tyr Ser Leu Glu Leu Ile Lys Leu Thr Ser Lys
        35                  40                  45

gat gaa gaa gat att ata aag cat aat gag gat gtg aga gaa gaa ata       192
Asp Glu Glu Asp Ile Ile Lys His Asn Glu Asp Val Arg Glu Glu Ile
    50                  55                  60

gaa gaa caa caa gaa gac atc gag                                       216
Glu Glu Gln Gln Glu Asp Ile Glu
65                  70


<210>  44
<211>  72
<212>  PRT
<213>  Plasmodium falciparum

<400>  44

Asp Asn Val Asn Ser Val Thr Gln Arg Gly Asn Asn Asn Tyr Asn Asn
1               5                   10                  15


Asn Leu Glu Arg Gly Leu Gly Ser Gly Ala Leu Pro Gly Thr Asn Ile
            20                  25                  30


Ile Thr Glu Glu Lys Tyr Ser Leu Glu Leu Ile Lys Leu Thr Ser Lys
        35                  40                  45


Asp Glu Glu Asp Ile Ile Lys His Asn Glu Asp Val Arg Glu Glu Ile
    50                  55                  60


Glu Glu Gln Gln Glu Asp Ile Glu
65                  70


<210>  45
<211>  216
<212>  DNA
<213>  Plasmodium falciparum


<220>
<221>  misc_feature
<222>  (1)..(216)
<223>  Motifs abcd of P. falciparum MSP3-7

<220>
<221>  CDS
<222>  (1)..(216)

<400>  45
cct gaa gga cca aga gca aac aat aga aat gaa aat aat caa aat aca        48

Pro Glu Gly Pro Arg Ala Asn Asn Arg Asn Glu Asn Asn Gln Asn Thr
1               5                   10                  15

gat cca tat aac cac tat ttt gca tgg gaa att gga ggt ggt gct cca      96
Asp Pro Tyr Asn His Tyr Phe Ala Trp Glu Ile Gly Gly Gly Ala Pro
            20                  25                  30

acg tat aaa ccc gag aac aat aag aac gat aat att ttg cta gaa cac      144
Thr Tyr Lys Pro Glu Asn Asn Lys Asn Asp Asn Ile Leu Leu Glu His
        35                  40                  45

gta aaa att acc tcg tgg gat aaa gaa gat ata att aaa gaa aat gaa      192
Val Lys Ile Thr Ser Trp Asp Lys Glu Asp Ile Ile Lys Glu Asn Glu
    50                  55                  60

gac aca aaa cgc gaa gtt caa gaa                                      216
Asp Thr Lys Arg Glu Val Gln Glu
65                  70


<210>  46
<211>  72
<212>  PRT
<213>  Plasmodium falciparum

<400>  46

Pro Glu Gly Pro Arg Ala Asn Asn Arg Asn Glu Asn Asn Gln Asn Thr
1               5                   10                  15


Asp Pro Tyr Asn His Tyr Phe Ala Trp Glu Ile Gly Gly Gly Ala Pro
            20                  25                  30


Thr Tyr Lys Pro Glu Asn Asn Lys Asn Asp Asn Ile Leu Leu Glu His
        35                  40                  45


Val Lys Ile Thr Ser Trp Asp Lys Glu Asp Ile Ile Lys Glu Asn Glu
    50                  55                  60


Asp Thr Lys Arg Glu Val Gln Glu
65                  70


<210>  47
<211>  264
<212>  DNA
<213>  Plasmodium falciparum


<220>
<221>  misc_feature
<222>  (1)..(264)
<223>  Motifs abcd of P. falciparum MSP3-8

<220>
<221>  CDS
<222>  (1)..(264)

<400>  47
cat gaa agt aat gtt ggt agc atc caa gaa gta aac caa ggt agc gtg      48
His Glu Ser Asn Val Gly Ser Ile Gln Glu Val Asn Gln Gly Ser Val
1               5                   10                  15

agc gaa gaa tca cat tct aaa act ata gat cct tct aag att gac gac      96

Ser Glu Glu Ser His Ser Lys Thr Ile Asp Pro Ser Lys Ile Asp Asp
            20                  25                  30

cgt ttg gaa tta agt agt ggg tca tca tct ctt gaa caa cac tct aag       144
Arg Leu Glu Leu Ser Ser Gly Ser Ser Ser Leu Glu Gln His Ser Lys
        35                  40                  45

gaa gat gta aaa aag gga tgt gct tta gaa ttg gta cct tta tct tta       192
Glu Asp Val Lys Lys Gly Cys Ala Leu Glu Leu Val Pro Leu Ser Leu
    50                  55                  60

tcg gat att gaa cag ata gct aat gaa agc gaa gat gta ctg gaa gag       240
Ser Asp Ile Glu Gln Ile Ala Asn Glu Ser Glu Asp Val Leu Glu Glu
65                  70                  75                  80

ata gaa gaa gaa att aat aca gat                                       264
Ile Glu Glu Glu Ile Asn Thr Asp
                85

<210>  48
<211>  88
<212>  PRT
<213>  Plasmodium falciparum

<400>  48

His Glu Ser Asn Val Gly Ser Ile Gln Glu Val Asn Gln Gly Ser Val
1               5                   10                  15

Ser Glu Glu Ser His Ser Lys Thr Ile Asp Pro Ser Lys Ile Asp Asp
            20                  25                  30

Arg Leu Glu Leu Ser Ser Gly Ser Ser Ser Leu Glu Gln His Ser Lys
        35                  40                  45

Glu Asp Val Lys Lys Gly Cys Ala Leu Glu Leu Val Pro Leu Ser Leu
    50                  55                  60

Ser Asp Ile Glu Gln Ile Ala Asn Glu Ser Glu Asp Val Leu Glu Glu
65                  70                  75                  80

Ile Glu Glu Glu Ile Asn Thr Asp
                85

<210>  49
<211>  237
<212>  DNA
<213>  Plasmodium falciparum

<220>
<221>  CDS
<222>  (1)..(237)

<220>
<221>  misc_feature
<222>  (1)..(237)
<223>  Motifs ef of P. falciparum MSP3-2

<400>  49
gaa aaa aaa cca gac aat gaa att aca aat gaa gtt aaa gag gaa caa       48

Glu Lys Lys Pro Asp Asn Glu Ile Thr Asn Glu Val Lys Glu Glu Gln
1               5                   10                  15

aaa tat agt tca cca agt gat ata aat gcc caa aat tta att tct aat      96
Lys Tyr Ser Ser Pro Ser Asp Ile Asn Ala Gln Asn Leu Ile Ser Asn
            20                  25                  30

aag aat aaa aag aat gat gaa aca aaa aag act gct gaa aat ata gtt      144
Lys Asn Lys Lys Asn Asp Glu Thr Lys Lys Thr Ala Glu Asn Ile Val
        35                  40                  45

aaa aca ttg gtt gga tta ttt aat gaa aaa aat gag ata gat tct act      192
Lys Thr Leu Val Gly Leu Phe Asn Glu Lys Asn Glu Ile Asp Ser Thr
    50                  55                  60

ata aat aat tta gta caa gaa atg atc cat cta ttt agt aat aat          237
Ile Asn Asn Leu Val Gln Glu Met Ile His Leu Phe Ser Asn Asn
65                  70                  75

<210>  50
<211>  79
<212>  PRT
<213>  Plasmodium falciparum

<400>  50

Glu Lys Lys Pro Asp Asn Glu Ile Thr Asn Glu Val Lys Glu Glu Gln
1               5                   10                  15

Lys Tyr Ser Ser Pro Ser Asp Ile Asn Ala Gln Asn Leu Ile Ser Asn
            20                  25                  30

Lys Asn Lys Lys Asn Asp Glu Thr Lys Lys Thr Ala Glu Asn Ile Val
        35                  40                  45

Lys Thr Leu Val Gly Leu Phe Asn Glu Lys Asn Glu Ile Asp Ser Thr
    50                  55                  60

Ile Asn Asn Leu Val Gln Glu Met Ile His Leu Phe Ser Asn Asn
65                  70                  75

<210>  51
<211>  81
<212>  DNA
<213>  Plasmodium falciparum

<220>
<221>  CDS
<222>  (1)..(81)

<220>
<221>  misc_feature
<222>  (1)..(81)
<223>  p27

<400>  51
aaa aaa aga aac gtc gaa gag gaa tta cac agc tta agg aaa aat tat      48
Lys Lys Arg Asn Val Glu Glu Glu Leu His Ser Leu Arg Lys Asn Tyr
1               5                   10                  15

aat atc att aat gaa gaa att gaa gaa ata aca                          81

Asn Ile Ile Asn Glu Glu Ile Glu Glu Ile Thr
            20                  25

<210>  52
<211>  27
<212>  PRT
<213>  Plasmodium falciparum

<400>  52

Lys Lys Arg Asn Val Glu Glu Glu Leu His Ser Leu Arg Lys Asn Tyr
1               5                   10                  15

Asn Ile Ile Asn Glu Glu Ile Glu Glu Ile Thr
            20                  25

<210>  53
<211>  144
<212>  DNA
<213>  Plasmodium falciparum

<220>
<221>  CDS
<222>  (1)..(144)

<220>
<221>  misc_feature
<222>  (1)..(144)
<223>  LSA3

<400>  53
gta gaa gaa aat gta gaa gaa aat gac gac gaa agt gta gcc tca agt        48
Val Glu Glu Asn Val Glu Glu Asn Asp Asp Glu Ser Val Ala Ser Ser
1               5                   10                  15

gtt gaa gaa agt ata gct tca agt gtt gaa gaa agt gta gct gaa aat        96
Val Glu Glu Ser Ile Ala Ser Ser Val Glu Glu Ser Val Ala Glu Asn
            20                  25                  30

gtt gaa gaa agt gta gct gaa aat gtt gaa gaa agt gta gct gaa aat        144
Val Glu Glu Ser Val Ala Glu Asn Val Glu Glu Ser Val Ala Glu Asn
        35                  40                  45

<210>  54
<211>  48
<212>  PRT
<213>  Plasmodium falciparum

<400>  54

Val Glu Glu Asn Val Glu Glu Asn Asp Asp Glu Ser Val Ala Ser Ser
1               5                   10                  15

Val Glu Glu Ser Ile Ala Ser Ser Val Glu Glu Ser Val Ala Glu Asn
            20                  25                  30

Val Glu Glu Ser Val Ala Glu Asn Val Glu Glu Ser Val Ala Glu Asn
        35                  40                  45

<210>  55

<211>  1434
<212>  DNA
<213>  Artificial

<220>
<223>  Polypeptide


<220>
<221>  misc_feature
<222>  (1)..(1434)
<223>  construct 1

<220>
<221>  CDS
<222>  (1)..(1434)

<400>  55
gca aaa gaa gct tct agt tat gat tat att tta ggt tgg gaa ttt gga      48
Ala Lys Glu Ala Ser Ser Tyr Asp Tyr Ile Leu Gly Trp Glu Phe Gly
1               5                   10                  15

gga ggc gtt cca gaa cac aaa aaa gaa gaa aat atg tta tca cat tta      96
Gly Gly Val Pro Glu His Lys Lys Glu Glu Asn Met Leu Ser His Leu
            20                  25                  30

tat gtt tct tca aag gat aag gaa aat ata tct aag gaa aat gat gat      144
Tyr Val Ser Ser Lys Asp Lys Glu Asn Ile Ser Lys Glu Asn Asp Asp
        35                  40                  45

gta tta gat gag aag gaa gaa gag gca gaa gaa aca gaa gaa gaa gaa      192
Val Leu Asp Glu Lys Glu Glu Glu Ala Glu Glu Thr Glu Glu Glu Glu
    50                  55                  60

ctt gaa gaa aaa tct gaa aca aat aaa aat cct act tct cat agt aat      240
Leu Glu Glu Lys Ser Glu Thr Asn Lys Asn Pro Thr Ser His Ser Asn
65                  70                  75                  80

agt act aca act tct ctg aat aat aat ata ctt gga tgg gaa ttt gga      288
Ser Thr Thr Thr Ser Leu Asn Asn Asn Ile Leu Gly Trp Glu Phe Gly
                85                  90                  95

gga ggt gct cct caa aat gga gct gca gaa gat aaa aag aca gaa tat      336
Gly Gly Ala Pro Gln Asn Gly Ala Ala Glu Asp Lys Lys Thr Glu Tyr
            100                 105                 110

tta cta gaa caa ata aaa att cca tca tgg gat aga aat aac atc ccc      384
Leu Leu Glu Gln Ile Lys Ile Pro Ser Trp Asp Arg Asn Asn Ile Pro
        115                 120                 125

gat gag aat gaa caa gta ata gag gac cct caa gaa gat aat aaa gat      432
Asp Glu Asn Glu Gln Val Ile Glu Asp Pro Gln Glu Asp Asn Lys Asp
    130                 135                 140

gaa gat gaa gat gaa gaa aca gaa aca gaa aat ttg gaa aca gaa gat      480
Glu Asp Glu Asp Glu Glu Thr Glu Thr Glu Asn Leu Glu Thr Glu Asp
145                 150                 155                 160

gat aat aat gaa gag tat gag aag aaa aat gaa aat aaa aat gta tca      528
Asp Asn Asn Glu Glu Tyr Glu Lys Lys Asn Glu Asn Lys Asn Val Ser
                165                 170                 175

aat gta gat tca aaa aca aaa tca aat gaa aaa gga aga cct cct aca      576
Asn Val Asp Ser Lys Thr Lys Ser Asn Glu Lys Gly Arg Pro Pro Thr
            180                 185                 190

tat tct cct att ctg gat gat ggg ata gaa ttt agt ggt ggt tta tat      624
Tyr Ser Pro Ile Leu Asp Asp Gly Ile Glu Phe Ser Gly Gly Leu Tyr

195                 200                 205

ttt aat gag aaa aag tcg act gaa gaa aat aaa caa aaa aat gtt tta      672
Phe Asn Glu Lys Lys Ser Thr Glu Glu Asn Lys Gln Lys Asn Val Leu
    210                 215                 220

gaa tca gta aat tta aca tcg tgg gat aaa gag gat att gtt aag gaa      720
Glu Ser Val Asn Leu Thr Ser Trp Asp Lys Glu Asp Ile Val Lys Glu
225                 230                 235                 240

aat gag gat gtt aaa gat aat gta aac tct gta acg caa aga gga aat      768
Asn Glu Asp Val Lys Asp Asn Val Asn Ser Val Thr Gln Arg Gly Asn
                245                 250                 255

aat aac tac aac aat aat tta gag cgt gga ttg ggt tct ggt gct ctt      816
Asn Asn Tyr Asn Asn Asn Leu Glu Arg Gly Leu Gly Ser Gly Ala Leu
            260                 265                 270

cct ggt aca aat att att act gaa gaa aaa tat tct cta gaa tta ata      864
Pro Gly Thr Asn Ile Ile Thr Glu Glu Lys Tyr Ser Leu Glu Leu Ile
        275                 280                 285

aaa tta aca tca aag gat gaa gaa gat att ata aag cat aat gag gat      912
Lys Leu Thr Ser Lys Asp Glu Glu Asp Ile Ile Lys His Asn Glu Asp
    290                 295                 300

gtg aga gaa gaa ata gaa gaa caa caa gaa gac atc gag cct gaa gga      960
Val Arg Glu Glu Ile Glu Glu Gln Gln Glu Asp Ile Glu Pro Glu Gly
305                 310                 315                 320

cca aga gca aac aat aga aat gaa aat aat caa aat aca gat cca tat     1008
Pro Arg Ala Asn Asn Arg Asn Glu Asn Asn Gln Asn Thr Asp Pro Tyr
                325                 330                 335

aac cac tat ttt gca tgg gaa att gga ggt ggt gct cca acg tat aaa     1056
Asn His Tyr Phe Ala Trp Glu Ile Gly Gly Gly Ala Pro Thr Tyr Lys
            340                 345                 350

ccc gag aac aat aag aac gat aat att ttg cta gaa cac gta aaa att     1104
Pro Glu Asn Asn Lys Asn Asp Asn Ile Leu Leu Glu His Val Lys Ile
        355                 360                 365

acc tcg tgg gat aaa gaa gat ata att aaa gaa aat gaa gac aca aaa     1152
Thr Ser Trp Asp Lys Glu Asp Ile Ile Lys Glu Asn Glu Asp Thr Lys
    370                 375                 380

cgc gaa gtt caa gaa cat gaa agt aat gtt ggt agc atc caa gaa gta     1200
Arg Glu Val Gln Glu His Glu Ser Asn Val Gly Ser Ile Gln Glu Val
385                 390                 395                 400

aac caa ggt agc gtg agc gaa gaa tca cat tct aaa act ata gat cct     1248
Asn Gln Gly Ser Val Ser Glu Glu Ser His Ser Lys Thr Ile Asp Pro
                405                 410                 415

tct aag att gac gac cgt ttg gaa tta agt agt ggg tca tca tct ctt     1296
Ser Lys Ile Asp Asp Arg Leu Glu Leu Ser Ser Gly Ser Ser Ser Leu
            420                 425                 430

gaa caa cac tct aag gaa gat gta aaa aag gga tgt gct tta gaa ttg     1344
Glu Gln His Ser Lys Glu Asp Val Lys Lys Gly Cys Ala Leu Glu Leu
        435                 440                 445

gta cct tta tct tta tcg gat att gaa cag ata gct aat gaa agc gaa     1392
Val Pro Leu Ser Leu Ser Asp Ile Glu Gln Ile Ala Asn Glu Ser Glu
    450                 455                 460

gat gta ctg gaa gag ata gaa gaa gaa att aat aca gat tag             1434
Asp Val Leu Glu Glu Ile Glu Glu Glu Ile Asn Thr Asp

465                 470                 475

<210>  56
<211>  477
<212>  PRT
<213>  Artificial

<220>
<223>  Synthetic Construct

<400>  56

Ala Lys Glu Ala Ser Ser Tyr Asp Tyr Ile Leu Gly Trp Glu Phe Gly
1               5                   10                  15

Gly Gly Val Pro Glu His Lys Lys Glu Glu Asn Met Leu Ser His Leu
            20                  25                  30

Tyr Val Ser Ser Lys Asp Lys Glu Asn Ile Ser Lys Glu Asn Asp Asp
        35                  40                  45

Val Leu Asp Glu Lys Glu Glu Glu Ala Glu Glu Thr Glu Glu Glu Glu
    50                  55                  60

Leu Glu Glu Lys Ser Glu Thr Asn Lys Asn Pro Thr Ser His Ser Asn
65                  70                  75                  80

Ser Thr Thr Thr Ser Leu Asn Asn Asn Ile Leu Gly Trp Glu Phe Gly
                85                  90                  95

Gly Gly Ala Pro Gln Asn Gly Ala Ala Glu Asp Lys Lys Thr Glu Tyr
            100                 105                 110

Leu Leu Glu Gln Ile Lys Ile Pro Ser Trp Asp Arg Asn Asn Ile Pro
        115                 120                 125

Asp Glu Asn Glu Gln Val Ile Glu Asp Pro Gln Glu Asp Asn Lys Asp
    130                 135                 140

Glu Asp Glu Asp Glu Glu Thr Glu Thr Glu Asn Leu Glu Thr Glu Asp
145                 150                 155                 160

Asp Asn Asn Glu Glu Tyr Glu Lys Lys Asn Glu Asn Lys Asn Val Ser
                165                 170                 175

Asn Val Asp Ser Lys Thr Lys Ser Asn Glu Lys Gly Arg Pro Pro Thr
            180                 185                 190

Tyr Ser Pro Ile Leu Asp Asp Gly Ile Glu Phe Ser Gly Gly Leu Tyr
        195                 200                 205

Phe Asn Glu Lys Lys Ser Thr Glu Glu Asn Lys Gln Lys Asn Val Leu
    210                 215                 220

Glu Ser Val Asn Leu Thr Ser Trp Asp Lys Glu Asp Ile Val Lys Glu
225                 230                 235                 240

Asn Glu Asp Val Lys Asp Asn Val Asn Ser Val Thr Gln Arg Gly Asn
                245                 250                 255

Asn Asn Tyr Asn Asn Asn Leu Glu Arg Gly Leu Gly Ser Gly Ala Leu
            260                 265                 270

Pro Gly Thr Asn Ile Ile Thr Glu Glu Lys Tyr Ser Leu Glu Leu Ile
        275                 280                 285

Lys Leu Thr Ser Lys Asp Glu Glu Asp Ile Ile Lys His Asn Glu Asp
    290                 295                 300

Val Arg Glu Glu Ile Glu Glu Gln Gln Glu Asp Ile Glu Pro Glu Gly
305                 310                 315                 320

Pro Arg Ala Asn Asn Arg Asn Glu Asn Asn Gln Asn Thr Asp Pro Tyr
                325                 330                 335

Asn His Tyr Phe Ala Trp Glu Ile Gly Gly Gly Ala Pro Thr Tyr Lys
            340                 345                 350

Pro Glu Asn Asn Lys Asn Asp Asn Ile Leu Leu Glu His Val Lys Ile
        355                 360                 365

Thr Ser Trp Asp Lys Glu Asp Ile Ile Lys Glu Asn Glu Asp Thr Lys
    370                 375                 380

Arg Glu Val Gln Glu His Glu Ser Asn Val Gly Ser Ile Gln Glu Val
385                 390                 395                 400

Asn Gln Gly Ser Val Ser Glu Glu Ser His Ser Lys Thr Ile Asp Pro
                405                 410                 415

Ser Lys Ile Asp Asp Arg Leu Glu Leu Ser Ser Gly Ser Ser Ser Leu
            420                 425                 430

Glu Gln His Ser Lys Glu Asp Val Lys Lys Gly Cys Ala Leu Glu Leu
        435                 440                 445

Val Pro Leu Ser Leu Ser Asp Ile Glu Gln Ile Ala Asn Glu Ser Glu
    450                 455                 460

Asp Val Leu Glu Glu Ile Glu Glu Glu Ile Asn Thr Asp
465                 470                 475

<210>  57
<211>  930
<212>  DNA

<213>  Artificial

<220>
<223>  Polypeptide


<220>
<221>  CDS
<222>  (1)..(930)

<220>
<221>  misc_feature
<222>  (1)..(930)
<223>  construct 2

<400>  57
gca aaa gaa gct tct agt tat gat tat att tta ggt tgg gaa ttt gga      48
Ala Lys Glu Ala Ser Ser Tyr Asp Tyr Ile Leu Gly Trp Glu Phe Gly
1               5                   10                  15

gga ggc gtt cca gaa cac aaa aaa gaa gaa aat atg tta tca cat tta      96
Gly Gly Val Pro Glu His Lys Lys Glu Glu Asn Met Leu Ser His Leu
            20                  25                  30

tat gtt tct tca aag gat aag gaa aat ata tct aag gaa aat gat gat      144
Tyr Val Ser Ser Lys Asp Lys Glu Asn Ile Ser Lys Glu Asn Asp Asp
        35                  40                  45

gta tta gat gag aag gaa gaa gag gca gaa gaa aca gaa gaa gaa gaa      192
Val Leu Asp Glu Lys Glu Glu Glu Ala Glu Glu Thr Glu Glu Glu Glu
    50                  55                  60

ctt gaa gaa aaa tct gaa aca aat aaa aat cct act tct cat agt aat      240
Leu Glu Glu Lys Ser Glu Thr Asn Lys Asn Pro Thr Ser His Ser Asn
65                  70                  75                  80

agt act aca act tct ctg aat aat aat ata ctt gga tgg gaa ttt gga      288
Ser Thr Thr Thr Ser Leu Asn Asn Asn Ile Leu Gly Trp Glu Phe Gly
                85                  90                  95

gga ggt gct cct caa aat gga gct gca gaa gat aaa aag aca gaa tat      336
Gly Gly Ala Pro Gln Asn Gly Ala Ala Glu Asp Lys Lys Thr Glu Tyr
            100                 105                 110

tta cta gaa caa ata aaa att cca tca tgg gat aga aat aac atc ccc      384
Leu Leu Glu Gln Ile Lys Ile Pro Ser Trp Asp Arg Asn Asn Ile Pro
        115                 120                 125

gat gag aat gaa caa gta ata gag gac cct caa gaa gat aat aaa gat      432
Asp Glu Asn Glu Gln Val Ile Glu Asp Pro Gln Glu Asp Asn Lys Asp
    130                 135                 140

gaa gat gaa gat gaa gaa aca gaa aca gaa aat ttg gaa aca gaa gat      480
Glu Asp Glu Asp Glu Glu Thr Glu Thr Glu Asn Leu Glu Thr Glu Asp
145                 150                 155                 160

gat aat aat gaa gag gat aat gta aac tct gta acg caa aga gga aat      528
Asp Asn Asn Glu Glu Asp Asn Val Asn Ser Val Thr Gln Arg Gly Asn
                165                 170                 175

aat aac tac aac aat aat tta gag cgt gga ttg ggt tct ggt gct ctt      576
Asn Asn Tyr Asn Asn Asn Leu Glu Arg Gly Leu Gly Ser Gly Ala Leu
            180                 185                 190

cct ggt aca aat att att act gaa gaa aaa tat tct cta gaa tta ata      624
Pro Gly Thr Asn Ile Ile Thr Glu Glu Lys Tyr Ser Leu Glu Leu Ile
        195                 200                 205

aaa tta aca tca aag gat gaa gaa gat att ata aag cat aat gag gat 672
Lys Leu Thr Ser Lys Asp Glu Glu Asp Ile Ile Lys His Asn Glu Asp
    210                 215                 220

gtg aga gaa gaa ata gaa gaa caa caa gaa gac atc gag cct gaa gga 720
Val Arg Glu Glu Ile Glu Glu Gln Gln Glu Asp Ile Glu Pro Glu Gly
225                 230                 235                 240

cca aga gca aac aat aga aat gaa aat aat caa aat aca gat cca tat 768
Pro Arg Ala Asn Asn Arg Asn Glu Asn Asn Gln Asn Thr Asp Pro Tyr
                245                 250                 255

aac cac tat ttt gca tgg gaa att gga ggt ggt gct cca acg tat aaa 816
Asn His Tyr Phe Ala Trp Glu Ile Gly Gly Gly Ala Pro Thr Tyr Lys
            260                 265                 270

ccc gag aac aat aag aac gat aat att ttg cta gaa cac gta aaa att 864
Pro Glu Asn Asn Lys Asn Asp Asn Ile Leu Leu Glu His Val Lys Ile
        275                 280                 285

acc tcg tgg gat aaa gaa gat ata att aaa gaa aat gaa gac aca aaa 912
Thr Ser Trp Asp Lys Glu Asp Ile Ile Lys Glu Asn Glu Asp Thr Lys
    290                 295                 300

cgc gaa gtt caa gaa tag 930
Arg Glu Val Gln Glu
305

<210> 58
<211> 309
<212> PRT
<213> Artificial

<220>
<223> Synthetic Construct

<400> 58

Ala Lys Glu Ala Ser Ser Tyr Asp Tyr Ile Leu Gly Trp Glu Phe Gly
1               5                   10                  15

Gly Gly Val Pro Glu His Lys Lys Glu Glu Asn Met Leu Ser His Leu
            20                  25                  30

Tyr Val Ser Ser Lys Asp Lys Glu Asn Ile Ser Lys Glu Asn Asp Asp
        35                  40                  45

Val Leu Asp Glu Lys Glu Glu Glu Ala Glu Glu Thr Glu Glu Glu Glu
    50                  55                  60

Leu Glu Glu Lys Ser Glu Thr Asn Lys Asn Pro Thr Ser His Ser Asn
65                  70                  75                  80

Ser Thr Thr Thr Ser Leu Asn Asn Asn Ile Leu Gly Trp Glu Phe Gly
                85                  90                  95

Gly Gly Ala Pro Gln Asn Gly Ala Ala Glu Asp Lys Lys Thr Glu Tyr
            100                 105                 110

Leu Leu Glu Gln Ile Lys Ile Pro Ser Trp Asp Arg Asn Asn Ile Pro

115                 120                 125

Asp Glu Asn Glu Gln Val Ile Glu Asp Pro Gln Glu Asp Asn Lys Asp
    130                 135                 140

Glu Asp Glu Asp Glu Glu Thr Glu Thr Glu Asn Leu Glu Thr Glu Asp
145                 150                 155                 160

Asp Asn Asn Glu Glu Asp Asn Val Asn Ser Val Thr Gln Arg Gly Asn
                165                 170                 175

Asn Asn Tyr Asn Asn Asn Leu Glu Arg Gly Leu Gly Ser Gly Ala Leu
            180                 185                 190

Pro Gly Thr Asn Ile Ile Thr Glu Glu Lys Tyr Ser Leu Glu Leu Ile
        195                 200                 205

Lys Leu Thr Ser Lys Asp Glu Glu Asp Ile Ile Lys His Asn Glu Asp
    210                 215                 220

Val Arg Glu Glu Ile Glu Glu Gln Gln Glu Asp Ile Glu Pro Glu Gly
225                 230                 235                 240

Pro Arg Ala Asn Asn Arg Asn Glu Asn Asn Gln Asn Thr Asp Pro Tyr
                245                 250                 255

Asn His Tyr Phe Ala Trp Glu Ile Gly Gly Gly Ala Pro Thr Tyr Lys
            260                 265                 270

Pro Glu Asn Asn Lys Asn Asp Asn Ile Leu Leu Glu His Val Lys Ile
        275                 280                 285

Thr Ser Trp Asp Lys Glu Asp Ile Ile Lys Glu Asn Glu Asp Thr Lys
    290                 295                 300

Arg Glu Val Gln Glu
305

<210>  59
<211>  1173
<212>  DNA
<213>  Artificial

<220>
<223>  Polypeptide

<220>
<221>  CDS
<222>  (1)..(1173)

<220>
<221>  misc_feature
<222>  (1)..(1173)
<223>  construct 3

<400> 59
gca aaa gaa gct tct agt tat gat tat att tta ggt tgg gaa ttt gga 48
Ala Lys Glu Ala Ser Ser Tyr Asp Tyr Ile Leu Gly Trp Glu Phe Gly
1               5                   10                  15

gga ggc gtt cca gaa cac aaa aaa gaa gaa aat atg tta tca cat tta 96
Gly Gly Val Pro Glu His Lys Lys Glu Glu Asn Met Leu Ser His Leu
            20                  25                  30

tat gtt tct tca aag gat aag gaa aat ata tct aag gaa aat gat gat 144
Tyr Val Ser Ser Lys Asp Lys Glu Asn Ile Ser Lys Glu Asn Asp Asp
        35                  40                  45

gta tta gat gag aag gaa gaa gag gca gaa gaa aca gaa gaa gaa gaa 192
Val Leu Asp Glu Lys Glu Glu Glu Ala Glu Glu Thr Glu Glu Glu Glu
    50                  55                  60

ctt gaa gaa aaa aaa aaa aga aac gtc gaa gag gaa tta cac agc tta 240
Leu Glu Glu Lys Lys Lys Arg Asn Val Glu Glu Glu Leu His Ser Leu
65                  70                  75                  80

agg aaa aat tat aat atc att aat gaa gaa att gaa gaa ata aca tct 288
Arg Lys Asn Tyr Asn Ile Ile Asn Glu Glu Ile Glu Glu Ile Thr Ser
                85                  90                  95

gaa aca aat aaa aat cct act tct cat agt aat agt act aca act tct 336
Glu Thr Asn Lys Asn Pro Thr Ser His Ser Asn Ser Thr Thr Thr Ser
            100                 105                 110

ctg aat aat aat ata ctt gga tgg gaa ttt gga gga ggt gct cct caa 384
Leu Asn Asn Asn Ile Leu Gly Trp Glu Phe Gly Gly Gly Ala Pro Gln
        115                 120                 125

aat gga gct gca gaa gat aaa aag aca gaa tat tta cta gaa caa ata 432
Asn Gly Ala Ala Glu Asp Lys Lys Thr Glu Tyr Leu Leu Glu Gln Ile
    130                 135                 140

aaa att cca tca tgg gat aga aat aac atc ccc gat gag aat gaa caa 480
Lys Ile Pro Ser Trp Asp Arg Asn Asn Ile Pro Asp Glu Asn Glu Gln
145                 150                 155                 160

gta ata gag gac cct caa gaa gat aat aaa gat gaa gat gaa gat gaa 528
Val Ile Glu Asp Pro Gln Glu Asp Asn Lys Asp Glu Asp Glu Asp Glu
                165                 170                 175

gaa aca gaa aca gaa aat ttg gaa aca gaa gat gat aat aat gaa gag 576
Glu Thr Glu Thr Glu Asn Leu Glu Thr Glu Asp Asp Asn Asn Glu Glu
            180                 185                 190

aaa aaa aga aac gtc gaa gag gaa tta cac agc tta agg aaa aat tat 624
Lys Lys Arg Asn Val Glu Glu Glu Leu His Ser Leu Arg Lys Asn Tyr
        195                 200                 205

aat atc att aat gaa gaa att gaa gaa ata aca gat aat gta aac tct 672
Asn Ile Ile Asn Glu Glu Ile Glu Glu Ile Thr Asp Asn Val Asn Ser
    210                 215                 220

gta acg caa aga gga aat aat aac tac aac aat aat tta gag cgt gga 720
Val Thr Gln Arg Gly Asn Asn Asn Tyr Asn Asn Asn Leu Glu Arg Gly
225                 230                 235                 240

ttg ggt tct ggt gct ctt cct ggt aca aat att att act gaa gaa aaa 768
Leu Gly Ser Gly Ala Leu Pro Gly Thr Asn Ile Ile Thr Glu Glu Lys
                245                 250                 255

tat tct cta gaa tta ata aaa tta aca tca aag gat gaa gaa gat att 816
Tyr Ser Leu Glu Leu Ile Lys Leu Thr Ser Lys Asp Glu Glu Asp Ile

260                 265                 270

ata aag cat aat gag gat gtg aga gaa gaa ata gaa gaa caa caa gaa        864
Ile Lys His Asn Glu Asp Val Arg Glu Glu Ile Glu Glu Gln Gln Glu
        275                 280                 285

gac atc gag aaa aaa aga aac gtc gaa gag gaa tta cac agc tta agg        912
Asp Ile Glu Lys Lys Arg Asn Val Glu Glu Glu Leu His Ser Leu Arg
    290                 295                 300

aaa aat tat aat atc att aat gaa gaa att gaa gaa ata aca cct gaa        960
Lys Asn Tyr Asn Ile Ile Asn Glu Glu Ile Glu Glu Ile Thr Pro Glu
305                 310                 315                 320

gga cca aga gca aac aat aga aat gaa aat aat caa aat aca gat cca       1008
Gly Pro Arg Ala Asn Asn Arg Asn Glu Asn Asn Gln Asn Thr Asp Pro
                325                 330                 335

tat aac cac tat ttt gca tgg gaa att gga ggt ggt gct cca acg tat       1056
Tyr Asn His Tyr Phe Ala Trp Glu Ile Gly Gly Gly Ala Pro Thr Tyr
            340                 345                 350

aaa ccc gag aac aat aag aac gat aat att ttg cta gaa cac gta aaa       1104
Lys Pro Glu Asn Asn Lys Asn Asp Asn Ile Leu Leu Glu His Val Lys
        355                 360                 365

att acc tcg tgg gat aaa gaa gat ata att aaa gaa aat gaa gac aca       1152
Ile Thr Ser Trp Asp Lys Glu Asp Ile Ile Lys Glu Asn Glu Asp Thr
    370                 375                 380

aaa cgc gaa gtt caa gaa tag                                           1173
Lys Arg Glu Val Gln Glu
385                 390

<210>  60
<211>  390
<212>  PRT
<213>  Artificial

<220>
<223>  Synthetic Construct

<400>  60

Ala Lys Glu Ala Ser Ser Tyr Asp Tyr Ile Leu Gly Trp Glu Phe Gly
1               5                   10                  15

Gly Gly Val Pro Glu His Lys Lys Glu Glu Asn Met Leu Ser His Leu
            20                  25                  30

Tyr Val Ser Ser Lys Asp Lys Glu Asn Ile Ser Lys Glu Asn Asp Asp
        35                  40                  45

Val Leu Asp Glu Lys Glu Glu Glu Ala Glu Glu Thr Glu Glu Glu Glu
    50                  55                  60

Leu Glu Glu Lys Lys Lys Arg Asn Val Glu Glu Glu Leu His Ser Leu
65                  70                  75                  80

Arg Lys Asn Tyr Asn Ile Ile Asn Glu Glu Ile Glu Glu Ile Thr Ser
                85                  90                  95

Glu Thr Asn Lys Asn Pro Thr Ser His Ser Asn Ser Thr Thr Thr Ser
            100                 105                 110

Leu Asn Asn Asn Ile Leu Gly Trp Glu Phe Gly Gly Gly Ala Pro Gln
        115                 120                 125

Asn Gly Ala Ala Glu Asp Lys Lys Thr Glu Tyr Leu Leu Glu Gln Ile
    130                 135                 140

Lys Ile Pro Ser Trp Asp Arg Asn Asn Ile Pro Asp Glu Asn Glu Gln
145                 150                 155                 160

Val Ile Glu Asp Pro Gln Glu Asp Asn Lys Asp Glu Asp Glu Asp Glu
                165                 170                 175

Glu Thr Glu Thr Glu Asn Leu Glu Thr Glu Asp Asp Asn Asn Glu Glu
            180                 185                 190

Lys Lys Arg Asn Val Glu Glu Glu Leu His Ser Leu Arg Lys Asn Tyr
        195                 200                 205

Asn Ile Ile Asn Glu Glu Ile Glu Glu Ile Thr Asp Asn Val Asn Ser
    210                 215                 220

Val Thr Gln Arg Gly Asn Asn Asn Tyr Asn Asn Asn Leu Glu Arg Gly
225                 230                 235                 240

Leu Gly Ser Gly Ala Leu Pro Gly Thr Asn Ile Ile Thr Glu Glu Lys
                245                 250                 255

Tyr Ser Leu Glu Leu Ile Lys Leu Thr Ser Lys Asp Glu Glu Asp Ile
            260                 265                 270

Ile Lys His Asn Glu Asp Val Arg Glu Glu Ile Glu Glu Gln Gln Glu
        275                 280                 285

Asp Ile Glu Lys Lys Arg Asn Val Glu Glu Glu Leu His Ser Leu Arg
    290                 295                 300

Lys Asn Tyr Asn Ile Ile Asn Glu Glu Ile Glu Glu Ile Thr Pro Glu
305                 310                 315                 320

Gly Pro Arg Ala Asn Asn Arg Asn Glu Asn Asn Gln Asn Thr Asp Pro
                325                 330                 335

Tyr Asn His Tyr Phe Ala Trp Glu Ile Gly Gly Gly Ala Pro Thr Tyr
            340                 345                 350

Lys Pro Glu Asn Asn Lys Asn Asp Asn Ile Leu Leu Glu His Val Lys
        355                 360                 365

Ile Thr Ser Trp Asp Lys Glu Asp Ile Ile Lys Glu Asn Glu Asp Thr
    370                 375                 380

Lys Arg Glu Val Gln Glu
385                 390

<210>  61
<211>  1362
<212>  DNA
<213>  Artificial

<220>
<223>  Polypeptide

<220>
<221>  CDS
<222>  (1)..(1362)

<220>
<221>  misc_feature
<222>  (1)..(1362)
<223>  construct 4

<400>  61
gca aaa gaa gct tct agt tat gat tat att tta ggt tgg gaa ttt gga      48
Ala Lys Glu Ala Ser Ser Tyr Asp Tyr Ile Leu Gly Trp Glu Phe Gly
1               5                   10                  15

gga ggc gtt cca gaa cac aaa aaa gaa gaa aat atg tta tca cat tta      96
Gly Gly Val Pro Glu His Lys Lys Glu Glu Asn Met Leu Ser His Leu
            20                  25                  30

tat gtt tct tca aag gat aag gaa aat ata tct aag gaa aat gat gat      144
Tyr Val Ser Ser Lys Asp Lys Glu Asn Ile Ser Lys Glu Asn Asp Asp
        35                  40                  45

gta tta gat gag aag gaa gaa gag gca gaa gaa aca gaa gaa gaa gaa      192
Val Leu Asp Glu Lys Glu Glu Glu Ala Glu Glu Thr Glu Glu Glu Glu
    50                  55                  60

ctt gaa gaa aaa gta gaa gaa aat gta gaa gaa aat gac gac gaa agt      240
Leu Glu Glu Lys Val Glu Glu Asn Val Glu Glu Asn Asp Asp Glu Ser
65                  70                  75                  80

gta gcc tca agt gtt gaa gaa agt ata gct tca agt gtt gaa gaa agt      288
Val Ala Ser Ser Val Glu Glu Ser Ile Ala Ser Ser Val Glu Glu Ser
                85                  90                  95

gta gct gaa aat gtt gaa gaa agt gta gct gaa aat gtt gaa gaa agt      336
Val Ala Glu Asn Val Glu Glu Ser Val Ala Glu Asn Val Glu Glu Ser
            100                 105                 110

gta gct gaa aat tct gaa aca aat aaa aat cct act tct cat agt aat      384
Val Ala Glu Asn Ser Glu Thr Asn Lys Asn Pro Thr Ser His Ser Asn
        115                 120                 125

agt act aca act tct ctg aat aat aat ata ctt gga tgg gaa ttt gga      432
Ser Thr Thr Thr Ser Leu Asn Asn Asn Ile Leu Gly Trp Glu Phe Gly
    130                 135                 140

gga ggt gct cct caa aat gga gct gca gaa gat aaa aag aca gaa tat      480
Gly Gly Ala Pro Gln Asn Gly Ala Ala Glu Asp Lys Lys Thr Glu Tyr
145                 150                 155                 160

tta cta gaa caa ata aaa att cca tca tgg gat aga aat aac atc ccc 528
Leu Leu Glu Gln Ile Lys Ile Pro Ser Trp Asp Arg Asn Asn Ile Pro
                165                 170                 175

gat gag aat gaa caa gta ata gag gac cct caa gaa gat aat aaa gat 576
Asp Glu Asn Glu Gln Val Ile Glu Asp Pro Gln Glu Asp Asn Lys Asp
            180                 185                 190

gaa gat gaa gat gaa gaa aca gaa aca gaa aat ttg gaa aca gaa gat 624
Glu Asp Glu Asp Glu Glu Thr Glu Thr Glu Asn Leu Glu Thr Glu Asp
        195                 200                 205

gat aat aat gaa gag gta gaa gaa aat gta gaa gaa aat gac gac gaa 672
Asp Asn Asn Glu Glu Val Glu Glu Asn Val Glu Glu Asn Asp Asp Glu
    210                 215                 220

agt gta gcc tca agt gtt gaa gaa agt ata gct tca agt gtt gaa gaa 720
Ser Val Ala Ser Ser Val Glu Glu Ser Ile Ala Ser Ser Val Glu Glu
225                 230                 235                 240

agt gta gct gaa aat gtt gaa gaa agt gta gct gaa aat gtt gaa gaa 768
Ser Val Ala Glu Asn Val Glu Glu Ser Val Ala Glu Asn Val Glu Glu
                245                 250                 255

agt gta gct gaa aat gat aat gta aac tct gta acg caa aga gga aat 816
Ser Val Ala Glu Asn Asp Asn Val Asn Ser Val Thr Gln Arg Gly Asn
            260                 265                 270

aat aac tac aac aat aat tta gag cgt gga ttg ggt tct ggt gct ctt 864
Asn Asn Tyr Asn Asn Asn Leu Glu Arg Gly Leu Gly Ser Gly Ala Leu
        275                 280                 285

cct ggt aca aat att att act gaa gaa aaa tat tct cta gaa tta ata 912
Pro Gly Thr Asn Ile Ile Thr Glu Glu Lys Tyr Ser Leu Glu Leu Ile
    290                 295                 300

aaa tta aca tca aag gat gaa gaa gat att ata aag cat aat gag gat 960
Lys Leu Thr Ser Lys Asp Glu Glu Asp Ile Ile Lys His Asn Glu Asp
305                 310                 315                 320

gtg aga gaa gaa ata gaa gaa caa caa gaa gac atc gag gta gaa gaa 1008
Val Arg Glu Glu Ile Glu Glu Gln Gln Glu Asp Ile Glu Val Glu Glu
                325                 330                 335

aat gta gaa gaa aat gac gac gaa agt gta gcc tca agt gtt gaa gaa 1056
Asn Val Glu Glu Asn Asp Asp Glu Ser Val Ala Ser Ser Val Glu Glu
            340                 345                 350

agt ata gct tca agt gtt gaa gaa agt gta gct gaa aat gtt gaa gaa 1104
Ser Ile Ala Ser Ser Val Glu Glu Ser Val Ala Glu Asn Val Glu Glu
        355                 360                 365

agt gta gct gaa aat gtt gaa gaa agt gta gct gaa aat cct gaa gga 1152
Ser Val Ala Glu Asn Val Glu Glu Ser Val Ala Glu Asn Pro Glu Gly
    370                 375                 380

cca aga gca aac aat aga aat gaa aat aat caa aat aca gat cca tat 1200
Pro Arg Ala Asn Asn Arg Asn Glu Asn Asn Gln Asn Thr Asp Pro Tyr
385                 390                 395                 400

aac cac tat ttt gca tgg gaa att gga ggt ggt gct cca acg tat aaa 1248
Asn His Tyr Phe Ala Trp Glu Ile Gly Gly Gly Ala Pro Thr Tyr Lys
                405                 410                 415

ccc gag aac aat aag aac gat aat att ttg cta gaa cac gta aaa att 1296
Pro Glu Asn Asn Lys Asn Asp Asn Ile Leu Leu Glu His Val Lys Ile
            420                 425                 430

acc tcg tgg gat aaa gaa gat ata att aaa gaa aat gaa gac aca aaa      1344
Thr Ser Trp Asp Lys Glu Asp Ile Ile Lys Glu Asn Glu Asp Thr Lys
        435                 440                 445

cgc gaa gtt caa gaa tag                                             1362
Arg Glu Val Gln Glu
    450

<210>  62
<211>  453
<212>  PRT
<213>  Artificial

<220>
<223>  Synthetic Construct

<400>  62

Ala Lys Glu Ala Ser Ser Tyr Asp Tyr Ile Leu Gly Trp Glu Phe Gly
1               5                   10                  15

Gly Gly Val Pro Glu His Lys Lys Glu Glu Asn Met Leu Ser His Leu
            20                  25                  30

Tyr Val Ser Ser Lys Asp Lys Glu Asn Ile Ser Lys Glu Asn Asp Asp
        35                  40                  45

Val Leu Asp Glu Lys Glu Glu Glu Ala Glu Glu Thr Glu Glu Glu Glu
    50                  55                  60

Leu Glu Glu Lys Val Glu Glu Asn Val Glu Glu Asn Asp Asp Glu Ser
65                  70                  75                  80

Val Ala Ser Ser Val Glu Glu Ser Ile Ala Ser Ser Val Glu Glu Ser
                85                  90                  95

Val Ala Glu Asn Val Glu Glu Ser Val Ala Glu Asn Val Glu Glu Ser
            100                 105                 110

Val Ala Glu Asn Ser Glu Thr Asn Lys Asn Pro Thr Ser His Ser Asn
        115                 120                 125

Ser Thr Thr Thr Ser Leu Asn Asn Asn Ile Leu Gly Trp Glu Phe Gly
    130                 135                 140

Gly Gly Ala Pro Gln Asn Gly Ala Ala Glu Asp Lys Lys Thr Glu Tyr
145                 150                 155                 160

Leu Leu Glu Gln Ile Lys Ile Pro Ser Trp Asp Arg Asn Asn Ile Pro
                165                 170                 175

Asp Glu Asn Glu Gln Val Ile Glu Asp Pro Gln Glu Asp Asn Lys Asp
            180                 185                 190

Glu Asp Glu Asp Glu Glu Thr Glu Thr Glu Asn Leu Glu Thr Glu Asp

195                 200                 205

Asp Asn Asn Glu Glu Val Glu Glu Asn Val Glu Glu Asn Asp Asp Glu
    210                 215                 220

Ser Val Ala Ser Ser Val Glu Glu Ser Ile Ala Ser Ser Val Glu Glu
225                 230                 235                 240

Ser Val Ala Glu Asn Val Glu Glu Ser Val Ala Glu Asn Val Glu Glu
                245                 250                 255

Ser Val Ala Glu Asn Asp Asn Val Asn Ser Val Thr Gln Arg Gly Asn
            260                 265                 270

Asn Asn Tyr Asn Asn Asn Leu Glu Arg Gly Leu Gly Ser Gly Ala Leu
        275                 280                 285

Pro Gly Thr Asn Ile Ile Thr Glu Glu Lys Tyr Ser Leu Glu Leu Ile
    290                 295                 300

Lys Leu Thr Ser Lys Asp Glu Glu Asp Ile Ile Lys His Asn Glu Asp
305                 310                 315                 320

Val Arg Glu Glu Ile Glu Glu Gln Gln Glu Asp Ile Glu Val Glu Glu
                325                 330                 335

Asn Val Glu Glu Asn Asp Asp Glu Ser Val Ala Ser Ser Val Glu Glu
            340                 345                 350

Ser Ile Ala Ser Ser Val Glu Glu Ser Val Ala Glu Asn Val Glu Glu
        355                 360                 365

Ser Val Ala Glu Asn Val Glu Glu Ser Val Ala Glu Asn Pro Glu Gly
    370                 375                 380

Pro Arg Ala Asn Asn Arg Asn Glu Asn Asn Gln Asn Thr Asp Pro Tyr
385                 390                 395                 400

Asn His Tyr Phe Ala Trp Glu Ile Gly Gly Gly Ala Pro Thr Tyr Lys
                405                 410                 415

Pro Glu Asn Asn Lys Asn Asp Asn Ile Leu Leu Glu His Val Lys Ile
            420                 425                 430

Thr Ser Trp Asp Lys Glu Asp Ile Ile Lys Glu Asn Glu Asp Thr Lys
        435                 440                 445

Arg Glu Val Gln Glu
    450

<210>  63

<211>  1002
<212>  DNA
<213>  Artificial

<220>
<223>  Polypeptide


<220>
<221>  misc_feature
<222>  (1)..(1002)
<223>  construct 5

<220>
<221>  CDS
<222>  (1)..(1002)

<400>  63
gca aaa gaa gct tct agt tat gat tat att tta ggt tgg gaa ttt gga      48
Ala Lys Glu Ala Ser Ser Tyr Asp Tyr Ile Leu Gly Trp Glu Phe Gly
1               5                   10                  15

gga ggc gtt cca gaa cac aaa aaa gaa gaa aat atg tta tca cat tta      96
Gly Gly Val Pro Glu His Lys Lys Glu Glu Asn Met Leu Ser His Leu
            20                  25                  30

tat gtt tct tca aag gat aag gaa aat ata tct aag gaa aat gat gat      144
Tyr Val Ser Ser Lys Asp Lys Glu Asn Ile Ser Lys Glu Asn Asp Asp
        35                  40                  45

gta tta gat gag aag gaa gaa gag gca gaa gaa aca gaa gaa gaa gaa      192
Val Leu Asp Glu Lys Glu Glu Glu Ala Glu Glu Thr Glu Glu Glu Glu
    50                  55                  60

ctt gaa gaa aaa tct gaa aca aat aaa aat cct act tct cat agt aat      240
Leu Glu Glu Lys Ser Glu Thr Asn Lys Asn Pro Thr Ser His Ser Asn
65                  70                  75                  80

agt act aca act tct ctg aat aat aat ata ctt gga tgg gaa ttt gga      288
Ser Thr Thr Thr Ser Leu Asn Asn Asn Ile Leu Gly Trp Glu Phe Gly
                85                  90                  95

gga ggt gct cct caa aat gga gct gca gaa gat aaa aag aca gaa tat      336
Gly Gly Ala Pro Gln Asn Gly Ala Ala Glu Asp Lys Lys Thr Glu Tyr
            100                 105                 110

tta cta gaa caa ata aaa att cca tca tgg gat aga aat aac atc ccc      384
Leu Leu Glu Gln Ile Lys Ile Pro Ser Trp Asp Arg Asn Asn Ile Pro
        115                 120                 125

gat gag aat gaa caa gta ata gag gac cct caa gaa gat aat aaa gat      432
Asp Glu Asn Glu Gln Val Ile Glu Asp Pro Gln Glu Asp Asn Lys Asp
    130                 135                 140

gaa gat gaa gat gaa gaa aca gaa aca gaa aat ttg gaa aca gaa gat      480
Glu Asp Glu Asp Glu Glu Thr Glu Thr Glu Asn Leu Glu Thr Glu Asp
145                 150                 155                 160

gat aat aat gaa gag tat gag aag aaa aat gaa aat aaa aat gta tca      528
Asp Asn Asn Glu Glu Tyr Glu Lys Lys Asn Glu Asn Lys Asn Val Ser
                165                 170                 175

aat gta gat tca aaa aca aaa tca aat gaa aaa gga aga cct cct aca      576
Asn Val Asp Ser Lys Thr Lys Ser Asn Glu Lys Gly Arg Pro Pro Thr
            180                 185                 190

tat tct cct att ctg gat gat ggg ata gaa ttt agt ggt ggt tta tat      624
Tyr Ser Pro Ile Leu Asp Asp Gly Ile Glu Phe Ser Gly Gly Leu Tyr

195                 200                 205

ttt aat gag aaa aag tcg act gaa gaa aat aaa caa aaa aat gtt tta          672
Phe Asn Glu Lys Lys Ser Thr Glu Glu Asn Lys Gln Lys Asn Val Leu
    210                 215                 220

gaa tca gta aat tta aca tcg tgg gat aaa gag gat att gtt aag gaa          720
Glu Ser Val Asn Leu Thr Ser Trp Asp Lys Glu Asp Ile Val Lys Glu
225                 230                 235                 240

aat gag gat gtt aaa cat gaa agt aat gtt ggt agc atc caa gaa gta          768
Asn Glu Asp Val Lys His Glu Ser Asn Val Gly Ser Ile Gln Glu Val
                245                 250                 255

aac caa ggt agc gtg agc gaa gaa tca cat tct aaa act ata gat cct          816
Asn Gln Gly Ser Val Ser Glu Glu Ser His Ser Lys Thr Ile Asp Pro
            260                 265                 270

tct aag att gac gac cgt ttg gaa tta agt agt ggg tca tca tct ctt          864
Ser Lys Ile Asp Asp Arg Leu Glu Leu Ser Ser Gly Ser Ser Ser Leu
        275                 280                 285

gaa caa cac tct aag gaa gat gta aaa aag gga tgt gct tta gaa ttg          912
Glu Gln His Ser Lys Glu Asp Val Lys Lys Gly Cys Ala Leu Glu Leu
    290                 295                 300

gta cct tta tct tta tcg gat att gaa cag ata gct aat gaa agc gaa          960
Val Pro Leu Ser Leu Ser Asp Ile Glu Gln Ile Ala Asn Glu Ser Glu
305                 310                 315                 320

gat gta ctg gaa gag ata gaa gaa gaa att aat aca gat tag                 1002
Asp Val Leu Glu Glu Ile Glu Glu Glu Ile Asn Thr Asp
                325                 330

<210>  64
<211>  333
<212>  PRT
<213>  Artificial

<220>
<223>  Synthetic Construct

<400>  64
   Ala Lys Glu Ala Ser Ser Tyr Asp Tyr Ile Leu Gly Trp Glu Phe Gly
   1               5                   10                  15
   Gly Gly Val Pro Glu His Lys Lys Glu Glu Asn Met Leu Ser His Leu
               20                  25                  30
   Tyr Val Ser Ser Lys Asp Lys Glu Asn Ile Ser Lys Glu Asn Asp Asp
           35                  40                  45
   Val Leu Asp Glu Lys Glu Glu Glu Ala Glu Glu Thr Glu Glu Glu Glu
       50                  55                  60
   Leu Glu Glu Lys Ser Glu Thr Asn Lys Asn Pro Thr Ser His Ser Asn
   65                  70                  75                  80
   Ser Thr Thr Thr Ser Leu Asn Asn Asn Ile Leu Gly Trp Glu Phe Gly
                   85                  90                  95
   Gly Gly Ala Pro Gln Asn Gly Ala Ala Glu Asp Lys Lys Thr Glu Tyr
               100                 105                 110
   Leu Leu Glu Gln Ile Lys Ile Pro Ser Trp Asp Arg Asn Asn Ile Pro
           115                 120                 125
   Asp Glu Asn Glu Gln Val Ile Glu Asp Pro Gln Glu Asp Asn Lys Asp
       130                 135                 140
   Glu Asp Glu Asp Glu Glu Thr Glu Thr Glu Asn Leu Glu Thr Glu Asp
   145                 150                 155                 160
   Asp Asn Asn Glu Glu Tyr Glu Lys Lys Asn Glu Asn Lys Asn Val Ser
                   165                 170                 175
   Asn Val Asp Ser Lys Thr Lys Ser Asn Glu Lys Gly Arg Pro Pro Thr
               180                 185                 190
   Tyr Ser Pro Ile Leu Asp Asp Gly Ile Glu Phe Ser Gly Gly Leu Tyr
           195                 200                 205
   Phe Asn Glu Lys Lys Ser Thr Glu Glu Asn Lys Gln Lys Asn Val Leu
       210                 215                 220
   Glu Ser Val Asn Leu Thr Ser Trp Asp Lys Glu Asp Ile Val Lys Glu
   225                 230                 235                 240
   Asn Glu Asp Val Lys His Glu Ser Asn Val Gly Ser Ile Gln Glu Val
                   245                 250                 255
   Asn Gln Gly Ser Val Ser Glu Glu Ser His Ser Lys Thr Ile Asp Pro
               260                 265                 270
   Ser Lys Ile Asp Asp Arg Leu Glu Leu Ser Ser Gly Ser Ser Ser Leu
           275                 280                 285
   Glu Gln His Ser Lys Glu Asp Val Lys Lys Gly Cys Ala Leu Glu Leu
       290                 295                 300
   Val Pro Leu Ser Leu Ser Asp Ile Glu Gln Ile Ala Asn Glu Ser Glu
   305                 310                 315                 320
   Asp Val Leu Glu Glu Ile Glu Glu Glu Ile Asn Thr Asp
                   325                 330

<210>  65
<211>  714
<212>  DNA
<213>  Artificial

<220>
<223>  Polypeptide

<220>
<221>  misc_feature
<222>  (1)..(714)
<223>  construct 6

<220>
<221>  CDS
<222>  (1)..(714)

<400>  65
gca aaa gaa gct tct agt tat gat tat att tta ggt tgg gaa ttt gga        48
Ala Lys Glu Ala Ser Ser Tyr Asp Tyr Ile Leu Gly Trp Glu Phe Gly
1               5                   10                  15

gga ggc gtt cca gaa cac aaa aaa gaa gaa aat atg tta tca cat tta        96
Gly Gly Val Pro Glu His Lys Lys Glu Glu Asn Met Leu Ser His Leu
            20                  25                  30

tat gtt tct tca aag gat aag gaa aat ata tct aag gaa aat gat gat        144
Tyr Val Ser Ser Lys Asp Lys Glu Asn Ile Ser Lys Glu Asn Asp Asp
        35                  40                  45

gta tta gat gag aag gaa gaa gag gca gaa gaa aca gaa gaa gaa gaa        192
Val Leu Asp Glu Lys Glu Glu Glu Ala Glu Glu Thr Glu Glu Glu Glu
    50                  55                  60

ctt gaa gaa aaa tct gaa aca aat aaa aat cct act tct cat agt aat        240
Leu Glu Glu Lys Ser Glu Thr Asn Lys Asn Pro Thr Ser His Ser Asn
65                  70                  75                  80

agt act aca act tct ctg aat aat aat ata ctt gga tgg gaa ttt gga        288
Ser Thr Thr Thr Ser Leu Asn Asn Asn Ile Leu Gly Trp Glu Phe Gly
                85                  90                  95

gga ggt gct cct caa aat gga gct gca gaa gat aaa aag aca gaa tat        336
Gly Gly Ala Pro Gln Asn Gly Ala Ala Glu Asp Lys Lys Thr Glu Tyr
            100                 105                 110

tta cta gaa caa ata aaa att cca tca tgg gat aga aat aac atc ccc        384
Leu Leu Glu Gln Ile Lys Ile Pro Ser Trp Asp Arg Asn Asn Ile Pro
        115                 120                 125

gat gag aat gaa caa gta ata gag gac cct caa gaa gat aat aaa gat        432
Asp Glu Asn Glu Gln Val Ile Glu Asp Pro Gln Glu Asp Asn Lys Asp
    130                 135                 140

gaa gat gaa gat gaa gaa aca gaa aca gaa aat ttg gaa aca gaa gat        480
Glu Asp Glu Asp Glu Glu Thr Glu Thr Glu Asn Leu Glu Thr Glu Asp
145                 150                 155                 160

gat aat aat gaa gag cct gaa gga cca aga gca aac aat aga aat gaa        528
Asp Asn Asn Glu Glu Pro Glu Gly Pro Arg Ala Asn Asn Arg Asn Glu
                165                 170                 175

aat aat caa aat aca gat cca tat aac cac tat ttt gca tgg gaa att        576
Asn Asn Gln Asn Thr Asp Pro Tyr Asn His Tyr Phe Ala Trp Glu Ile
            180                 185                 190

gga ggt ggt gct cca acg tat aaa ccc gag aac aat aag aac gat aat        624
Gly Gly Gly Ala Pro Thr Tyr Lys Pro Glu Asn Asn Lys Asn Asp Asn
        195                 200                 205

att ttg cta gaa cac gta aaa att acc tcg tgg gat aaa gaa gat ata        672
Ile Leu Leu Glu His Val Lys Ile Thr Ser Trp Asp Lys Glu Asp Ile
    210                 215                 220

att aaa gaa aat gaa gac aca aaa cgc gaa gtt caa gaa tag                    714
Ile Lys Glu Asn Glu Asp Thr Lys Arg Glu Val Gln Glu
225                 230                 235

<210>  66
<211>  237
<212>  PRT
<213>  Artificial

<220>
<223>  Synthetic Construct

<400>  66

Ala Lys Glu Ala Ser Ser Tyr Asp Tyr Ile Leu Gly Trp Glu Phe Gly
1               5                   10                  15

Gly Gly Val Pro Glu His Lys Lys Glu Glu Asn Met Leu Ser His Leu
            20                  25                  30

Tyr Val Ser Ser Lys Asp Lys Glu Asn Ile Ser Lys Glu Asn Asp Asp
        35                  40                  45

Val Leu Asp Glu Lys Glu Glu Glu Ala Glu Glu Thr Glu Glu Glu Glu
    50                  55                  60

Leu Glu Glu Lys Ser Glu Thr Asn Lys Asn Pro Thr Ser His Ser Asn
65                  70                  75                  80

Ser Thr Thr Thr Ser Leu Asn Asn Asn Ile Leu Gly Trp Glu Phe Gly
                85                  90                  95

Gly Gly Ala Pro Gln Asn Gly Ala Ala Glu Asp Lys Lys Thr Glu Tyr
            100                 105                 110

Leu Leu Glu Gln Ile Lys Ile Pro Ser Trp Asp Arg Asn Asn Ile Pro
        115                 120                 125

Asp Glu Asn Glu Gln Val Ile Glu Asp Pro Gln Glu Asp Asn Lys Asp
    130                 135                 140

Glu Asp Glu Asp Glu Glu Thr Glu Thr Glu Asn Leu Glu Thr Glu Asp
145                 150                 155                 160

Asp Asn Asn Glu Glu Pro Glu Gly Pro Arg Ala Asn Asn Arg Asn Glu
                165                 170                 175

Asn Asn Gln Asn Thr Asp Pro Tyr Asn His Tyr Phe Ala Trp Glu Ile
            180                 185                 190

Gly Gly Gly Ala Pro Thr Tyr Lys Pro Glu Asn Asn Lys Asn Asp Asn
        195                 200                 205

Ile Leu Leu Glu His Val Lys Ile Thr Ser Trp Asp Lys Glu Asp Ile

210                     215                     220

Ile Lys Glu Asn Glu Asp Thr Lys Arg Glu Val Gln Glu
225                 230                 235

<210>  67
<211>  498
<212>  DNA
<213>  Artificial

<220>
<223>  Polypeptide

<220>
<221>  CDS
<222>  (1)..(498)

<220>
<221>  misc_feature
<222>  (1)..(498)
<223>  construct 7

<400>  67
gca aaa gaa gct tct agt tat gat tat att tta ggt tgg gaa ttt gga      48
Ala Lys Glu Ala Ser Ser Tyr Asp Tyr Ile Leu Gly Trp Glu Phe Gly
1               5                   10                  15

gga ggc gtt cca gaa cac aaa aaa gaa gaa aat atg tta tca cat tta      96
Gly Gly Val Pro Glu His Lys Lys Glu Glu Asn Met Leu Ser His Leu
            20                  25                  30

tat gtt tct tca aag gat aag gaa aat ata tct aag gaa aat gat gat      144
Tyr Val Ser Ser Lys Asp Lys Glu Asn Ile Ser Lys Glu Asn Asp Asp
        35                  40                  45

gta tta gat gag aag gaa gaa gag gca gaa gaa aca gaa gaa gaa gaa      192
Val Leu Asp Glu Lys Glu Glu Glu Ala Glu Glu Thr Glu Glu Glu Glu
    50                  55                  60

ctt gaa gaa aaa tct gaa aca aat aaa aat cct act tct cat agt aat      240
Leu Glu Glu Lys Ser Glu Thr Asn Lys Asn Pro Thr Ser His Ser Asn
65                  70                  75                  80

agt act aca act tct ctg aat aat aat ata ctt gga tgg gaa ttt gga      288
Ser Thr Thr Thr Ser Leu Asn Asn Asn Ile Leu Gly Trp Glu Phe Gly
                85                  90                  95

gga ggt gct cct caa aat gga gct gca gaa gat aaa aag aca gaa tat      336
Gly Gly Ala Pro Gln Asn Gly Ala Ala Glu Asp Lys Lys Thr Glu Tyr
            100                 105                 110

tta cta gaa caa ata aaa att cca tca tgg gat aga aat aac atc ccc      384
Leu Leu Glu Gln Ile Lys Ile Pro Ser Trp Asp Arg Asn Asn Ile Pro
        115                 120                 125

gat gag aat gaa caa gta ata gag gac cct caa gaa gat aat aaa gat      432
Asp Glu Asn Glu Gln Val Ile Glu Asp Pro Gln Glu Asp Asn Lys Asp
    130                 135                 140

gaa gat gaa gat gaa gaa aca gaa aca gaa aat ttg gaa aca gaa gat      480
Glu Asp Glu Asp Glu Glu Thr Glu Thr Glu Asn Leu Glu Thr Glu Asp
145                 150                 155                 160

gat aat aat gaa gag tag                                              498
Asp Asn Asn Glu Glu

                165

<210>  68
<211>  165
<212>  PRT
<213>  Artificial

<220>
<223>  Synthetic Construct

<400>  68

Ala Lys Glu Ala Ser Ser Tyr Asp Tyr Ile Leu Gly Trp Glu Phe Gly
1               5                   10                  15

Gly Gly Val Pro Glu His Lys Lys Glu Glu Asn Met Leu Ser His Leu
            20                  25                  30

Tyr Val Ser Ser Lys Asp Lys Glu Asn Ile Ser Lys Glu Asn Asp Asp
        35                  40                  45

Val Leu Asp Glu Lys Glu Glu Glu Ala Glu Glu Thr Glu Glu Glu Glu
    50                  55                  60

Leu Glu Glu Lys Ser Glu Thr Asn Lys Asn Pro Thr Ser His Ser Asn
65                  70                  75                  80

Ser Thr Thr Thr Ser Leu Asn Asn Asn Ile Leu Gly Trp Glu Phe Gly
                85                  90                  95

Gly Gly Ala Pro Gln Asn Gly Ala Ala Glu Asp Lys Lys Thr Glu Tyr
            100                 105                 110

Leu Leu Glu Gln Ile Lys Ile Pro Ser Trp Asp Arg Asn Asn Ile Pro
        115                 120                 125

Asp Glu Asn Glu Gln Val Ile Glu Asp Pro Gln Glu Asp Asn Lys Asp
    130                 135                 140

Glu Asp Glu Asp Glu Glu Thr Glu Thr Glu Asn Leu Glu Thr Glu Asp
145                 150                 155                 160

Asp Asn Asn Glu Glu
                165


<210>  69
<211>  531
<212>  DNA
<213>  Artificial

<220>
<223>  Polypeptide


<220>
<221>  misc_feature

<222>  (1)..(531)
<223>  construct 8

<220>
<221>  CDS
<222>  (1)..(531)

<400>  69
tct gaa aca aat aaa aat cct act tct cat agt aat agt act aca act      48
Ser Glu Thr Asn Lys Asn Pro Thr Ser His Ser Asn Ser Thr Thr Thr
1               5                   10                  15

tct ctg aat aat aat ata ctt gga tgg gaa ttt gga gga ggt gct cct      96
Ser Leu Asn Asn Asn Ile Leu Gly Trp Glu Phe Gly Gly Gly Ala Pro
            20                  25                  30

caa aat gga gct gca gaa gat aaa aag aca gaa tat tta cta gaa caa      144
Gln Asn Gly Ala Ala Glu Asp Lys Lys Thr Glu Tyr Leu Leu Glu Gln
        35                  40                  45

ata aaa att cca tca tgg gat aga aat aac atc ccc gat gag aat gaa      192
Ile Lys Ile Pro Ser Trp Asp Arg Asn Asn Ile Pro Asp Glu Asn Glu
    50                  55                  60

caa gta ata gag gac cct caa gaa gat aat aaa gat gaa gat gaa gat      240
Gln Val Ile Glu Asp Pro Gln Glu Asp Asn Lys Asp Glu Asp Glu Asp
65                  70                  75                  80

gaa gaa aca gaa aca gaa aat ttg gaa aca gaa gat gat aat aat gaa      288
Glu Glu Thr Glu Thr Glu Asn Leu Glu Thr Glu Asp Asp Asn Asn Glu
                85                  90                  95

gag gaa aaa aaa cca gac aat gaa att aca aat gaa gtt aaa gag gaa      336
Glu Glu Lys Lys Pro Asp Asn Glu Ile Thr Asn Glu Val Lys Glu Glu
            100                 105                 110

caa aaa tat agt tca cca agt gat ata aat gcc caa aat tta att tct      384
Gln Lys Tyr Ser Ser Pro Ser Asp Ile Asn Ala Gln Asn Leu Ile Ser
        115                 120                 125

aat aag aat aaa aag aat gat gaa aca aaa aag act gct gaa aat ata      432
Asn Lys Asn Lys Lys Asn Asp Glu Thr Lys Lys Thr Ala Glu Asn Ile
    130                 135                 140

gtt aaa aca ttg gtt gga tta ttt aat gaa aaa aat gag ata gat tct      480
Val Lys Thr Leu Val Gly Leu Phe Asn Glu Lys Asn Glu Ile Asp Ser
145                 150                 155                 160

act ata aat aat tta gta caa gaa atg atc cat cta ttt agt aat aat      528
Thr Ile Asn Asn Leu Val Gln Glu Met Ile His Leu Phe Ser Asn Asn
                165                 170                 175

tag                                                                  531


<210>  70
<211>  176
<212>  PRT
<213>  Artificial

<220>
<223>  Synthetic Construct

<400>  70

Ser Glu Thr Asn Lys Asn Pro Thr Ser His Ser Asn Ser Thr Thr Thr
1               5                   10                  15

Ser Leu Asn Asn Asn Ile Leu Gly Trp Glu Phe Gly Gly Gly Ala Pro
            20                  25                  30

Gln Asn Gly Ala Ala Glu Asp Lys Lys Thr Glu Tyr Leu Leu Glu Gln
        35                  40                  45

Ile Lys Ile Pro Ser Trp Asp Arg Asn Asn Ile Pro Asp Glu Asn Glu
    50                  55                  60

Gln Val Ile Glu Asp Pro Gln Glu Asp Asn Lys Asp Glu Asp Glu Asp
65                  70                  75                  80

Glu Glu Thr Glu Thr Glu Asn Leu Glu Thr Glu Asp Asp Asn Asn Glu
                85                  90                  95

Glu Glu Lys Lys Pro Asp Asn Glu Ile Thr Asn Glu Val Lys Glu Glu
            100                 105                 110

Gln Lys Tyr Ser Ser Pro Ser Asp Ile Asn Ala Gln Asn Leu Ile Ser
        115                 120                 125

Asn Lys Asn Lys Lys Asn Asp Glu Thr Lys Lys Thr Ala Glu Asn Ile
    130                 135                 140

Val Lys Thr Leu Val Gly Leu Phe Asn Glu Lys Asn Glu Ile Asp Ser
145                 150                 155                 160

Thr Ile Asn Asn Leu Val Gln Glu Met Ile His Leu Phe Ser Asn Asn
                165                 170                 175

<210>  71
<211>  386
<212>  PRT
<213>  Artificial

<220>
<223>  Polypeptide


<220>
<221>  MISC_FEATURE
<222>  (1)..(386)
<223>  poly-bcd construct used in examples 1 and 2

<400>  71

Ala Lys Glu Ala Ser Ser Tyr Asp Tyr Ile Leu Gly Trp Glu Phe Gly
1               5                   10                  15

Gly Gly Val Pro Glu His Lys Lys Glu Glu Asn Met Leu Ser His Leu
            20                  25                  30

Tyr Val Ser Ser Lys Asp Lys Glu Asn Ile Ser Lys Glu Asn Asp Asp
        35                  40                  45

Val Leu Asp Glu Lys Glu Glu Glu Ala Glu Glu Thr Glu Glu Glu Glu
    50                  55                  60

Leu Glu Glu Lys Leu Asn Asn Asn Ile Leu Gly Trp Glu Phe Gly Gly
65                  70                  75                  80

Gly Ala Pro Gln Asn Gly Ala Ala Glu Asp Lys Lys Thr Glu Tyr Leu
                85                  90                  95

Leu Glu Gln Ile Lys Ile Pro Ser Trp Asp Arg Asn Asn Ile Pro Asp
            100                 105                 110

Glu Asn Glu Gln Val Ile Glu Asp Pro Gln Glu Asp Asn Lys Asp Glu
        115                 120                 125

Asp Glu Asp Glu Glu Thr Glu Thr Glu Asn Leu Glu Thr Glu Asp Asp
    130                 135                 140

Asn Asn Glu Glu Ser Asn Glu Lys Gly Arg Pro Pro Thr Tyr Ser Pro
145                 150                 155                 160

Ile Leu Asp Asp Gly Ile Glu Phe Ser Gly Gly Leu Tyr Phe Asn Glu
                165                 170                 175

Lys Lys Ser Thr Glu Glu Asn Lys Gln Lys Asn Val Leu Glu Ser Val
            180                 185                 190

Asn Leu Thr Ser Trp Asp Lys Glu Asp Ile Val Lys Glu Asn Glu Asp
        195                 200                 205

Val Lys Leu Glu Arg Gly Leu Gly Ser Gly Ala Leu Pro Gly Thr Asn
    210                 215                 220

Ile Ile Thr Glu Glu Lys Tyr Ser Leu Glu Leu Ile Lys Leu Thr Ser
225                 230                 235                 240

Lys Asp Glu Glu Asp Ile Ile Lys His Asn Glu Asp Val Arg Glu Glu
                245                 250                 255

Ile Glu Glu Gln Gln Glu Asp Ile Glu Tyr Asn His Tyr Phe Ala Trp
            260                 265                 270

Glu Ile Gly Gly Gly Ala Pro Thr Tyr Lys Pro Glu Asn Asn Lys Asn
        275                 280                 285

Asp Asn Ile Leu Leu Glu His Val Lys Ile Thr Ser Trp Asp Lys Glu
    290                 295                 300

Asp Ile Ile Lys Glu Asn Glu Asp Thr Lys Arg Glu Val Gln Glu Ser
305                 310                 315                 320

Lys Thr Ile Asp Pro Ser Lys Ile Asp Asp Arg Leu Glu Leu Ser Ser
                325                 330                 335

Gly Ser Ser Ser Leu Glu Gln His Ser Lys Glu Asp Val Lys Lys Gly
            340                 345                 350

Cys Ala Leu Glu Leu Val Pro Leu Ser Leu Ser Asp Ile Glu Gln Ile
        355                 360                 365

Ala Asn Glu Ser Glu Asp Val Leu Glu Glu Ile Glu Glu Glu Ile Asn
    370                 375                 380

Thr Asp
385

## Description

The present invention relates to the protection against malaria and to polypeptides suitable in this respect. More particularly, the invention pertains to particular polypeptides, especially chimeric one, which are suitable for the prevention or treatment of malaria. Said polypeptides are derived from particular motifs of the C-terminal region of *Plasmodium* MSP-3 proteins.

The family of genes encompassing the MSP Merozoite Surface Protein (MSP) -3 gene (now designated MSP3-1), shows exceptional redundancy of exposed epitopes, which suggests that this family of genes plays an important part in the immunogenicity of the parasite. Immunogenicity and antigenicity analyses of proteins of the MSP-3 family enable the definition of novel immunogenic polypeptides which are suitable for use as a vaccine against *Plasmodium falciparum (P. falciparum).*

The parasites responsible for malaria in human, including especially P. *falciparum,* exhibit different morphologies in the human host and express different antigens as a function of their localization in the organism of the infected host. The morphological and antigenic differences of these parasites during their life cycle in man enable at least four distinct stages of development to be defined.

The very first stage of development of the parasite in man corresponds to the sporozoite form introduced into the blood of the host by bites of insect vectors of the parasite. The second stage corresponds to the passage of the parasite into the liver and to the infection of the hepatic cells in which the parasites develop to form the hepatic schizonts which, when they are mature (for example, in the case of *P. falciparum* on the 6^{th} day after penetration of the sporozoites) release hepatic merozoites by bursting. The third stage is characterized by the infection of the blood erythrocytes by the asexual forms (merozoites) of the parasite; this erythrocytic stage of development corresponds to the pathogenic phase of the disease. The fourth stage corresponds to the formation of the forms with sexual potential (or gametocytes) which will become extracellular sexual forms or gametes in the mosquito.

Antibodies have been repeatedly shown to play an important part in the development of clinical immunity to *P. falciparum* malaria. Numerous immunological studies now suggest that human antibodies of the cytophilic subclasses (lgG1 and lgG3) are particularly critical to the state of premunition. This anti-parasite immunity is a strain-independent, non-sterilizing type of immunity which is acquired after lengthy exposure (15-20 years) to the parasite. It is commonly observed in Africa and in Papua-New Guinea but it has only recently been documented in S-E Asia (Soe, Khin Saw et al. 2001). Although antibodies can act directly upon merozoite invasion of red blood cells, the most efficient *in vivo* mechanism for antibody-mediated parasite control in endemic areas requires the participation of monocytes (Khusmith and Druilhe 1983); (Lunel and Druilhe 1989). The antibody-dependent cellular inhibition (ADCI) assay mimics this cooperation between monocytes and cytophilic parasite-specific antibodies and appears today as the best *in vitro* surrogate marker of acquired immunity against *P. falciparum* blood stages.

Two molecules have so far been identified as targets of ADCI-effective human antibodies, namely the 48-kDa Merozoite surface-protein 3, - hereafter designated as *MSP-3 -* (Oeuvray, Bouharoun-Tayoun et al. 1994) and the 220-kDa Glutamate-rich protein, - hereafter designated as *GLURP -* (Theisen, Soe et al. 1998). It has also been shown that GLURP and MSP-3 can inhibit parasite growth *in vivo* by passive transfer in *P. falciparum-*humanized SCID mice (Badell, Oeuvray et al. 2000). The association of human antibodies against MSP-3 with clinical protection is also indicated by a number of immuno-epidemiological studies, which demonstrate that the levels of MSP-3 specific cytophilic antibodies (IgG1 and IgG3) are significantly associated with a reduced risk of malaria attacks (Roussilhon 1999). These studies have further shown that cytophilic IgG3 antibodies play a major part in protection against malaria, hence bringing epidemiological support to the concept that antibodies against MSP-3 can actively control parasite multiplication *in vivo* by cooperation with cells bearing Fcγ II receptors (Bouharoun-Tayoun, Oeuvray et al. 1995). These receptors display higher affinity for the IgG3 subclass than for the IgG1 subclass (Pleass and Woof 2001). The major B-cell epitopes recognized by these human IgG antibodies have been localized to conserved sequences in the MSP-3₂₁₂₋₂₅₇ region (Oeuvray, Bouharoun-Tayoun et al. 1994; Theisen, Soe et al. 2000; Theisen, Dodoo et al. 2001). Nucleotide-sequencing have demonstrated that these important epitopes are highly conserved among a number of *P. falciparum* laboratory lines and field isolates from Africa and Asia (Huber, Felger et al. 1997); (McColl and Anders 1997).

The inventors previously characterized a series of 9 *P. falciparum* genes, all clustered at the 3' terminus of chromosome 10. This chromosome indeed contains a series of 9 open reading frames, separated by non coding regions, and comprises in a row (5' - 3') genes encoding proteins denominated first GLURP, followed at 1300 base-pairs by MSP-3 (now denominated MSP-3-1) followed by 7 other genes denominated MSP-3-2 (also designated sometimes MSP6), MSP-3-3, MSP-3-4, MSP-3-5, MSP-3-6, MSP-3-7, MSP-3-8.

Besides being clustered in the same chromosomal region, those 9 genes have outstanding features, which indicate that they are privileged products for vaccine development against *P. falciparum* blood stage infection:
It was shown that all 9 genes were expressed simultaneously in all parasites studied, i.e., that the corresponding proteins could be detected in *P. falciparum* erythrocytic stages and are all located on the merozoite surface. This was previously shown for GLURP, MSP-3-1 and MSP-3-2 (designated "MSP6" by (Trucco, Fernandez-Reyes et al. 2001) and has been further demonstrated for the remaining by the construction of particular sequences in the N-terminus of those genes which are unique for each of them, which do not share cross-reactive epitopes, and which corresponding antibodies all react with the merozoite surface. Moreover, transcription was demonstrated by RT-PCR with unique primers specific of each.

These 9 *P. falciparum* genes encode proteins and epitopes within which are all targets for naturally occuring antibodies in malaria exposed individuals, mediating *P. falciparum* erythrocytic stage killing by cooperation with blood monocytes, and which exhibit an unusual degree of sequence conservation among various *P. falciparum* isolates.

Moreover, proteins of the MSP3-like family have common structural and immunological features. In particular they share an initial N-terminus "signature" of 4 aminoacids identical in each of them and identical to similar MSP3 homologous proteins described in *Plasmodium vivax* and *Plasmodium Knowlesi.*

In addition, MSP3-1, MSP3-2, MSP3-3, MSP3-4, MSP3-7 and MSP3-8 are also characterized by the fact that they have a conserved C-terminal sequence which encodes epitopes, especially T-epitopes which are conserved among the genes of the family and wherein said terminal sequence further comprises divergences in codons in regions outside of the epitopes which divergences are conserved among the genes of the MSP3 family.

The inventors have also previously demonstrated that there exists immunological cross-reactivity between the different proteins of the MSP-3 family, as a consequence of those structural homologies between members of the gene family.

The inventors have now designed novel vaccine constructs derived from the MSP3 gene family, based on knowledge of antigenicity and immunogenicity and have assessed their properties and immunogenicity in preclinical models; the detailed analysis of the antigenicity in humans exposed from endemic areas of six proteins belonging to the MSP3 family (MSP3-1, MSP3-2, MSP3-3, MSP3-4, MSP3-7 and MSP3-8), using six recombinant proteins, as well as immunogenicity studies with various constructs derived from the MSP-1 protein, has helped designing the polypeptides of the invention.

The present invention relates to polypeptides, especially chimeric polypeptides comprising particular epitopes derived from one or several *Plasmodium* proteins chosen among MSP3-1, MSP3-2, MSP3-3, MSP3-4, MSP3-7 and/or MSP3-8.

Other important aspects of the invention are immunogenic compositions and vaccines against malaria, comprising as an immunogen a polypeptide, especially a chimeric polypeptide of the invention.

Recombinant antibodies and part thereof, which cross-react with several polypeptides, especially chimeric polypeptides of the invention, also constitute an object of the present invention, either taken as such or in a medicament for passive immunotherapy or in a kit for the *in vitro* diagnosis of malaria.

The invention also concerns methods for the *in vitro* diagnosis of malaria in an individual, either by using a polypeptide of the invention, or by using an antibody as defined herein, as well as kits comprising at least part of the necessary reagents (polypeptide(s) of the invention, antibodies ...) for performing these methods.

Of course, a polynucleotide coding for a polypeptide of the invention as well as a cloning and/or expression vector comprising said polynucleotide, and their use in a medicament or a nucleic acid vaccine against *Plasmodium,* are also part of the invention.

Throughout the present text, a number of terms are used, that should be understood according to the following definitions:
By "chimeric polypeptide", "multigenic poplypeptide", "polyprotein" or "recombinant polypeptide", it is meant herein a polypeptide (in particular a protein), i.e., a structure having a single chain of consecutive amino acid residues, which is composed of a plurality of protein components that, while typically physically contained in distinct entities in their native state, are joined by the respective amino and carboxy termini through a peptide linkage to form a single continuous amino acid chain of a polypeptide. Hence the term "chimeric polypeptide" as used herein does not encompass full-length *Plasmodium* MSP3 native proteins. Especially, the chimeric polypeptide of the invention does not consist of and, preferably, does not comprise, native MSP3-1, MSP3-2, MSP3-3, MSP3-4, MSP3-7or MSP3-8 proteins.

The chimeric polypeptide of the invention can be in particular a fusion polypeptide, i.e., a polyueptide obtained by the expression of a recombinant genetic construct prepared by joining together at least two coding sequences with their reading frame in phase. The coding sequences joined together can be adjacent or separated by one or several linker(s).

"Plurality" in this context means at least two protein components, including two polypeptide fragments, and in particular two structural motifs as defined herein. Preferred embodiments generally consist of at least 6 up to 26 motifs as defined herein, although more may be used. It will be appreciated that the protein components can be joined (i.e., linked) directly through peptide bonds or joined through a peptide linker/spacer. In a particular embodiment of the invention, these protein components (at least some of them) are covalently joined in the polypeptide of the invention.

The polypeptide of the invention can be prepared by any appropriate preparation processes, including by processes involving recombinant expression or by chemical synthesis, or by a combination of these technologies.

According to the present invention, a "motif a, b, c, d, e or f of the C-terminal sequence of a *P. falciparum* MSP3 protein" is a specific structure having an amino acid sequence of 22 to 62 amino acids, which consists of a sequence chosen among SEQ ID Nos.: 1 to 36 as shown in table 2.

Further definitions are provided in the following text, when necessary.

The isolation and characterization of the various genes of the MSP3-like family has been significant for the comprehension of immunological response and for the design of means having improved interest for the preparation of immunogenic compositions or protective compositions, against *Plasmodium,* especially against *P. falciparum.*

The inventors have designed particular polypeptides and especially particular polypeptides which are safe and higly immunogenic in preclinical models. These constructs are especially able to induce a strong cytophilic antibody response in preclinical models. In addition, the antibodies elicited by those constructs display a large network of cross -reactivity across all 6 members of the MSP3 family (MSP3-1, MSP3-2, MSP3-3, MSP3-4, MSP3-7 and MSP3-8).

Hence, a first aspect of the invention is a polypeptide which comprises or consists of at least one polypeptide fragment encompassing motifs a, b, c and d of the C-terminal region of a *Plasmodium* MSP3 protein chosen among MSP3-2, MSP3-3, MSP3-4, MSP3-7, and MSP3-8.

In a particular embodiment, the polypeptide of the invention is a chimeric polypeptide.

By "polypeptide fragment encompassing motifs a, b, c and d" of a given MSP3 protein, it is meant herein a polypeptide fragment, i.e., a fragment of one of said MSP3 protein, which comprises or consists of the amino acid sequence of said motifs a, b, c and d. The same definition applies by analogy to the other constructions of motifs and in particular to a "polypeptide fragment encompassing motifs b, c and d" or a "polypeptide fragment encompassing motif e or f or motifs e and f'.

When two motifs having an overlapping sequence in the native protein from which they are derived are encompassed within such a polypeptide, said overlapping sequence is usually present only once in the polypeptide fragment included in the polypeptide of the invention, (i.e. is not repeated in said polypeptide).

Hence, in a particular embodiment, the "polypeptide fragment encompassing motifs a, b, c and d" (or encompassing motifs b, c and d, or encompassing motifs e and f) of a given MSP3 protein comprises or consists of the amino acid sequence ranging from the first amino acid of motif a to the last amino acid of motif d (or respectively ranging from the first amino acid of motif b to the last amino acid of motif d or ranging from the first amino acid of motif e to the last amino acid of motif f), from the N-terminal end to the C-terminal end, in the MSP3 protein from which said motifs are derived.

Alternatively, in another embodiment, in a polypeptide fragment encompassing two motifs having an overlapping sequence in the native protein from which they are derived, said overlapping sequence is repeated twice or more than twice in the polypeptide of the invention, thereby allowing each motif to possibly be isolated as a structure without impacting on the integrity of another motif in the polypeptide fragment.

As used herein, the term *"Plasmodium* MSP3 protein" preferably refers to a P. *falciparum* MSP3 protein, and more particularly to a MSP3 protein of the 3D7 strain. Alternatively, this term can also refer to a MSP3 protein of another *P. Falciparum* strain, or to a *Plasmodium vivax* or *Plasmodium Knowlesi* MSP3 protein.

The polypeptide, especially chimeric polypeptide of the invention generally consists of 30 to 1000 or 30 to 700 amino acids, preferably 60 to 600 amino acids and more preferably 120 to 550, 150 to 500 or 250-500 amino acids, or any combination of the figures limiting these ranges to define other ranges.

In a particular embodiment, the polypeptide of the invention is devoid of motif a of *Plasmodium* MSP3-1, and especially devoid of motif "a" of *P. falciparum* MSP3-1, i.e., devoid of motif "a" of sequence SEQ ID NO.:1.

In a particular embodiment, the polypeptide of the invention further comprises (or consists of) a polypeptide fragment consisting of motifs b, c and d of the C-terminal region of a *Plasmodium* MSP3-1 protein. For example, the polypeptide of the invention can further comprise (or consists of) a polypeptide fragment consisting of motifs b, c and d of the C-terminal region of *P. falciparum* MSP3-1, and in particular comprise (or consists of) a polypeptide fragment consisting of the polypeptide fragment of sequence SEQ ID NO: 38.

In a particular embodiment, within the polypeptide of the invention, the polypeptide fragment or at least one of the polypeptide fragment encompassing motifs a, b, c and d of the C-terminal region of a *Plasmodium* MSP3 protein, encompass(es) (i.e., comprises or consists of) motifs a, b, c and d of the C-terminal region of a *Plasmodium* MSP3-2 protein. For example, said polypeptide fragment(s) can encompass motifs a, b, c and d of the C-terminal region of *P. falciparum* MSP3-2, and in particular encompass sequence SEQ ID NO: 40.

In a particular embodiment, the polypeptide of the invention further comprises at least one polypeptide fragment encompassing motifs a, b, c and d of the C-terminal region of a *Plasmodium* MSP-3 protein chosen among the *Plasmodium* proteins MSP3-3, MSP3-4, MSP3-7, and MSP3-8 (in particular *P. Falciparum* MSP3-3, *P. Falciparum* MSP3-4, *P. Falciparum* MSP3-7 or *P. Falciparum* MSP3-8), and/or at least one polypeptide fragment encompassing motifs e and f of the C-terminal region of a *Plasmodium* MSP-3 protein chosen among the *Plasmodium* proteins MSP3-1, MSP3-2, MSP3-3, MSP3-4, MSP3-7, and MSP3-8 (in particular *P. Falciparum* MSP3-1, *P. Falciparum* MSP3-2, *P. Falciparum* MSP3-3, *P. Falciparum* MSP3-4, *P. Falciparum* MSP3-7 or *P. Falciparum* MSP3-8). For example, the polypeptide of the invention can further comprises at least one polypeptide chosen among polypeptides of sequence SEQ ID NO: 42, SEQ ID NO: 44, SEQ ID NO: 46, SEQ ID NO: 48 and SEQ ID NO: 50.

In addition or in combination with the above-mentionned embodiment, the polypeptide of the invention can further comprise a polypeptide fragment consisting of motif e or f or motifs e and f (or fragments thereof, in particular fragments consisting of at least 10 or 20 and preferably at least 30 consecutive amino acid residues of each of said motif(s)) of the C-terminal region of a *Plasmodium* MSP-3 protein chosen among the *Plasmodium* proteins MSP3-1, MSP3-2, MSP3-3, MSP3-4, MSP3-7, and MSP3-8 (in particular *P. Falciparum* MSP3-1, *P. Falciparum* MSP3-2, *P. Falciparum* MSP3-3, *P. Falciparum* MSP3-4, *P. Falciparum* MSP3-7 or *P. Falciparum* MSP3-8). Said MSP-3 protein is preferably a MSP3 protein providing motifs a, b, c and d.

In a particular embodiment, both motif d and motif e of a given *plasmodium* MSP3 protein (e.g., a p.falciparum MSP3 protein) are encompassed within the polypeptide of the invention but said motifs d and e are not separated in said polypeptide by the amino acid sequence naturally contained between them in the MSP3 protein from which they derive. In a preferred embodiment, the amino acid sequence naturally contained between said motifs d and e in the MSP3 protein from which they derive is not present in the polypeptide of the invention.

In a particular embodiment, in the polypeptide of the invention, the polypeptide fragment or at least one of the polypeptide fragments encompassing motifs a, b, c and d of the C-terminal region of a *Plasmodium* MSP3 protein is a polypeptide fragment encompassing
- motifs a, b, c and d of the C-terminal region of a *Plasmodium* MSP3-2 protein, and in particular a polypeptide fragment encompassing sequence SEQ ID NO: 40, and,
- optionally, motif e or f or motifs e and f (or fragments thereof, in particular fragments consisting of at least 10 or 20 and preferably at least 30 consecutive amino acid residues of each of said motif(s)) of the C-terminal region of a *Plasmodium* MSP-3 protein chosen among MSP3-1, MSP3-2, MSP3-3, MSP3-4, MSP3-7, and MSP3-8 (and especially a protein providing motifs a, b, c and d).

In a particular embodiment, the polypeptide of the invention comprises or results from a combination, in a polyprotein, of:
- at least two polypeptide fragments from the C-terminal region of a *Plasmodium* MSP-3 protein chosen among MSP3-2, MSP3-3, MSP3-4, MSP3-7 and MSP3-8, wherein each polypeptide fragment consists of the succession of motifs a, b, c, and d, and optionally,
- at the C-terminal end of one or more of said polypeptide fragments the succession of motif e or f or motifs e and f (or fragments thereof, in particular fragments consisting of at least 10 or 20 and preferably at least 30 consecutive amino acid residues of each of said motif(s)) of the C-terminal region of a *Plasmodium* MSP-3 protein as defined herein (and especially a protein providing motifs a, b, c and d).

In a particular embodiment, the polypeptide of the invention consists of a combination, in a polyprotein, of polyeptide fragments described herein.

In a particular embodiment, the polypeptide of the invention, comprising or consisting of an amino acid strand of motifs selected among motifs a, b, c, d, e and f, is prepared in such a way that said motifs when present are ordered as follows, from the N-terminal end to the C-terminal end of said polypeptide:
MSP3-1 (b - c - d - e - f) - MSP3-2 (a - b - c - d - e - f) - MSP3-3 (a - b-c - d - e - f) - MSP3-4 (a - b - c - d - e - f) - MSP3-7 (a - b - c - d - e - f) - MSP3-8 (a - b - c - d - e - f).

When originating from one determined MSP3 protein, said motifs a, b, c, d, e or f are present independently of each other and when one motif a, b, c, d, e, or f is present in the polypeptide fragment of one MSP3 protein the corresponding motif of a different MSP3 protein is independently present or absent from a further polypeptide fragment, in the polypeptide.

In another embodiment, a given motif a, b, c, d, e or f of a given MSP3 protein, when present in the polypeptide of the invention, can be repeated twice or more than twice in said polypeptide.

Motifs a, b, c, d, e and f present in the polyeptide of the invention, and in particular copies of a repeated given motif, can be contiguous or not in the polypeptide of the invention.

Hence, in a particular embodiment, the polypeptide of the invention is devoid of any flanking sequences found in a native MSP3 protein upstream of motif "a" and downstream of motif "d" or when motif "e" and/or "f" are present, downstream of motif "f".

In a particular embodiment, the polypeptide of the invention further comprises one or several coil-coiled linker(s) (i.e., one or several structure(s) as defined in Lupas, 1996, or in Oakley and Hollenbeck, 2001), in particular one or several linker(s) comprising or consisting of sequence SEQ ID NO: 52 or SEQ ID NO: 54.

Said coil-coiled linker(s) can be located at any position in the polypeptide of the invention and in particular between two motifs encompassed within said polyeptide. For example, said coil-coiled linker(s) can be located between two polypeptides encompassing motifs derived from two different MSP3 proteins, e.g. between a polypeptide fragment encompassing motifs b, c and d or a, b, c and d of a first MSP3 protein and a polypeptide encompassing motifs a, b, c and d of a second MSP3 protein.

In a particular embodiment, the polypeptide of the invention comprises or consists of a polypeptide chosen among the polypeptides of sequence SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70, and especially a polypeptide chosen among the polypeptides of sequence SEQ ID NO: 56, SEQ ID NO: 58, SEQ ID NO: 64, and SEQ ID NO: 68.

In a particular embodiment of the invention, a lipidic molecule is linked to at least part of the polypeptide of the invention. An example of lipidic molecule that can be used therefore is a C-terminal palmitoylysylamide residue.

In addition, at least part of the polypeptide of the invention can be bound to a support, thereby constituting conjugates. Examples of appropriate support include viral particles, nitrocellulose, polystyrene beads, or biodegradable polymers (for example lipophosphoglycanes or poly-L lactic acid).

In a particular embodiment, the polypeptide of the invention further comprises a tag sequence, which enables purification of the polypeptide. The tag sequence preferably comprises or consists of a plurality of consecutive amino acid residues, preferably a plurality of consecutive histidine residues, for example 6 consecutive histidine residues. The tag sequence, especially the poly-histidine sequence, is preferably located at the N- or C-terminal end (more preferably the C-terminal end) of the polypeptide polypeptide of the invention.

In a particular embodiment, the polypeptide of the invention further comprises one or several sequence(s) encoded by a nucleotide sequence which is recognized by a restriction enzyme, for example one or several Bgl-II restriction site(s) (nucleotide sequence AGATCT). In particular, both ends of said polypeptide can be flanked by the same restriction site (for example a Bgl-II restriction site), or by different restriction sites.

A second object of the invention is a polynucleotide comprising or consisting of a nucleic acid sequence coding for the polypeptide of the invention.

The term "polynucleotide" as used herein means any nucleic acid molecule which comprises or consists of at least one open reading frame (in particular at least one coding sequence) encoding the polypeptide of the invention. Said polynucleotide can further comprise regulatory elements.

The polynucleotide of the invention does not consist of a sequence coding for full-length *Plasmodium* MSP3 native proteins and especially native MSP3-1, MSP3-2, MSP3-3, MSP3-4, MSP3-7 and MSP3-8 proteins. According to a particular embodiment, the polynucleotide of the invention neither comprises such a sequence coding for a full-length *Plasmodium* MSP3 native protein.

According to a particular embodiment, the polynucleotide of the invention comprises or consists in a sequence chosen in the group consisting of SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67 and SEQ ID NO: 69.

According to a preferred embodiment, the polynucleotide of the invention comprises or consists in a sequence chosen in the group consisting of SEQ ID NO: 55, SEQ ID NO: 57, SEQ ID NO: 59, SEQ ID NO: 61, SEQ ID NO: 63, SEQ ID NO: 65, SEQ ID NO: 67 and SEQ ID NO: 69, and especially a sequence chosen in the group consisting of SEQ ID NO: 55, SEQ ID NO: 57, SEQ ID NO: 63 and SEQ ID NO: 67.

The invention also relates to variants of the above defined polynucleotides, which hybridize in stringent conditions with said polynucleotides or with the complement of said polynucleotides. These variants have especially the same length as, or alternatively are shorter than the polynucleotide to which they hybridize in stringent conditions.

"Stringent hybridization conditions" are defined herein as conditions that allow specific hybridization of two nucleic acid especially two DNA molecules at about 65°C, for example in a solution of 6X SSC, 0.5% SDS, 5X Denhardt's solution and 100 µg/ml of denatured non specific DNA or any solution with an equivalent ionic strength, and after a washing step carried out at 65°C, for example in a solution of at most 0.2X SSC and 0.1 % SDS or any solution with an equivalent ionic strength. However, the stringency of the conditions can be adapted by the skilled person as a function of the size of the sequence to be hybridized, its GC nucleotide content, and any other parameter, for example following protocols described by Sambrook et al, 2001 (Molecular Cloning: A Laboratory Manual, 3rd Edition, Laboratory Press, Cold Spring Harbor, New York).

In a particular embodiment, the sequence of the polynucleotide of the invention is optimized, especially codon-optimized, for use (including for the preparation) in a host cell. For example, said polynucleotide can be codon-optimized for expression in *Escherichia coli,* or in *Lactococcus lactis.* The sequence of the polynucleotide can be optimized using the program Genscript or any other optimization program.

In a further aspect, the present invention also relates to a recombinant cloning and/or expression vector, comprising a polynucleotide insert consisting of a polynucleotide of the invention.

In a particular embodiment of the recombinant cloning and/or expression vector of the invention, the polynucleotide insert is under the control of a promoter and/or regulatory elements homologous or heterologous vis-à-vis a host cell, for expression in said host cell.

The present invention also relates to a recombinant expression vector as defined herein, for use for genetic immunisation against *Plasmodium* and in particular against *P. falciparum* and to the use of said recombinant expression vector for the preparation of a medicament for genetic immunisation against *Plasmodium* and in particular against *P. falciparum.*

A recombinant host cell, which is transformed by a recombinant expression vector of the invention, is also part of the present invention. Examples of appropriate recombinant host cells a recombinant bacterium, a recombinant yeast, a recombinant insect cell and a recombinant mammalian cell.

Another aspect of the present invention pertains to a composition, in particular an immunogenic composition, comprising or consisting of as an immunogen one or several polypeptidic components (*i.e.,* polypeptides of the invention or molecules comprising a polypeptidic moiety including a polypeptide of the invention, such as lipopolypeptides, conjugates consisting of polypeptides bound to a support, etc.). or one or several recombinant cloning and/or expression vector(s) of the invention, and optionally, one or several pharmaceutically acceptable carrier(s), vehicle(s), diluent(s), excipient(s) or adjuvant(s) or any combination thereof.

The compositions according to the invention can be solutions, caplets, *etc.*

The compositions of the invention are advantageously immunogenic compositions, capable of eliciting or improving the production of antibodies in a host, especially in a human host.

The present invention also relates to the use of a polypeptide, a polynucleotide, a recombinant vector or a composition of the invention in a number of applications in the research, diagnostic and vaccinations fields.

In particular, the present invention pertains to a polypeptide, a polynucleotide, an expression vector or a composition of the invention, for use for preventing and/or treating malaria and especially for eliciting or improving an immunological response against *Plasmodium* strains, in a human host, in particular for eliciting or improving a protective response against *Plasmodium* strains, especially against *P. falciparum.*

The invention also relates to the use of a polypeptide, a polynucleotide or a recombinant vector of the invention, for the preparation of a vaccine against malaria.

The present invention also relates to a vaccine against malaria comprising or consisting of as an immunogen one or several polypeptide(s) of the invention or polypeptidic components as defined above, in association with a suitable pharmaceutical vehicle.

The invention also pertains to a nucleic acid vaccine, comprising a polynucleotide of the invention.

A composition and a vaccine according to the invention can further comprise at least one antigen selected amongst LSA-1 (Guerin-Marchand, Druilhe et al. 1987), LSA-3 (Daubersies, Thomas et al. 2000), LSA-5, SALSA (Bottius, BenMohamed et al. 1996), STARP (Fidock, Bottius et al. 1994), TRAP (Robson, Hall et al. 1988), PfEXP1 (Simmons et al. 1987), CS (Dame et al. 1984), MSP1 (Miller et al. 1993), MSP2 (Thomas et al. 1990), MSP4 (Marshall et al. 1998), MSP5 (Marshall et al. 1998), AMA-1 (Peterson et al. 1989; Escalante et al. 2001), SERP (Knapp et al. 1989) and GLURP (supra). GLURP, and/or LSA-3 and/or SERP proteins or their combinations or immunogenic functional fragments thereof are especially of interest, for use in an immunogenic composition or a vaccine of the invention. According to a particular embodiment, a composition or a vaccine of the invention further comprises an antigenic polypeptidic molecule comprising at least 10 consecutive amino acid residues from the RO region of GLURP, which corresponds to the N-terminal non-repeat region (amino acids 27-500) of the Glutamate-rich Protein (Carvalho *et al.,* 2004).

According to a particular embodiment, the composition or the vaccine of the invention is formulated for intradermal or intramuscular injection. In that case, the immunogenic composition or the vaccine of the invention preferably comprises between 1 and 100 µg of immunogen per injection dose, more preferably between 2 and 50 µg. Alternatively, the immunogenic composition or the vaccine can be formulated for oral administration, as described by BenMohamed et al., 2002.

The composition or vaccine of the invention can also further comprise SBAS2 and/or Alum and/or Montanide as an adjuvant.

Other aspects of the present invention relate to antibodies, especially purified antibodies, and/or fragments thereof, directed against one or several polypeptide of the invention.

According to a particular embodiment, antibodies directed against polypeptide(s) of the invention are able to mediate *P. falciparum* growth inhibition in mice infected by *P. Falciparum.*

A synthetic or recombinant antibody or fragment of antibody (antibodies) which cross-reacts with one or several polypeptide(s) of the invention, and which mediates *Plasmodium* (in particular *P. Falciparum)* growth inhibition or blood stage killing, in the monocyte-dependent, antibody-mediated ADCI mechanism, under *in vitro* conditions, is a particularly interesting antibody according to the invention.

A pool of antibodies and/or fragments of antibodies directed against several polypeptides of the invention is also part of the invention.

Preferred antibodies (or fragments) according to the invention are human or humanized antibodies. These antibodies or fragments of antibodies can be produced for example in *Lemna,* as well as in maize, tobacco, CHO cells, and the like. When produced in CHO cells, they can be obtained for example by using the method described in WO 03/016354.

The present invention also pertains to the use of a composition comprising an antibody or a pool of antibodies or fragments thereof as described herein, for the preparation of a medicament against malaria. Of course, a composition or a medicament for passive immunotherapy of malaria, comprising such an antibody or a pool of antibodies or fragments thereof, is also considered as part of the invention. Such composition or medicament can further comprise antibodies directed against at least one antigen selected amongst LSA-1, LSA-3, LSA-5, SALSA, STARP, TRAP, PfEXP1, CS, MSP1, MSP2, MSP4, MSP5, AMA-1, SERP and GLURP.

Methods for the prophylaxis, the attenuation or the treatment of malaria, by administering to a patient in need thereof, a composition, a vaccine, or a medicament comprising antibodies, as described herein, are also enclosed in the invention.

The invention also concerns a method for the *in vitro* diagnosis of malaria in an individual likely to be infected by *P. falciparum,* which comprises (i) the bringing of a biological sample from said individual into contact with a polypeptide of the invention, under conditions enabling the formation of antigen/antibody complexes between said polypeptide and the antibodies possibly present in the biological sample, and (ii) the *in vitro* detection of the antigen/antibody complexes possibly formed.

It is also possible to bring the biological sample into contact with one or several antigenic peptides originating from other antigens selected amongst LSA-1, LSA-3, LSA-5, SALSA, STARP, TRAP, PfEXP1, CS, MSP-3-1, MSP-3-2, MSP-3-5, MSP-3-6, MSP1, MSP2, MSP4, MSP5, AMA-1, SERP and GLURP, in particular from LSA-3, SERP and GLURP as an additional step of the method.

An alternative method for the *in vitro* diagnosis of malaria in an individual likely to be infected by *P. falciparum* comprises (i) the bringing of a biological sample from said individual into contact with antibodies according to the invention, under conditions enabling the formation of antigen/antibody complexes between said antibodies and the antigens specific for P. *falciparum* possibly present in the biological sample, and (ii) the *in vitro* detection of the antigen/antibody complexes possibly formed.

In these methods, the *in vitro* diagnosis can be performed by an ELISA assay.

Kits for the *in vitro* diagnosis of malaria, comprising at least one polypeptide of the invention, possibly bound to a support, and/or antibodies of the invention are also contemplated. Such a kit can further comprise reagents for enabling the formation of antigen/antibody complexes between said polypeptide and the antibodies possibly present in a biological sample or between said antibodies and the antigens specific for *Plasmodium falciparum* possibly present in the biological sample, and/or reagents enabling the *in vitro* detection of the antigen/antibody complexes possibly formed.

Several aspects and advantages of the present invention are illustrated in the following figures and experimental data.

### BRIEF DESCRIPTION OF THE FIGURES

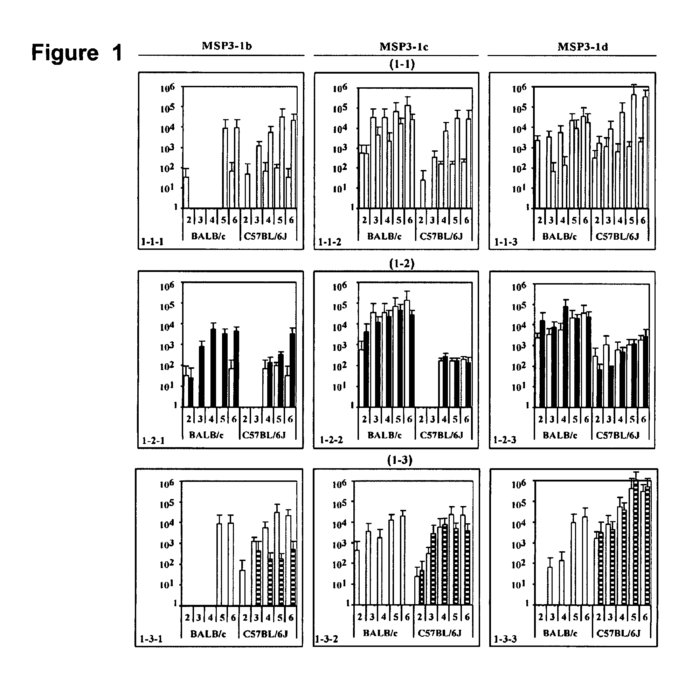

**Figure 1****: Antibody responses in BALB/c or C57BL/6J mice immunized with each of the four constructs of MSP3-1.** Sera of mice immunized with MSP3-1 (a-f) (white bars), MSP3-1 (b-f) (black bars), MSP3-1 LSP(a-d) (gray bars) and MSP3-1 LSP(b-d) (hatched bars) were tested by ELISA on the MSP3-1b (left column), MSP3-1 c (center column) and MSP3-1 d (right column) peptides. (1-1): Comparison of IgG responses elicited in MSP3-1(a-f) and MSP3-1 LSP(a-d) immunized mice to peptides MSP3-1 b (1-1-1), MSP3-1c (1-1-2) and MSP3-1d (1-1-3). (1-2): Comparison of IgG responses elicited in MSP3-1 (a-f) and MS3-1(b-f) immunized mice to peptides MSP3-1 b (1-2-1), MSP3-1c (1-2-2) and MSP3-1d (1-2-3). (1-3): Comparison of IgG responses elicited in MSP3-1 LSP(a-d) and MSP3-1 LSP(b-d) immunized mice to peptides MSP3-1 b (1-3-1), MSP3-1c (1-3-2) and MSP3-1d (1-3-3). Results are expressed as mean titers in groups of five mice after 2 to 6 injections of the immunogens administered at regular intervals of two weeks. ELISA titers lower than 50 are considered as negative.

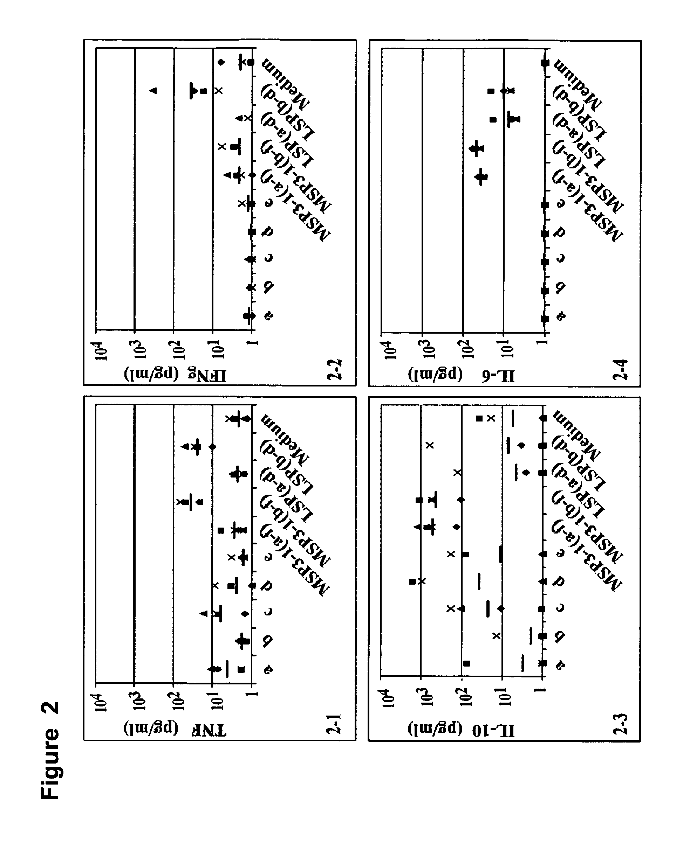

**Figure 2****: Cytokine levels in 48h culture supernatants of splenocytes from MSP3-1(b-f) immunized C57BL/6J, *in vitro* stimulated with MSP3-1 peptides or long constructs.** (2-1) TNF levels; (2-2) IFNγ levels; (2-3) IL-10 levels; (2-4) IL-6 levels. The black horizontal bars represent the geometric mean of five different mice tested in duplicate in samples taken after the third immunization.

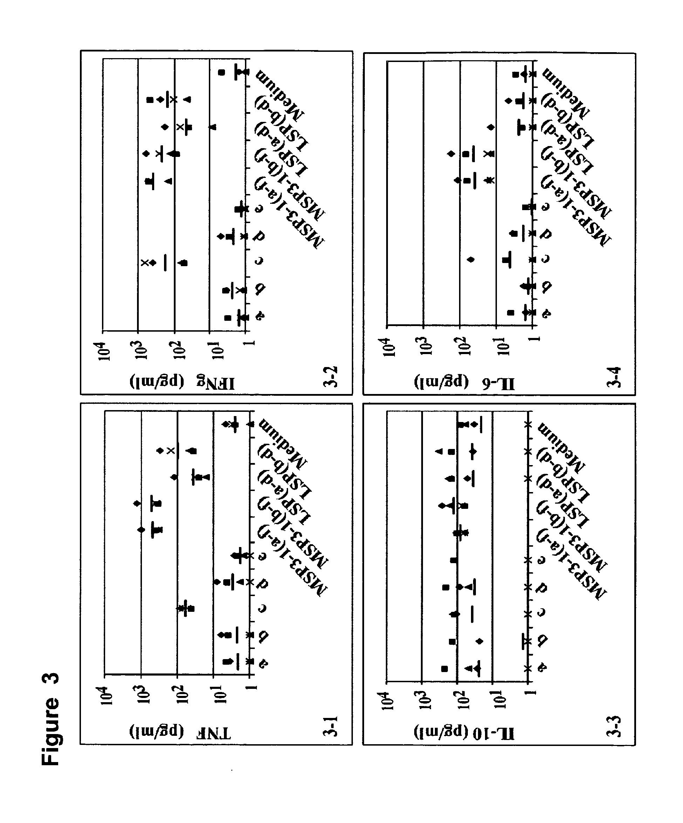

**Figure 3****: Cytokine levels in 48h culture supernatants of splenocytes from MSP3-1 LSP(b-d) immunized C57BL/6J, *in vitro* stimulated with MSP3-1 peptides or long constructs.** (3-1) TNF levels; (3-2) IFNγ levels; (3-3) IL-10 levels; (3-4) IL-6 levels. The black horizontal bars represent the geometric mean of five different mice tested in duplicate in samples taken after the third immunization.

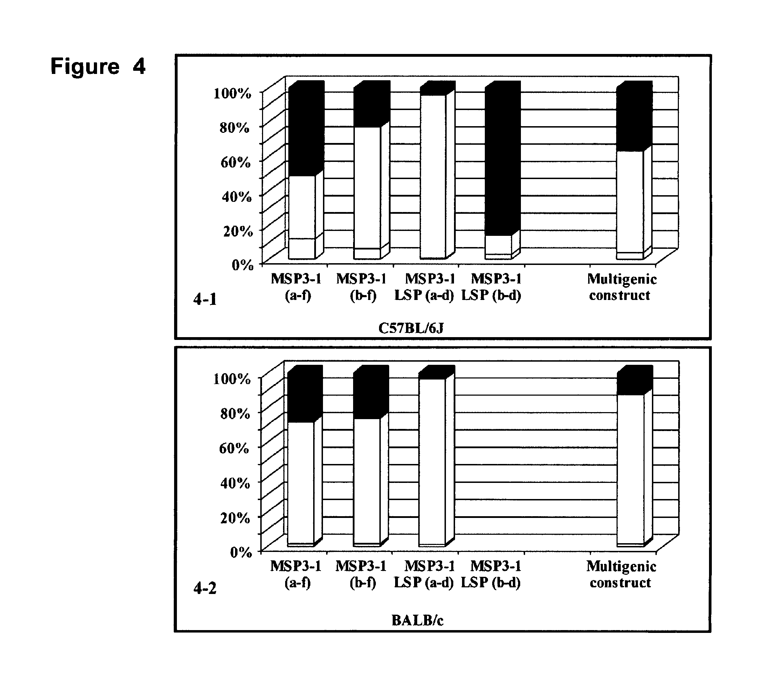

**Figure 4****: I*s*otypic distribution of the IgG responses against each of the four MSP3-1 immunizing antigens and the multigenic construct.** Specific IgG2a (black), IgG2b (dark gray), IgG1 (light gray), and IgG3 (white) against the immunogen itself were determined by ELISA. Geometric means were calculated for each of the four isotypes in each group of five mice, and are represented as percentages of the total IgG responses in C57BL/6J (4-1) and BALB/c (4-2). Geometric means (95% confidence interval) of the total IgG responses in C57BL/6J mice immunized with MSP3-1 (a-f), MSP3-1(b-f), MSP3-1 LSP (a-d), MSP3-1 LSP(b-d) and the multigenic construct were: 42.1 µg/ml (41.1-42.9); 262.0 µg/ml (258.5-265.5); 743.7 µg/ml (712.1-776.7), 179.4 µg/ml (172.3-186.8) and 30.2µg/ml (29.2-32.1), respectively. Geometric means (95% confidence interval) of the total IgG responses in BALB/c mice immunized with MSP3-1 (a-f), MSP3-1(b-f), MSP3-1 LSP (a-d), and the multigenic construct were : 556.4µg/ml (536.1-577.5); 313.4µg/ml (306.3-320.6), 277.7µg/ml (268.1-287.6) and 652.6µg/ml (635.1-670.6), respectively. No antibody responses were detectable in BALB/c mice which were immunized using the MSP3-1 LSP(b-d) construct. Results for the multigenic antigen described in the second part of the article are represented on the same figure for easier comparison.

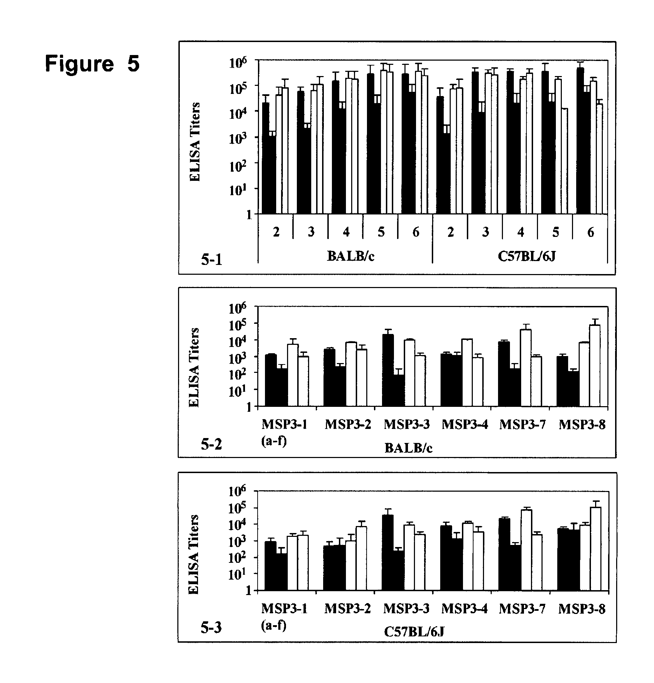

**Figure 5****: IgG responses in BALB/c and C57BL/6J mice immunized with the C-terminal recombinant proteins MSP3-3, MSP3-4, MSP3-7 and MSP3-8.** (5-1) ELISA titers using the immunogen as coating antigen in mice immunized 2 to 6 times with MSP3-3 (dark gray bars), MSP3-4 (black bars), MSP3-7 (white bars), or MSP3-8 (light gray bars). (5-2) Cross reactivity on the six members of the MSP3 protein family of sera from BALB/c mice, after 2 injections of MSP3-3 (dark gray bars), MSP3-4 (black bars), MSP3-7 (white bars), or MSP3-8 (light gray bars). (5-3) Cross reactivity on the six members of the MSP3 protein family of sera from C57BL/6J mice, after 2 injections of MSP3-3 (dark gray bars), MSP3-4 (black bars), MSP3-7 (white bars), or MSP3-8 (light gray bars).

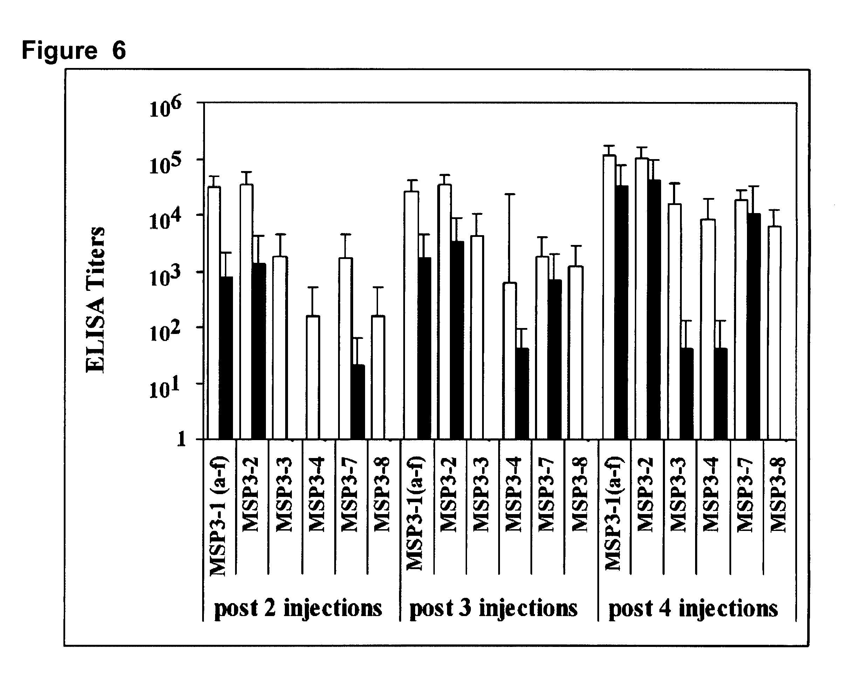

**Figure 6****: IgG responses against the C-terminal recombinant proteins of the MSP3 family in mice immunized with the new multigenic constructs.** Groups of five BALB/c (white bars) and C57BL/6J (black bars) mice were immunized with the multigenic construct and IgG responses were tested by ELISA after 2, 3 and 4 injections against MSP3-1 (a-f); MSP3-2; MSP3-3; MSP3-4; MSP3-7 and MSP3-8. Results are expressed as mean titers.

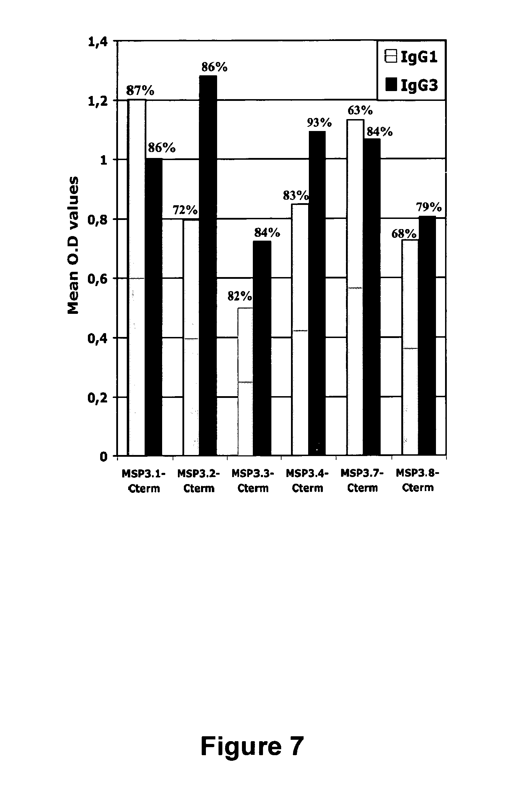

**Figure 7****: Cytophilic IgG subclasses responses upon the C-terminal part of the six MSP3 antigens in serum samples of individuals of all age groups (n=76, 0.9 -80.4 years, mean age =14.4 years) from Dielmo, Senegal.** To calculate the prevalence values, responses were considered positive when the net OD value of each serum sample, obtained by subtracting the mean OD+ 3SD of control serum samples, was > 0. The mean OD represents the mean responses of the positive serum samples.

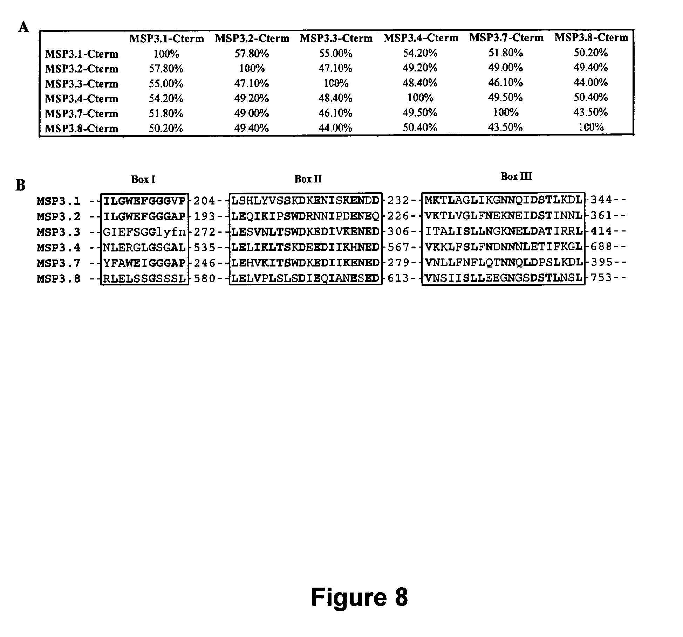

**Figure 8**: Panel A, Matrix of overall percent homology (identity and similarity) of the C-terminal sequences of the six MSP3 proteins obtained with EMBOSS pairwise alignments; Panel B, Truncated multiple sequence alignment of the MSP3 proteins family showing the three regions with common linear motifs present in the C-terminal part. Conserved residues are highlighted in bold.

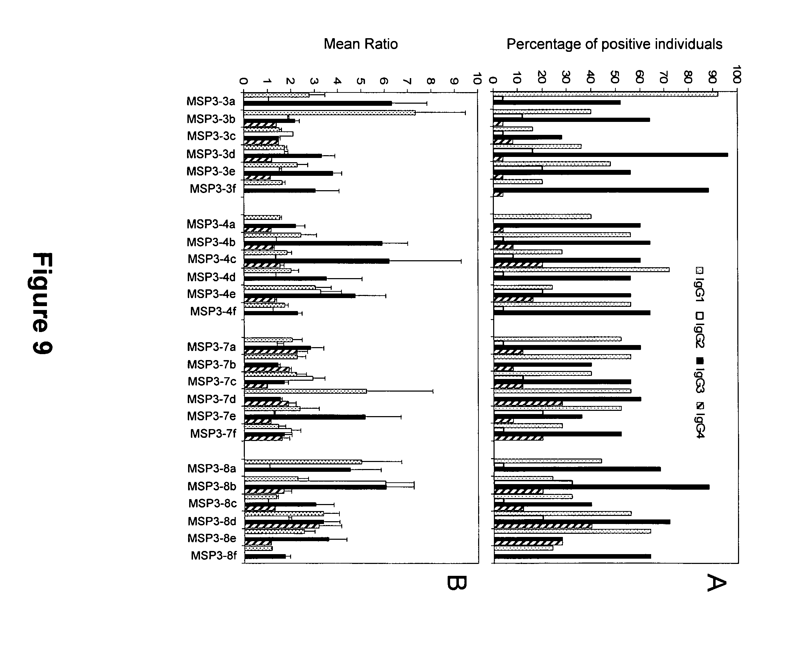

**Figure 9****: Prevalences (A) and Titers (B) of IgG subclasses distribution in 24 peptides derived from MSP3.3, MSP3.4, MSP3.7 and MSP3.8 in serum samples of malaria exposed individuals (n=25; age range 2-54 years, mean age 16.86) from Dielmo village.** Bars are standard errors of the mean.

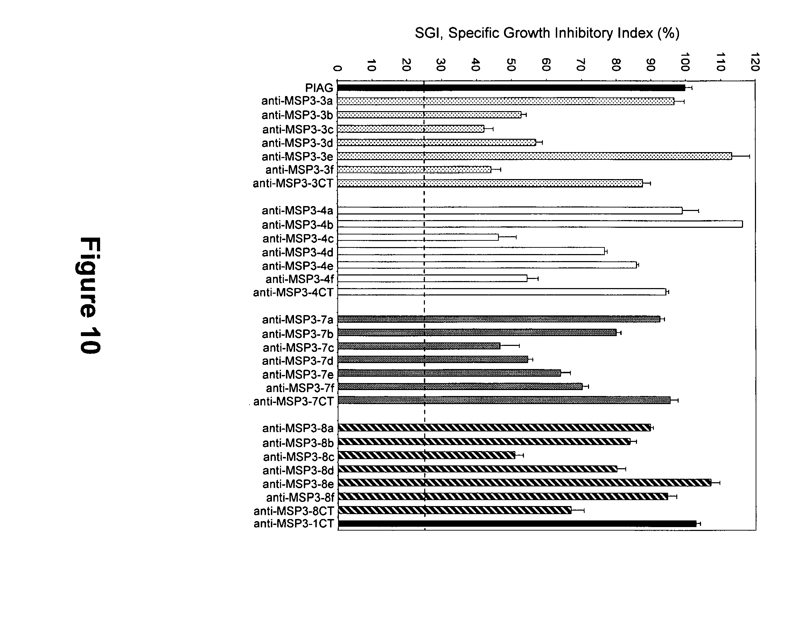

**Figure 10****: Antibody-dependent cellular inhibitory effect of human antibodies affinity purified upon the 24 peptides and the 4 recombinant antigens covering the C-terminal regions of MSP3 proteins.** In black positive controls, gray for anti-MSP3.3 peptides, White for anti-MSP3.4 peptides, Hatched for anti-MSP3.7 peptides and dotted for anti-MSP3.8 peptides. PIAG, Pool of African Immune Globulins. Each of the affinity-purified antibodies was tested in duplicate at a concentration corresponding to a 1/20 final ImmunoFluorescence Assay (IFA) titer. The PIAG and antibodies affinity purified upon recombinant MSP3.1-Cterm were used as positive controls in each experiment. The SGI values are expressed as a % of the effect of PIAG (100%); values > 30% are consider significant.

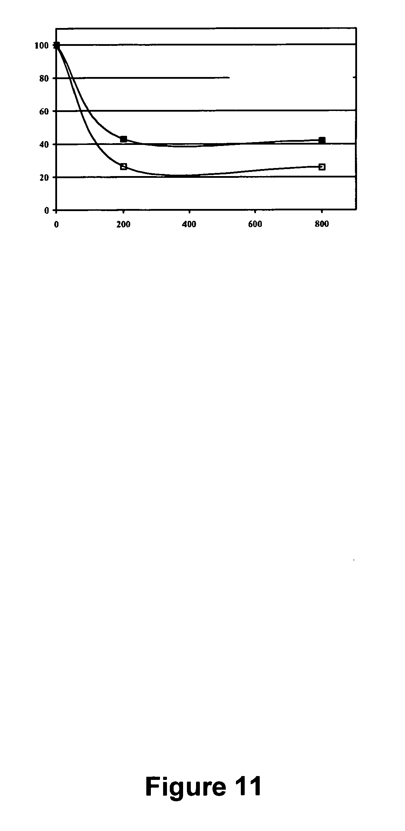

**Figure 11****: Antibodies cross-reactivity between peptides "b" of the MSP3 proteins family.** Open squares: binding of anti-MSP3.1 b specific antibodies upon the recombinant antigen MSP3.1-Cterm in the presence of of increasing concentrations of the MSP3.2b peptide. Plain squares: binding of anti-MSP3.7b specific antibodies upon the recombinant antigen MSP3.7-Cterm in the presence of increasing concentrations of the MSP3.1 b peptide. Affinity purified antibodies were preincubated with different amounts of peptides before being tested in ELISA.

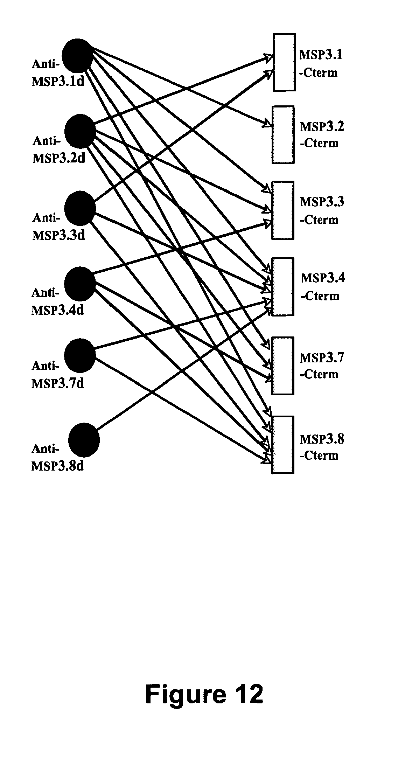

**Figure 12****: Pattern of cross-reactivity of antibodies to peptides "d" from the MSP3 proteins family.** Binding patterns of antibodies specific to the peptides "d" of the six MSP3 proteins were deduced from their ELISA reactivity against the different recombinant antigens. Note that the reactivity to the homologous protein (e.g. Anti-MSP3-8d peptide with MSP3-8 C-term protein) was present for each of them, however is not shown for clarity reasons. A significant cross-reactivity corresponds to OD values ≥ 0.2.

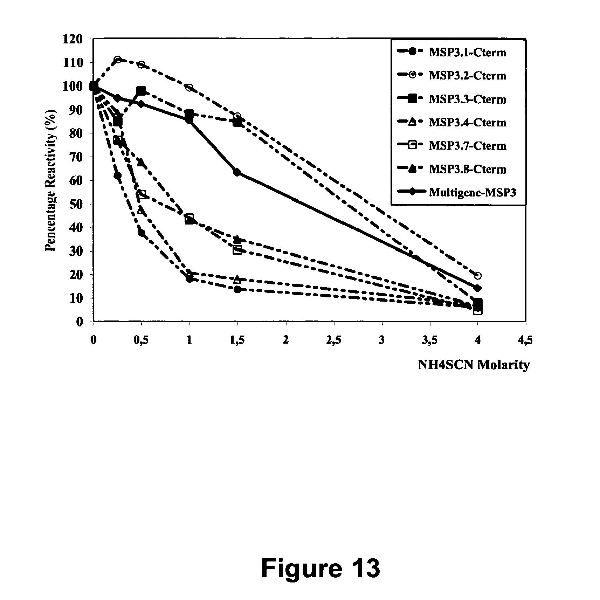

**Figure 13****: Avidity of antibodies induced in mice after immunisation with a MSP3- family multigenic construct.** The curves represent the reactivity on the MSP3 multigenic construct itself and on the different MSP3 antigens in the presence of increasing concentrations of chaotropic ion, ammonium thiocyanate (NH4SCN).

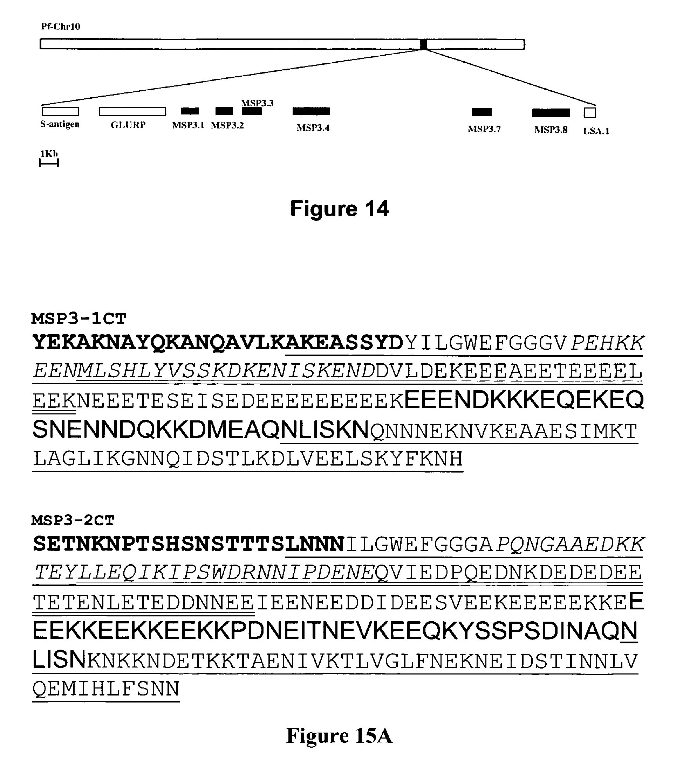

**Figure 14****: Graphical representation of the genomic location of msp3 related Genes.** The six msp3 genes are all in a cluster on *P. falciparum* chromosome 10 and close to the genes of S-antigen, GLURP and LSA-1.

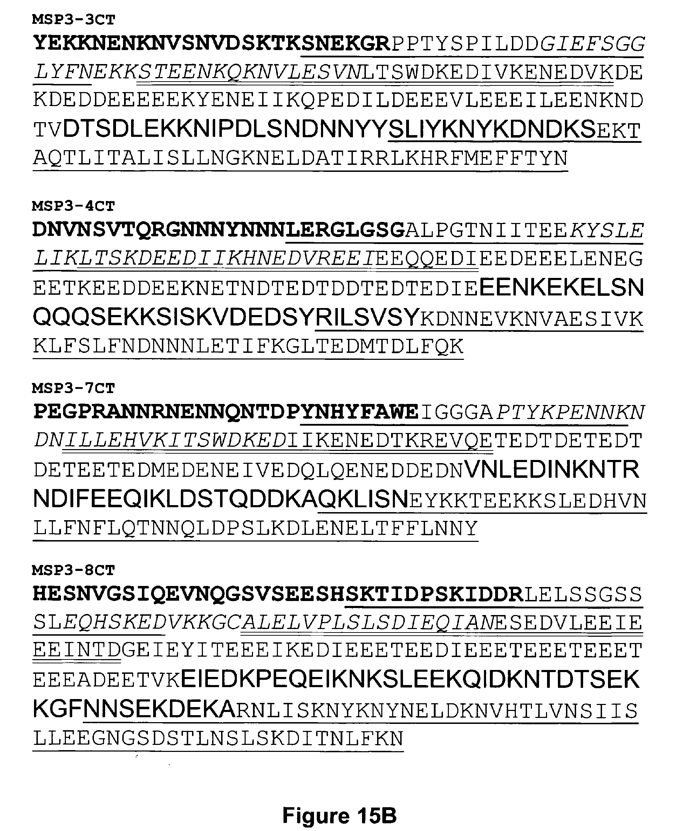

**Figure 15****: Schematic representation of the six overlapping peptides covering the C-terminal parts of six *P. falciparum* MSP3 proteins (MSP3-1, MSP3-2, MSP3-3, MSP3-4, MSP3-7 and MSP3-8).** The peptides "a" are in grey, the peptides "b" are underlined, the peptides "c" are in italics, the peptides "d" are double underlined, the peptides "e" are in larger letters and the peptides "f" (at the C-terminal end) are underlined.

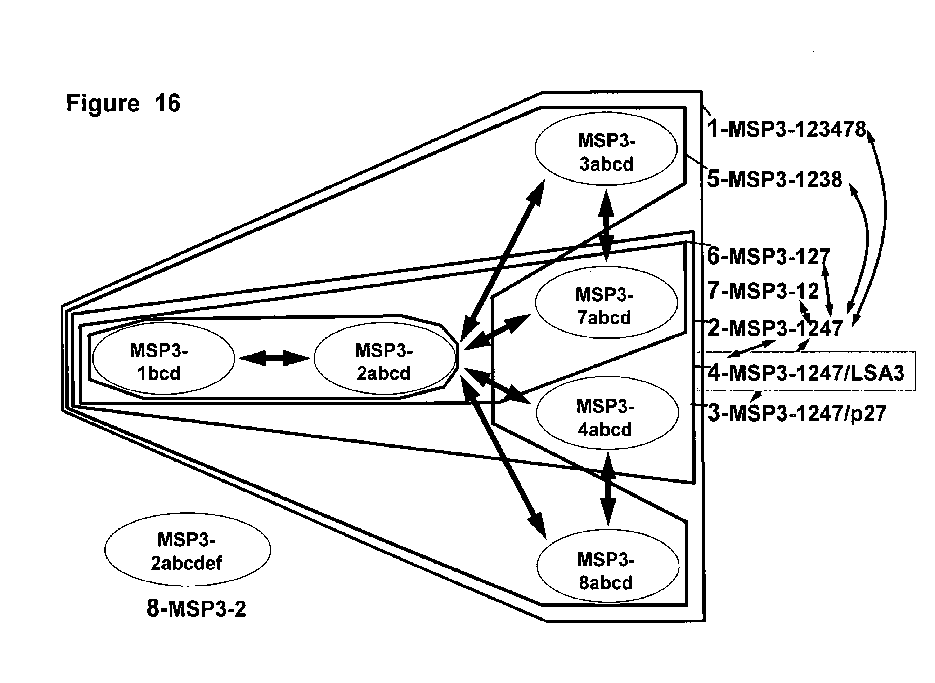

**Figure 16****: Choice of antigenic sequences to include in the polyprotein constructs** (cross reactivity network).

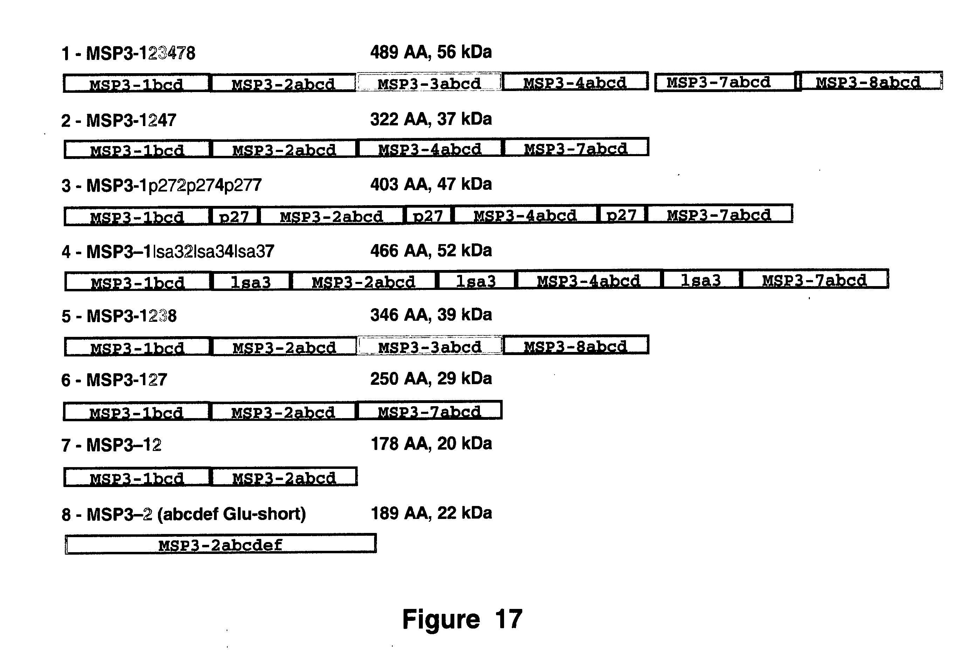

**Figure 17****: Schemes of polyproteins.**

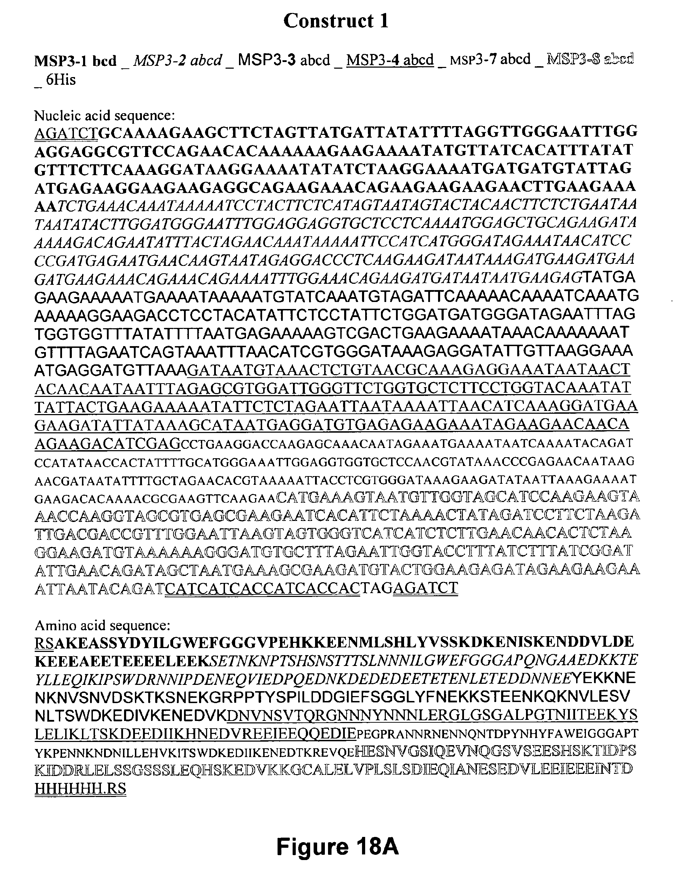

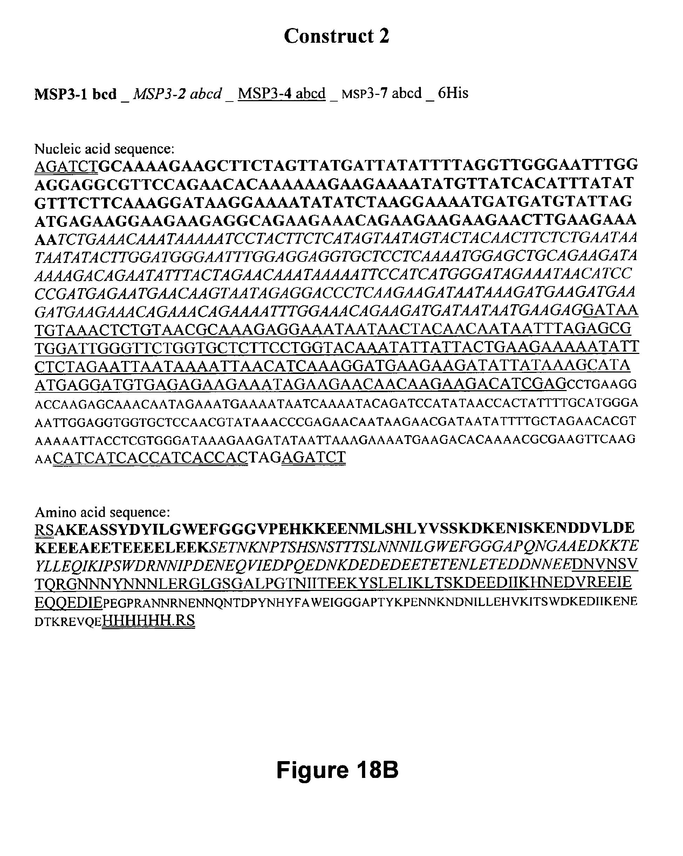

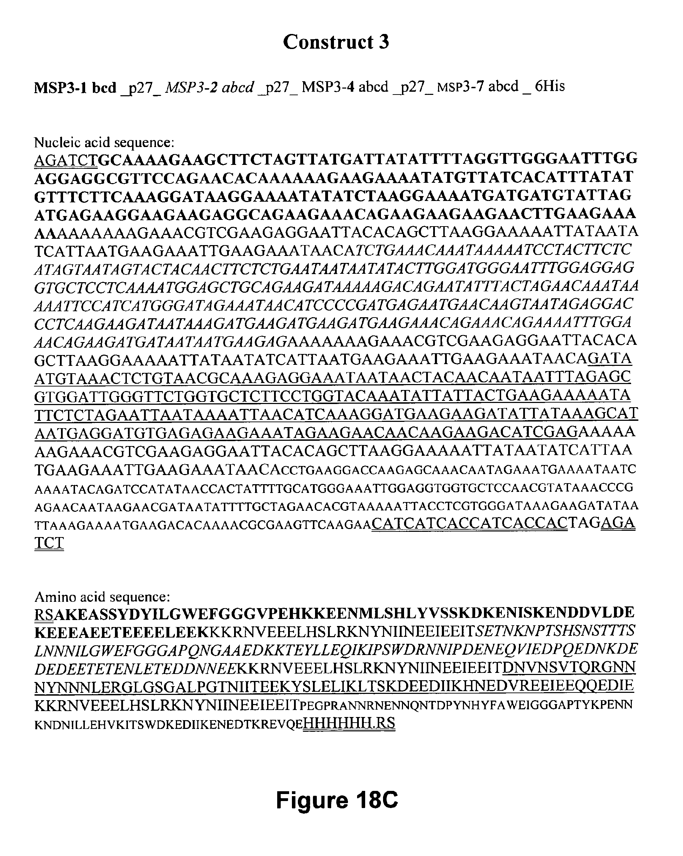

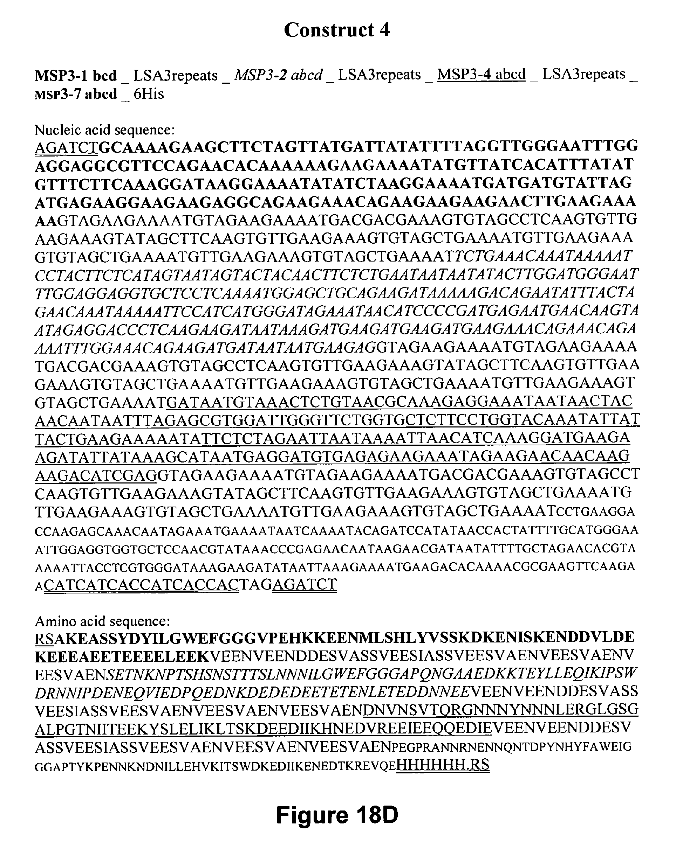

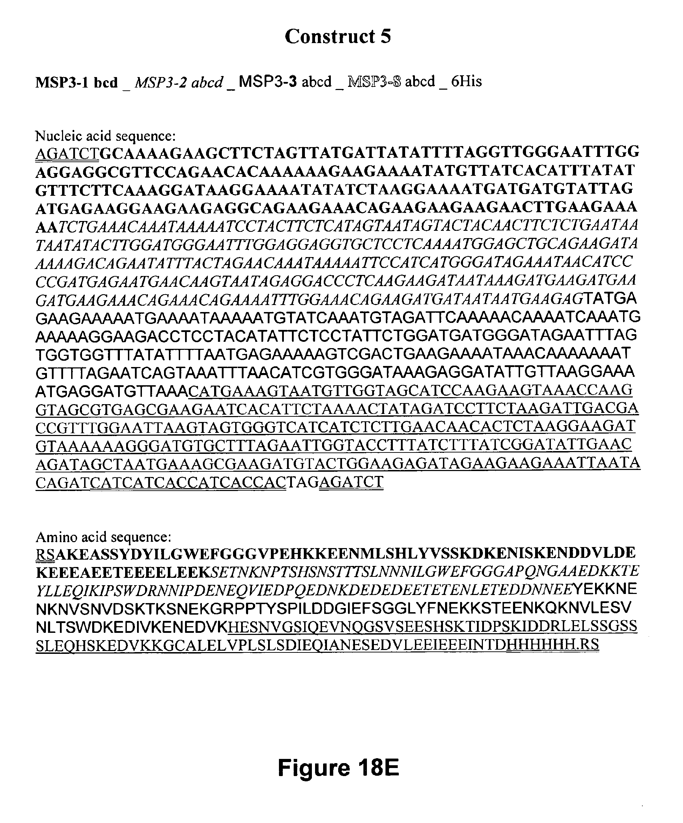

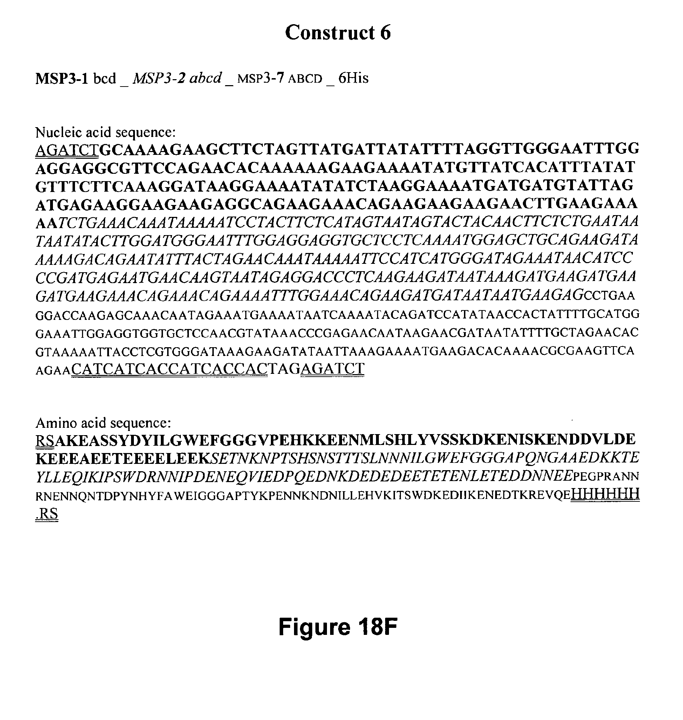

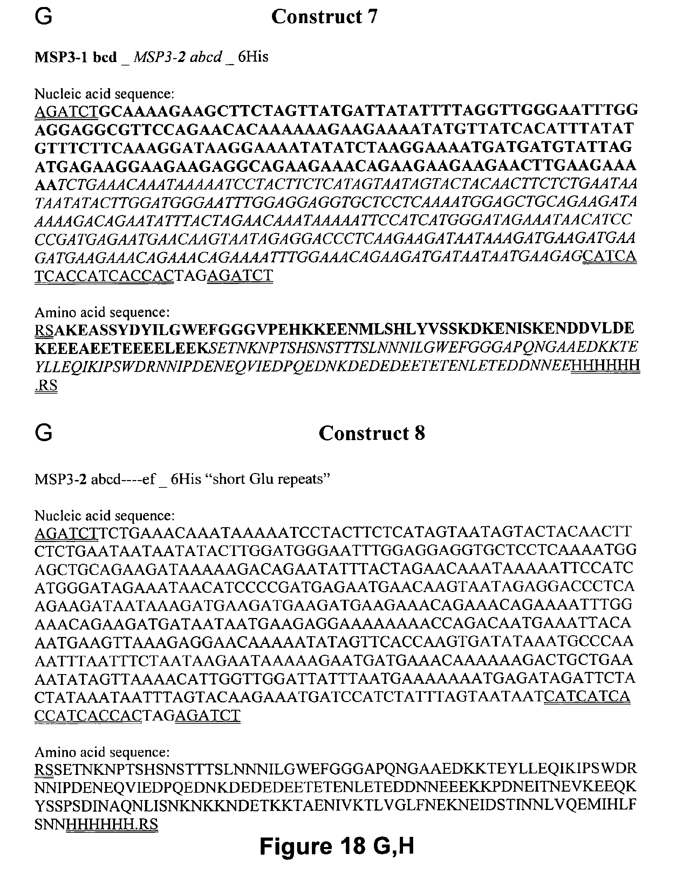

**Figure 18****: Nucleic acid and acid sequences for synthetic multi-proteins composed by motifs derived from *P. falciparum* (strain 3D7) MSP3 family of molecules.** The Bgl-II restriction sites at both ends of the constructs (nucleic acid sequence: AGATCT; corresponding amino acid sequence: RS), as well as the C-terminal tag sequence (nucleic acid sequence: CATCATCACCATCACCAC; corresponding amino acid sequence: HHHHHH), are optional. The construct shown in H corresponds to the C terminal end of *P. Faciparum* (strain 3D7) MSP3-2 in which the amino acid sequence IEENEEDDIDEESVEEKEEEEEKKEEEEKKEEKKE has been deleted between motifs d and e.

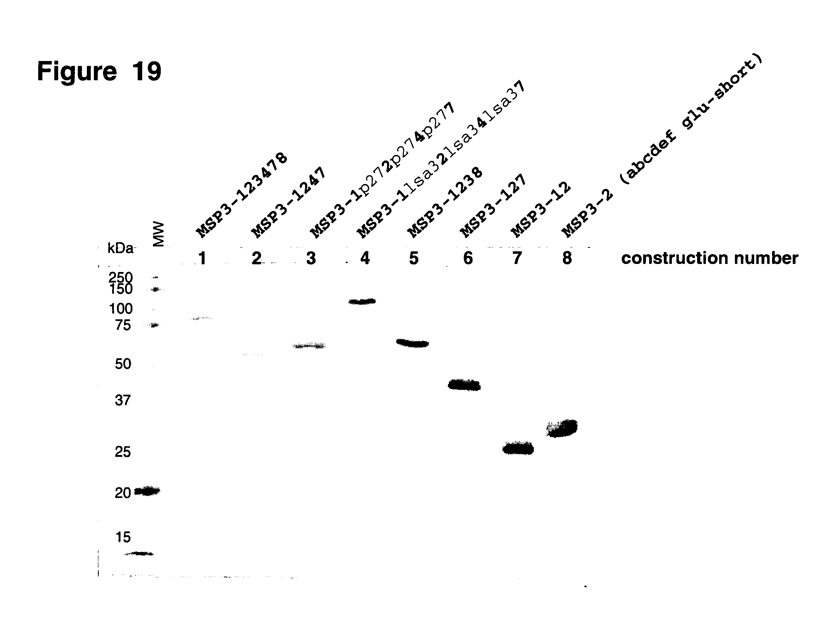

**Figure 19****: Production in the strain BL21 of E. *coli* of MSP3 polyproteins.** 1 µg of each of the produced recombinant proteins was run on SDS-Page 10%.

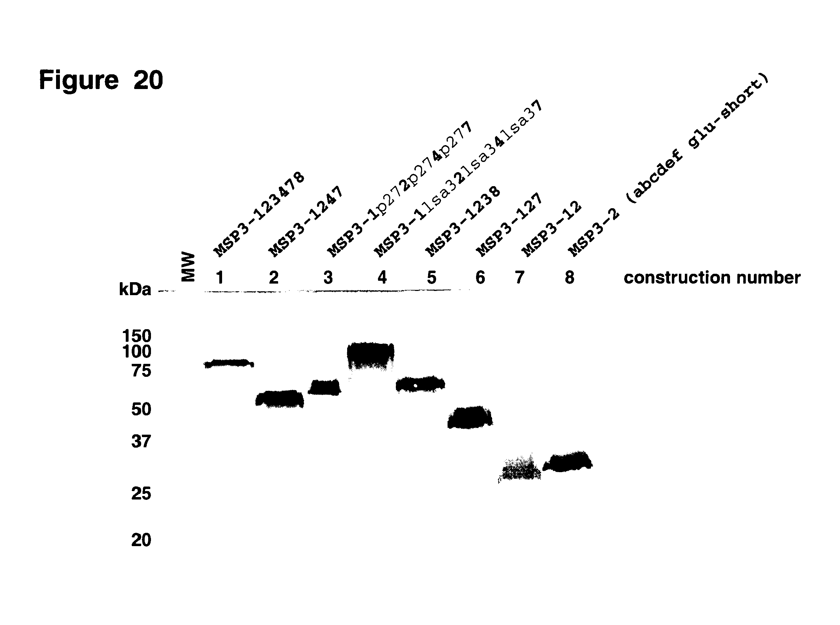

**Figure 20****: Antigenicity of MSP3 polyproteins.** 1 µg of each of the recombinant proteins was run on SDS-Page 10%.

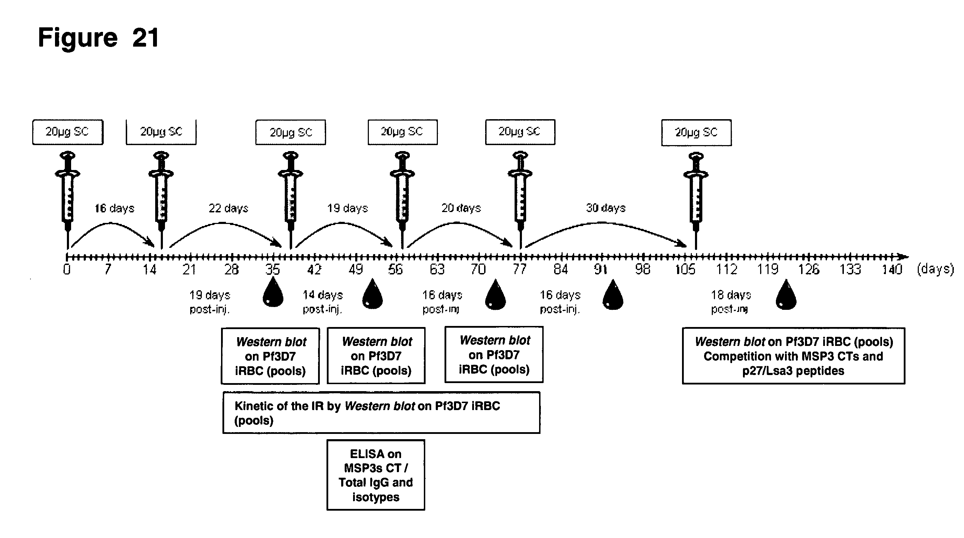

**Figure 21****: Schedule of immunisations and samples harvesting in mice immunised with MSP3 polyproteins.**

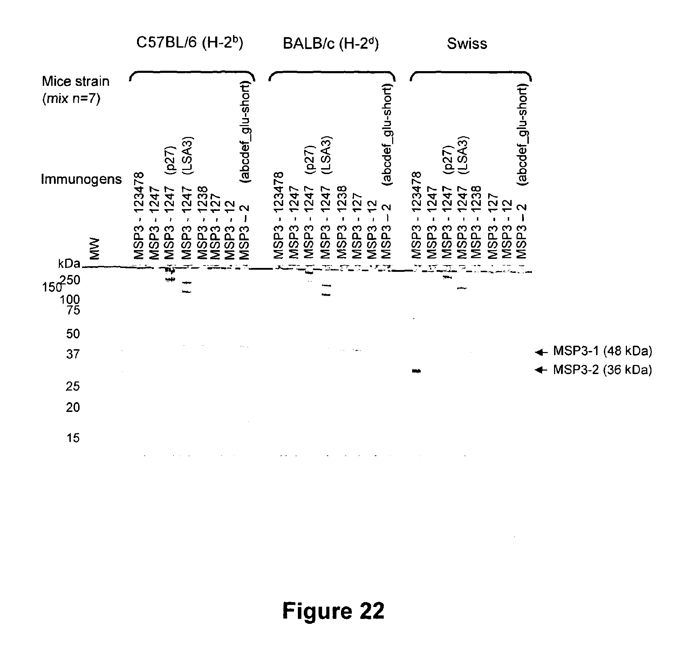

**Figure 22****: *Western blot* on *Plasmodium falciparum* (3D7) - infected red blood cells with pools of immune sera from mice immunised with the MSP3 recombinant polyproteins after the second injection.** All the recombinant polypeptides induced an antibody response in the three strains of mice. Responses against MSP3-1 and MSP3-2 seem to be predominant.

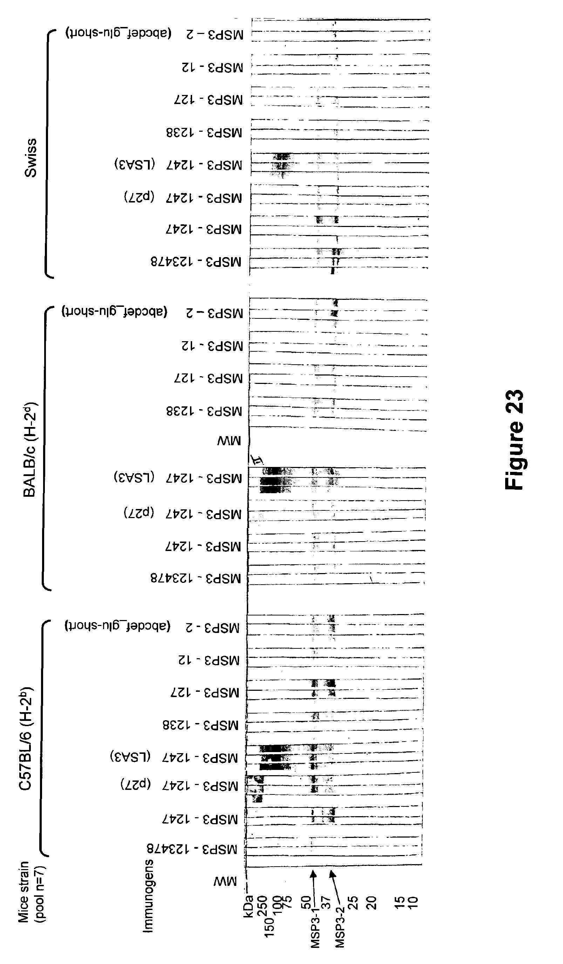

**Figure 23****: Kinetic of the immune response in mice immunised with MSP3 polyproteins by Western *blotting* on *Plasmodium falciparum* (3D7) - infected red blood cells after the second, third and fourth injections.** The antibody response was stable after the second injection.

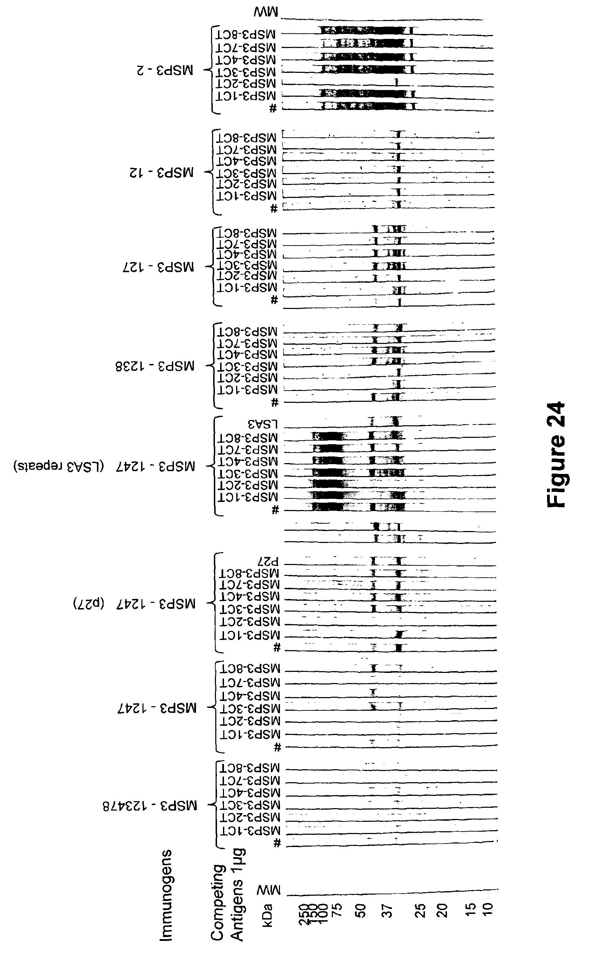

**Figure 24****: *Western blot* on *Plasmodium falciparum* (3D7) - infected red blood cells with pools of immune sera from BALB/c mice after the sixth immunization.** Competition with single antigens. Competition with the C-terminal end of MSP3-2 affects differently the recognition of MSP3-2 in function of the recombinant polypeptides used for immunization, which suggests participation of cross-reacting antibodies.

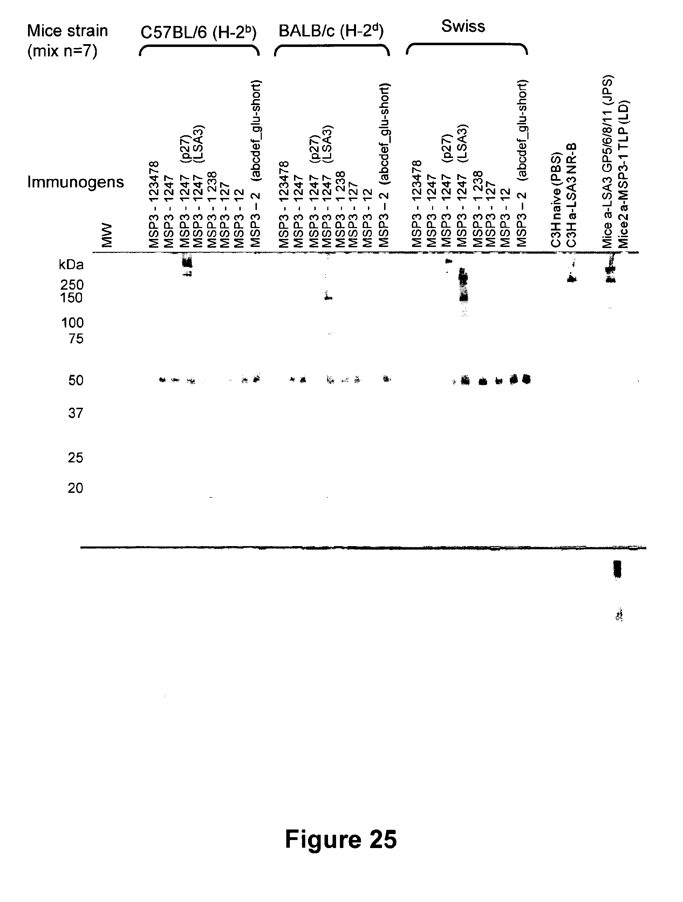

**Figure 25****: *Western blot* of *Plasmodium falciparum* 3D7 infected red blood cells with immune sera from immunised mice post 3rd injection with polyproteins composed of member parts of the Pf MSP3 family of molecules. A:** SDS-PAGE 8% - antigen; 60 x 106 Pf 3D7 infected hu RBC (28-9-07, parasitemia 40%) in 75x1 mm well - Mice immunesera pools (n=7) used at 1:100, a-LSA3 GP5/6/8/11 (JPS) and mice a-MSP3-1 TLP (LD) used at 1:200, 2nd AbAP 1:7500. **B:** SDS-PAGE 8% - antigen; 60 x 106 hu RBC in 75x1 mm well.

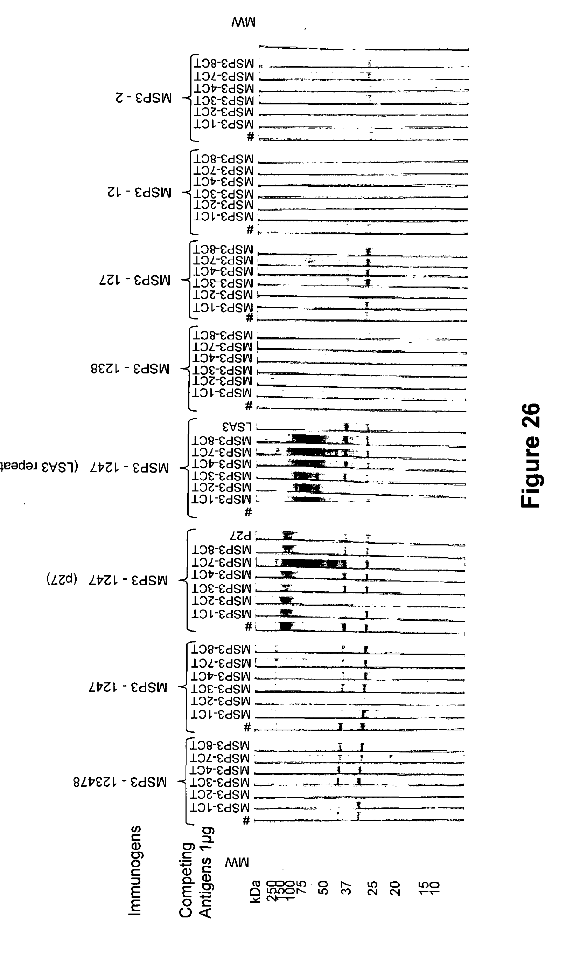

**Figure 26****: *Western blot* on *Plasmodium falciparum* (3D7) - infected red blood cells with pools of immune sera from C57BU6 mice after the 6th immunization.** Competition with single antigens

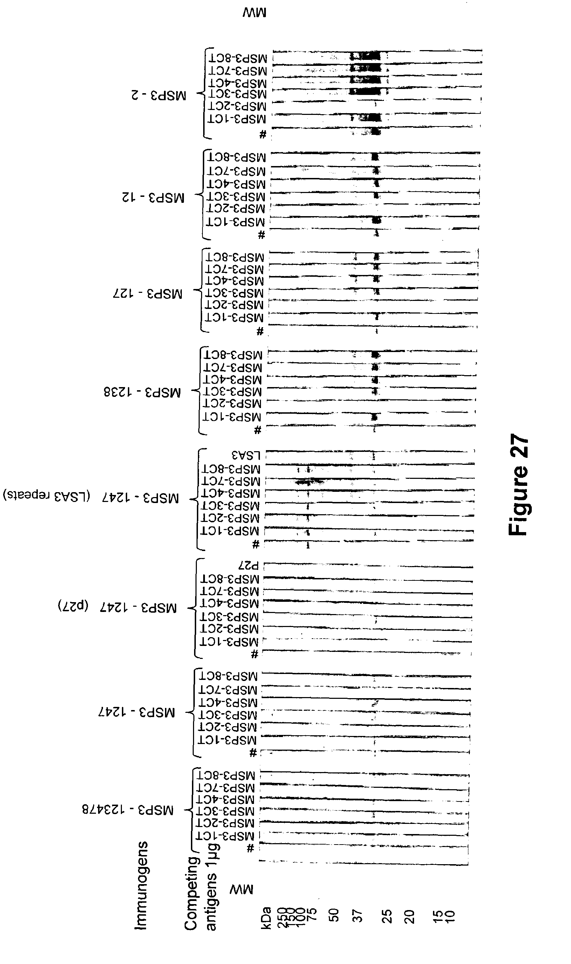

**Figure 27****: *Western blot* on *Plasmodium falciparum* (3D7) - infected red blood cells with pools of immune sera from Swiss mice after the 6th immunization.** Competition with single antigens

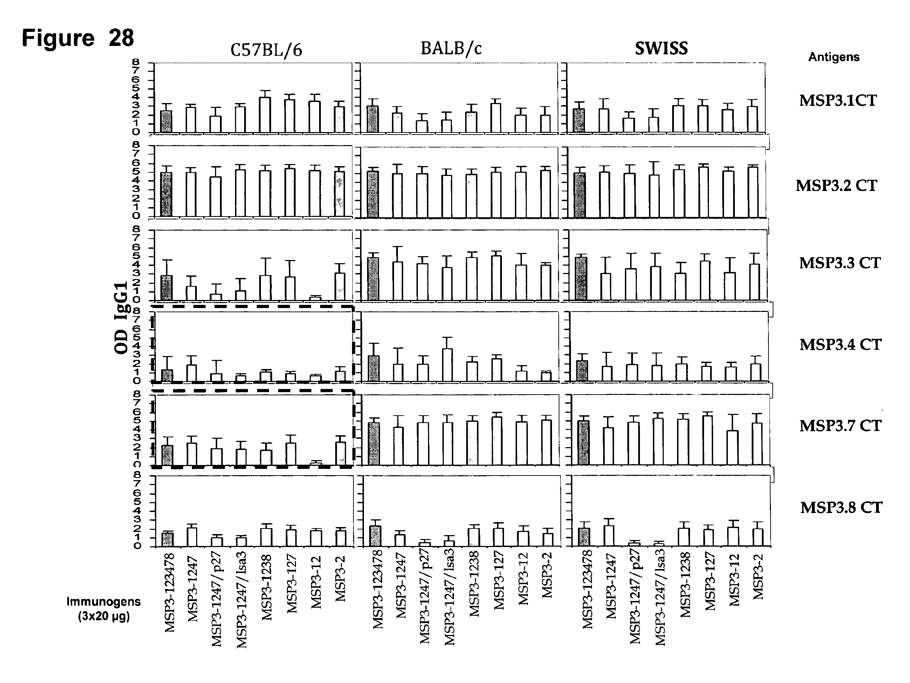

**Figure 28****: Cytophilic IgG1 subclass response directed against the C-terminal part of six MSP3 proteins in the 3 strains of mice (C57BU6, Balbc and SWISS) 19 days after the third immunization with MSP3 polyproteins.** There is general good immunogenicity induced against the C-terminal part of the 6 MSP3 family members in the 3 strains of mice.

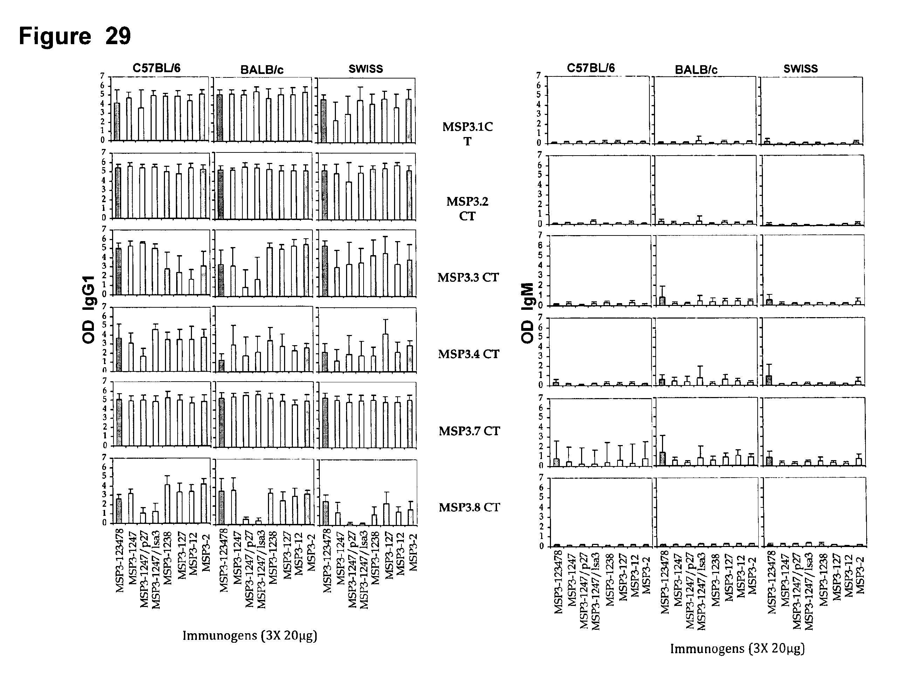

**Figure 29****: Cytophilic IgG1 and IgM subclasses responses directed against the C-terminal part of six MSP3 proteins in the 3 strains of mice (C57BU6, Balbc and SWISS) after the third immunization with MSP3 polyproteins.**

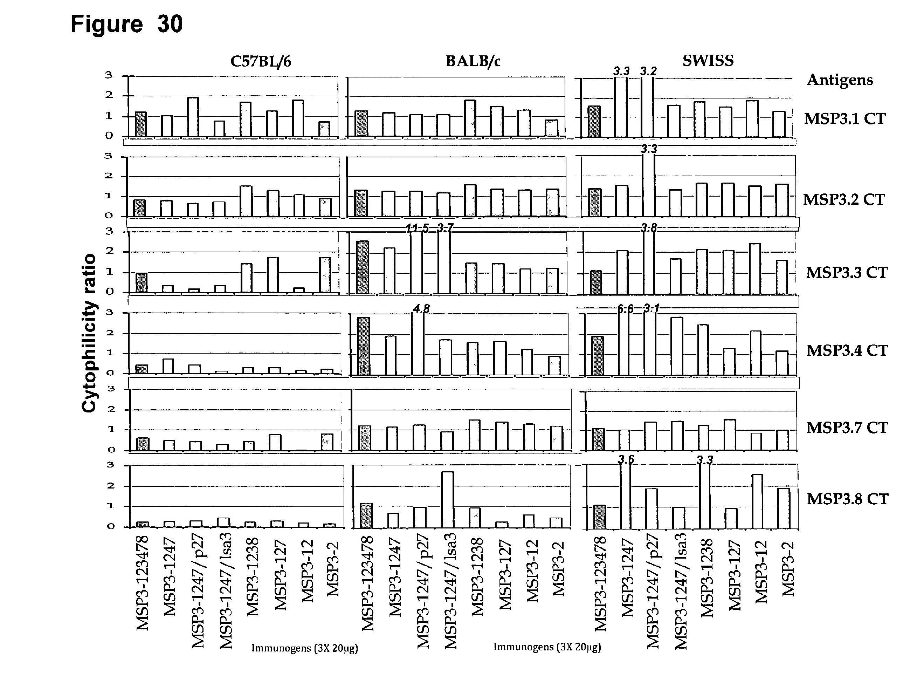

**Figure 30****: Cytophilicity ratio of three strains of mice (C57BU6, Balbc and SWISS) against the C-terminal part of six MSP3 proteins after the third immunization with different multigenic constructs.**

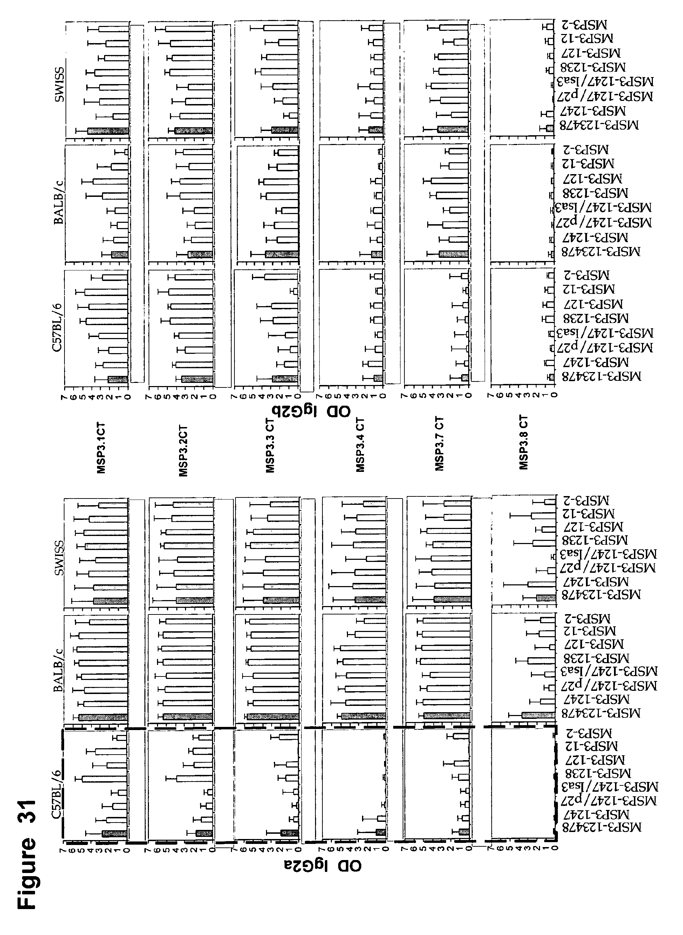

**Figure 31****: Cytophilic IgG2a and IgG2b subclasses responses directed against the C-terminal part of six MSP3 antigens in the 3 strains of mice (C57BU6, Balbc and SWISS) after the third immunization with MSP3 polyprotein.**

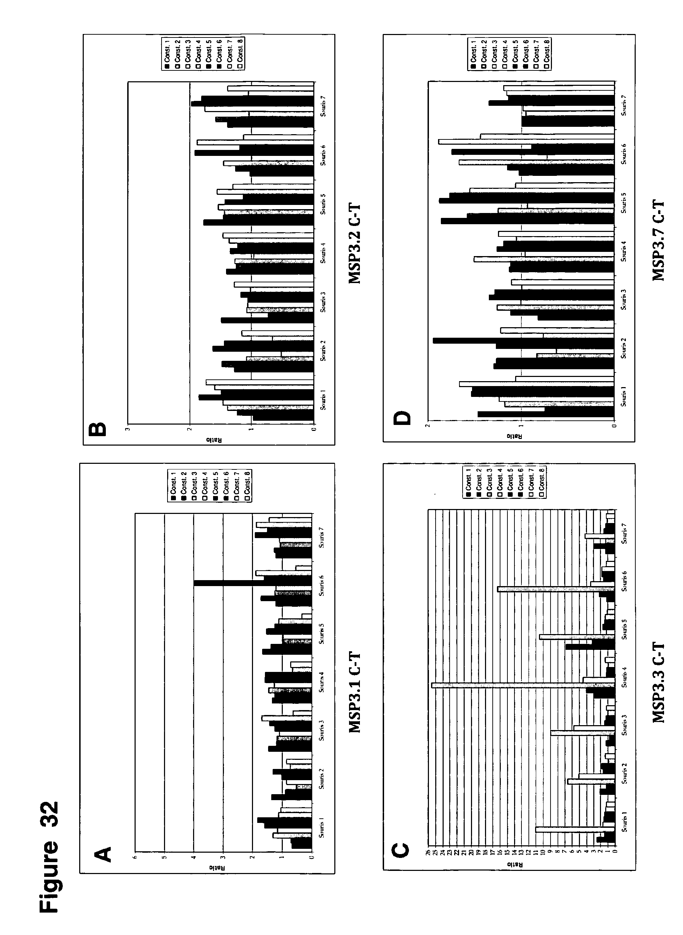

**Figure 32****: Homogeneity of the response (cytophilicity ratio) between each mouse (Balbc) after the third immunization and against four MSP3 C-termini antigens.** Cytophilicity ratio of Balbc mice against the C-terminal end of MSP3-1 (A), MSP3-2 (B), MSP3-3 (C) and MSP3-7 (D) after the third immunization with different multigenic constructs.

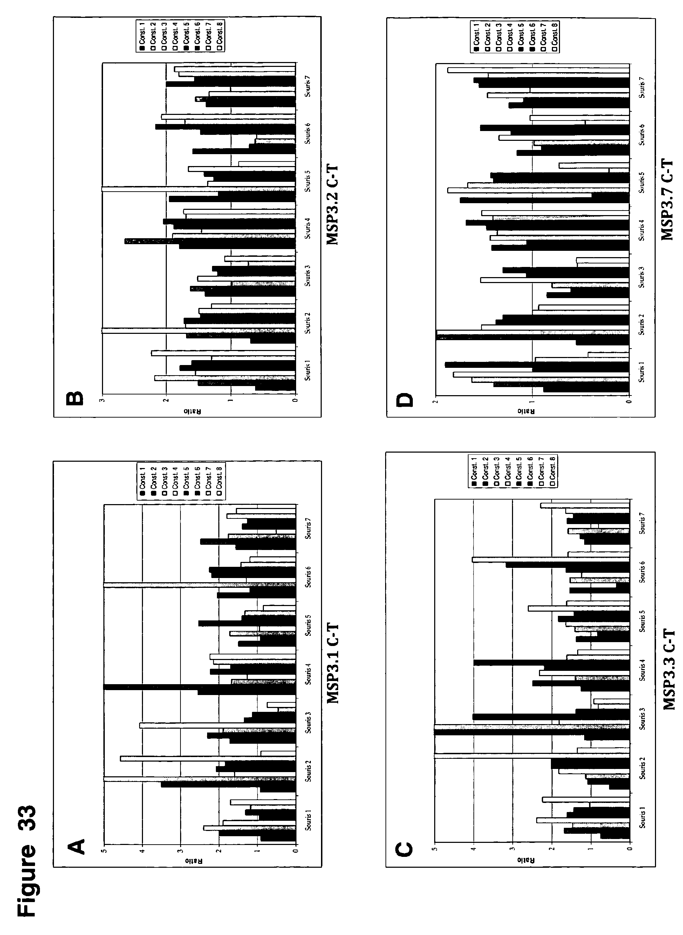

**Figure 33****: Homogeneity of the response (cytophilicity ratio) between each mouse (SWISS) after the third immunization and against four MSP3 C-termini antigens.** Cytophilicity ratio of Swiss mice against the C-terminal end of MSP3-1 (A), MSP3-2 (B), MSP3-3 (C) and MSP3-7 (D) after the third immunization with different multigenic constructs.

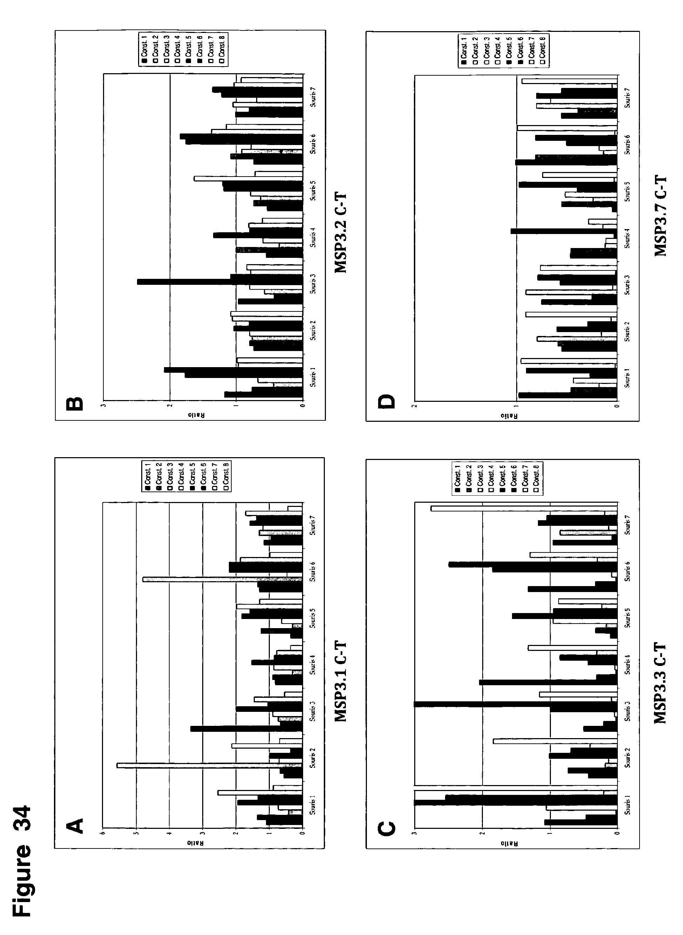

**Figure 34****: Homogeneity of the response (cytophilicity ratio) between each mouse (C57BU6) after the third immunization and against four MSP3 C-termini antigens.** Cytophilicity ratio of C57BL/6 mice against the C-terminal end of MSP3-1 (A), MSP3-2 (B), MSP3-3 (C) and MSP3-7 (D) after the third immunization with different multigenic constructs.

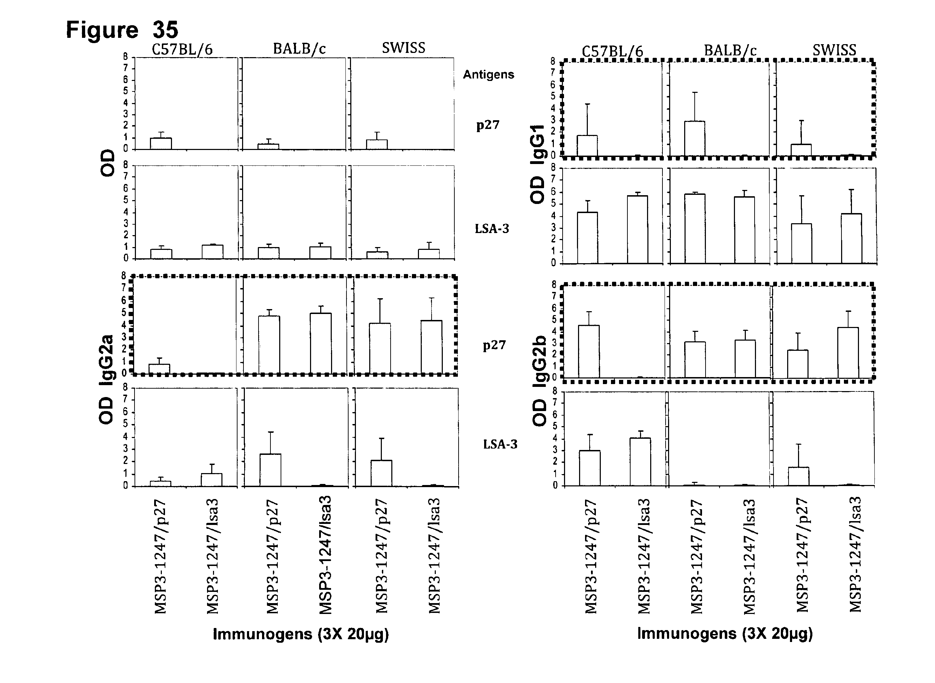

**Figure 35****: Cytophilic IgG1, IgG2a and IgG2b subclass response directed against the antigens p27 and LSA-3 in the 3 strains of mice (C57BU6, Balbc and SWISS) after the third immunization with MSP3 polyproteins.**

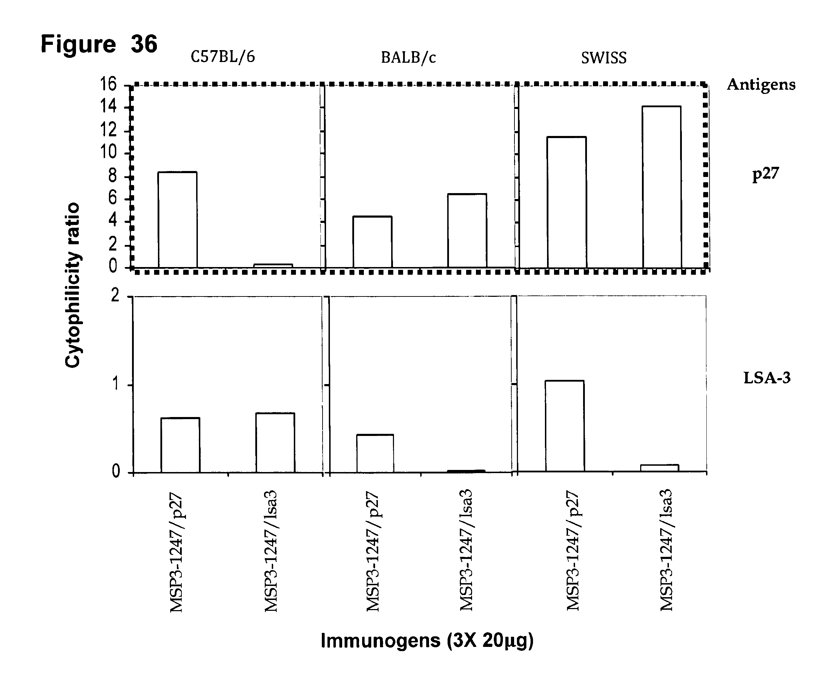

**Figure 36****: Cytophilicity ratio of three strains of mice (C57BU6, Balbc and SWISS) against the antigens p27 and LSA-3 after the third immunization with MSP3 polyproteins.**

### EXAMPLES

### Example 1 : Attempt to rationally design a malaria vaccine construct: example of the MSP3 family. Part I: Immunogenicity studies

*P. falciparum* Merozoite Surface Protein 3 (MSP3), which is the target of antibodies which mediate parasite killing in cooperation with blood monocytes and are associated with protection in exposed populations, is a vaccine candidate under development. It belongs to a family of six structurally related genes. To optimize immunogenicity we attempted to improve its design based on knowledge of antigenicity of various regions from the conserved C-terminus of the six proteins and an analysis of the immunogenicity of "tailored" constructs. The immunogenicity studies were conducted in BALB/c and C57BL/6J mice, using MSP3 (referred to as MSP3-1) as a model. Four constructs were designed in order to assess the effect of sequences flanking the 69 amino acids region of MSP3-1 previously shown to be the target of biologically active antibodies. The results indicate major beneficial effects of removing i) the sub-region downstream from the 69 amino acid sequence, as antibody titers increased by two orders of magnitude, and ii) the upstream sub-region which although it defines a T helper cell epitope, is not the target of antibodies. The construct excluding both flanking sequences was able to induce Th1 like responses, with a dominance of cytophilic antibodies. This led to design a multigenic construct based on those results, combining the six members of the MSP3 family. This new construct was immunogenic in mice, induced antibodies which recognized the parasite native proteins and inhibited parasite growth in the functional ADCI assay, thus satisfying the preclinical criteria for a valuable vaccine candidate.

### 1. A. Materials and methods

### Antigens

MSP3 recombinant proteins and synthetic peptides were designed on the basis of the *P.falciparum* 3D7 strain sequence. The denomination of peptides "a, b, c, d, e, f" is as described in (Singh et al., 2004). For MSP3-1(PlasmoDB ID: PF10_0345), two recombinant hexahistidine-tagged proteins MSP3-1(a-f) (MSP3-1-CTHis ₁₆₇₋₃₅₄) and MSP3-1(b-f) (MSP3-1-CTHis ₁₉₁₋₃₅₄) were produced, and purified as described elsewhere (Theisen et al., 1995) and then detoxified using Triton X114 (Reichelt et al., 2006). Seven peptides were synthesized as previously described (Roggero et al., 1997): five short peptides: MSP3-1 a ₁₆₇₋₁₉₁, MSP3-1b ₁₈₄₋₂₁₀, MSP3-1c ₂₀₃₋₂₃₀, MSP3-1d ₂₁₁₋₂₅₂ and MSP3-1e ₂₇₅₋₃₀₇, and two long peptides MSP3-1 LSP(a-d) (MSP3-1-LSP ₁₅₄₋₂₄₉) and MSP3-1 LSP(b-d) (MSP3-1 LSP ₁₈₄₋₂₅₀). For the other molecules of the MSP3 proteins family we expressed and purified, following the same protocol as for MSP3-1, five recombinant proteins corresponding to the C-terminal part of each: MSP3-2-CTHis₁₆₁₋₃₇₁ (PlasmoDB ID: PF10_0346); MSP3-3-CTHis₂₂₈₋₄₂₄ (PlasmoDB ID: PF10_0347); MSP3-4-CTHis₅₀₈₋₆₉₇ (PlasmoDB ID: PF10_0348); MSP3-7-CTHis₂₁₄₋₄₀₅ (PlasmoDB ID PF10_ 0352) and MSP3-8-CTHis₅₃₇₋₇₆₂ (Plasmo DB ID: PF10_0355).

The multigenic MSP3 construct, shown below, was designed on the basis of antigenicity studies, immunogenicity studies reported herein, and those previously obtained with MSP3-LSP_{154- 249} in human volunteers (Druilhe et al., 2005), and bioinformatics alignments to choose in each protein of the family, the sequence most related to the 69 amino acid region of MSP3-1 that proved crucial for vaccine development. The resulting multigenic construct combines in the following order those regions from respectively MSP3-1, MSP3-2, MSP3-3, MSP3-4, MSP3-7, MSP3-8:
[AKEASSYDYILGWEFGGGVPEHKKEENMLSHLYVSSKDKENISKENDDVL DEKEEEAEETEEEELEEK][LNNNILGWEFGGGAPQNGAAEDKKTEYLLEQI KIPSWDRNNIPDENEQVIEDPQEDNKDEDEDEETETENLETEDDNNEE][SN EKGRPPTYSPILDDGIEFSGGLYFNEKKSTEENKQKNVLESVNLTSWDKEDI VKENEDVK][LERGLGSGALPGTNIITEEKYSLELIKLTSKDEEDIIKHNEDVRE EIEEQQEDIE][YNHYFAWEIGGGAPTYKPENNKNDNILLEHVKITSWDKEDII KENEDTKREVQE][SKTIDPSKIDDRLELSSGSSSLEQHSKEDVKKGCALELV PLSLSDIEQIANESEDVLEEIEEEINTD] (SEQ ID NO: 71).

The multigenic construct is produced as a recombinant protein expressed in *Lactococcus lactis* and purified as described by Theisen et al., 2004.

### Mice

BALB/c and C57BL/6J aged 6 to 10 weeks old were purchased from Charles River laboratories (L'Arbresle, France).

### Immunization

For each experiment, groups of four to five mice were immunized with one of the four constructs for MSP3-1: MSP3-1 (a-f); MSP3-1(b-f) ; MSP3-1 LSP(a-d) or MSP3-1 LSP(b-d) , or with the C-terminal recombinant proteins of the other proteins of the family, or with the multigenic construct. Twenty micrograms of antigen adjuvated with Montanide ISA720 were administered subcutaneously to each mouse at two weeks intervals. As the cumulative effect of repeated immunizations were found of interest in preliminary experiments, we performed 3 immunizations, sampled the animals after the second and the third immunizations, and continued the immunizations to reach a total of 6 per animal.

### Determination of total IgG responses

Specific IgG responses to the various MSP3 constructs and MSP3 peptides were assessed by ELISA in sera collected from immunized mice. ELISA plates were coated overnight at 4°C with the MSP3 antigens diluted in PBS to a final concentration of either 5µg/ml (peptides) or 2.5 µg/ml (recombinant proteins). Wells were blocked with 300µl of PBS supplemented with 3% non-fat milk (Regilait, france) for one hour at room temperature and then washed in PBS containing 0.05% Tween 20. Sera were added at serial two fold dilutions (beginning at a 1/100 dilution) for 90 minutes at room temperature and washed as described above, followed by adding 50µl per well of HRP-conjugated goat anti-mouse IgG (Caltag, Invitrogen, Cergy Pontoise, France) diluted at 1:2000 in PBS/3% milk/0.05% Tween 20. Plates were incubated for one hour at room temperature, washed and then developed with 50µl of TMB for 15 minutes. Reactions were stopped by adding 50µl of 1 M H₃PO₄ and were read on an ELISA plate reader at 450nm.

### Assessment of Isotype distribution of antibodies

IgG sub-classes ELISA were performed essentially as described above, using class-specific second antibodies. Sera were tested in duplicate at a single dilution 1/1000. HRP-conjugated antibodies specific for mouse IgG subclasses (Caltag, Invitrogen, Cergy Pontoise, France) were used at dilutions of 1/4000 for anti-IgG1, anti-IgG2a and anti-IgG2b, and of 1/2000 for the anti-IgG3. Antibody concentration of each sub-class was determined using murine standards for IgG1, IgG2a, IgG2b or IgG3 (Zymed Laboratories, Invitrogen, Cergy Pontoise, France) at concentrations of 4, 8, 16, 32, 64, 128 and 256 ng/ml assayed in triplicate in 96-well microtiter plates coated with a 2 µg/ml of a goat anti-mouse immunoglobulins antibody (Caltag, Invitrogen, Cergy Pontoise, France).

### Western blots

Western blots were performed using a total parasite extract prepared from the 3D7 clone of *P.falciparum;* proteins were separated by gel electrophoresis under denaturing conditions (SDS-PAGE, 12% acrylamide), and then transferred onto nitrocellulose. Nitrocellulose membranes were blocked using TBS/ 5% milk and then incubated with sera diluted 1:100 in TBS/ 5% milk/ 0.05%Tween 20. Bound immunoglobulins were detected using an alkaline-phosphatase conjugated antibody (Promega, Charbonnieres-les-Bains, France) diluted 1/7500 in TBS/0.05% Tween 20 followed by an incubation with NBT/BCIP substrate (Promega, Charbonnieres -les-Bains, France).

### Immunofluorescence assays

Immunofluorescence assays were performed using air-dried, acetone fixed, thin smears from red blood cells cultures containing predominantly mature schizonts of *P.falciparum* (3D7). Sera diluted (1/100) in PBS/1%BSA were incubated at 37°C in a humid chamber for one hour. After washing with PBS, antibodies were detected using an alexa-fluor conjugated goat anti-mouse IgG (Molecular probes, Invitrogen, Cergy Pontoise, France) diluted to 1/400 in PBS with Evans' blue counterstain (1/200).

### T cell assays

Spleens were removed from mice sacrificed by cervical dislocation 7 days after the third or the sixth immunization. Splenocytes were resuspended at a concentration of 3x10⁶ cells/ml in RPMI supplemented with 100 U/ml penicillin, 100 µg/ml streptomycin, 2 mM L-glutamine, 50 µM β-mercaptoethanol, 1% non essential amino-acids, 10% fetal calf serum. Aliquots of 100 µl of cell suspension were distributed into round-bottomed 96-well microculture plates (Costar, Sigma, Lyon, France) and 100µl of each antigen was added at concentrations of 10, 20 or 40µg/ml. Splenocytes stimulated with 2.5 µg/ml of Concanavalin A (Sigma, Lyon, France) or with 1µg/ml LPS (Sigma, Lyon, France) were used as positive controls and with medium alone as negative controls. Cultures were performed in triplicate. Following 48-h culture (37 °C and 5% CO₂), 50 µl/well of supernatant was collected and stored at -70 °C for cytokine titration. [³H]Thymidine (Amersham Life Science, Buckinghamshire, UK) was added (1 µCi/well) during the last 12 h of a 72-h incubation period. The cells were harvested using an automatic cell harvester (Skatron, Sterling, VA, USA) and [³H]thymidine incorporation was quantified by scintillation counting. Stimulation index (SI) was calculated as follows: (mean cpm of triplicate test wells) / (mean cpm + 2 StDev of sextuplicate control wells). Proliferation was considered as positive when the Stimulation Index (SI) was > 1.

Cytokine concentrations were assayed in culture supernatants using the CBA flex set kit for mouse IFNγ, TNF, IL12p-70, MCP-1, IL-10, IL-6 and IL-4 (BD biosciences, Le Pont de Claix, France). Results were acquired on a FACS Calibur according to BD biosciences instructions and analysed using the FCAP array software (Soft Flow, Kedves, Hungary).

### Antibody dependent cellular inhibition Assay (ADCI)

The ADCI assay was carried out essentially as described previously (Bouharoun-Tayoun et al., 1990; Druilhe and Bouharoun-Tayoun, 2002a).

### 1. B. Results

The development of a malaria vaccine holds considerable promise, but has been limited by major conceptual and practical difficulties (Moorthy et al., 2004). We have chosen an approach based on the analysis of *P.falciparum-*human interactions in order to select both vaccine candidates and surrogate markers of protection able to guide vaccine development. Merozoite surface protein 3 (MSP3) is a blood stage antigen identified by this novel approach in which the protection, which could be passively transferred by IgG from protected African adults into naive, infected individuals (Sabchareon et al., 1991), was used to identify a mechanism of defence (Bouharoun-Tayoun et al., 1990; Oeuvray et al., 1994). The latter, called antibody-dependent cellular inhibition (ADCI), was used to identify MSP3 as the target of protective antibodies in humans.

We attempted to "tailor" vaccine constructs to design optimized vaccine candidate.

We have recently identified a series of 6 MSP3-related genes which are located downstream from the MSP3 gene in the same locus on chromosome 10, which share a similar organization of their C-terminal regions (Singh, 2004). These 6 MSP3-related genes showing structural and sequence relatedness and antigenic cross reactivity have been regrouped into a MSP3 family of proteins.

In the present study, we investigated the immunogenicity in mice of four MSP3-1 C-terminal based constructs and their ability to induce cytophilic antibodies against the region previously identified as target of protective antibodies (Singh et al., 2004). We also present here the immunogenicity study of one such tailored multigenic vaccine candidate.

### The MSP3-1 model: Immunogen components influence the B-cell epitope dominance and the induction of appropriate antibody responses against the 69 amino acid target region

MSP3-1 is the molecule that was first described among the MSP3 family of proteins. Immunoepidemiological studies and functional assays have led us to define a 69-amino acid region (amino acids 184-252; SEQ ID NO: 38), covering peptides MSP3-1 b, MSP3-1 c, and MSP3-1 d, as a target of protective antibodies (Singh et al., 2004). Sub-regions with identical sequence or a very high degree of homology were identified in other members of the MSP3 family. *We thus considered MSP3-1* as a *model, to provide indications for the design* of a *multigenic construct based on the MSP3 family.* We investigated in two mouse strains, BALB/c and C57BL/6J, the immunogenicity of four MSP3-1 constructs, in order to assess the effect of amino-acid sequences upstream and downstream from the amino acid 184-252 region upon the immune responses to the three peptides MSP3-1 b, MSP3-1c, and MSP3-1d.

In a first instance, we questioned whether the downstream region (amino acids 253-354) of this sequence of interest, containing peptides "e" and "f", had an effect on the induction of appropriate antibody responses. We therefore compared the MSP3-1 (a-f) and the MSP3-1 LSP(a-d) immunogens (figure 1-1). Results show that the inclusion of this region clearly impaired the anti-b responses (figure 1-1-1) in both mouse strains as well as the anti-c (figure 1-1-2) and anti-d (figure 1-1-3) responses in C57BL/6J mice.

In a second instance, we assessed the effect of the upstream region (amino acids167-184) of the 69 amino acid sequence, which corresponds to peptide MSP3-1a. The comparison of the immunogenicity of MSP3-1 (a-f) and MSP3-1 (b-f) constructs (figure 1-2) indicates that, in both mouse strains, the "a" sequence also has a deleterious influence upon the induction of anti-b responses (figure 1-2-1), however has no effect upon the anti-c (figure 1-2-2) and anti-d responses (figure 1-2-3). This deleterious effect of the "MSP3-1a" region upon the anti-b response was no longer detected when the MSP3-1 LSP(a-d) and the MSP3-1 LSP(b-d) were compared (figure 1-3), most likely because MSP3-1 LSP(b-d) proved to be poorly immunogenic. It did not induce antibody responses against any of the three peptides in BALB/c mice, and low anti-b response in C57BL/6J mice (Figure 1-3-1). Taken together, these results suggest that the simultaneous presence, and to a lesser extent the simultaneous absence, of both upstream and downstream regions of the 69 amino acid sequence profoundly impairs the immunogenicity towards this sequence targeted by biologically active antibodies, particularly towards peptide b. Considering both mouse strains, LSP(a-d) is the only construct that induced antibody responses against the three overlapping peptides covering the 69 amino acid target region.

### The MSP3-1 model: Immunogen components influence the Th1/Th2 balance

To determine the T cell responses induced by the MSP3-1 constructs immunization, we assessed the lymphoproliferation of splenocytes taken from immunized mice, in response to *in vitro* stimulation with the MSP3-1 short peptides or long constructs, at two distinct points during the follow up period (after the third and the sixth injection of the vaccine). Different epitopes were inducers of lymphoproliferation depending on the MSP3-1 construct used as immunogen.

Spleen cells taken from C57BL/6J mice immunized with MSP3-1(a-f) or MSP3-1 (b-f) showed no proliferation in response to stimulation with MSP3-1 peptides (a, b, c, d or e) after the third injection. Low lymphoproliferation was recorded in response to the stimulation with "e" peptide after the sixth immunization (SI_{6injections}=1.80 ± 0,48) with splenocytes isolated from the MSP3-1(b-f) immunized mice. MSP3-1 LSP(a-d) vaccinated mice splenocytes responded at the two time points against the "a" peptide only (SI_{3injections}=1,72±0,27; SI_{6injections}=2,58±0,46), whereas splenocytes taken from C57BL/6J mice immunized with MSP3-1 LSP(b-d) responded to the "c" peptide only (SI_{3injections}= 2,61 ± 0,48; SI_{6injections}=3,79 ± 0,22). Similar lymphoproliferation patterns were observed when using immunized BALB/c splenocytes. Higher lymphoproliferative responses were obtained when stimulating cells with the MSP3-1 recombinants or long peptides as compared to the short peptides.

In order to determine the Th1/Th2 balance of T cell responses, the levels of TNF, IFNγ, IL-10, IL-6, IL-12p70 and IL-4 were determined in 48h supernatants following *in vitro* stimulation by the four immunogens and by the five short peptides "a" to "e". Similar cytokine profiles were observed in supernatants of stimulated spleen cells from both mouse strains, and these profiles did not differ substantially after the third or the sixth immunization. Therefore we present here representative examples of this large series of experiments, those obtained in C57BL/6 after the 3^{rd} immunization (figure 2 and 3). Values for IL-12p70 and IL-4 are not reported on the figures, as IL-12p70 secretion was at the background levels and IL-4 was detected only in supernatants of spleen cells of MSP3-1 LSP(a-d) immunized groups.

Results obtained in MSP3-1 (a-f) immunized mice splenocytes did not indicate neither a Th1 profile (with predominant TNF and IFNγ secretion) nor a Th2 profile (with predominant IL-10 and IL-6 secretion): high levels of TNF secretion were detected upon boosting by the immunogen itself varying from 315 pg/ml to 836 pg/ml, but IFNγ levels were low and varied between 43 and 72 pg/ml. IL-10 secretion was heterogeneous, varying between 1 and 53 pg/ml and IL-6 levels varied between 48 and 74 pg/ml.

As shown in figure 2, in mice immunized with MSP3-1 (b-f) responses were skewed towards a Th2 profile of cytokines; following stimulation with the immunogen itself, high concentrations of IL-10 were detected (ranging from 101 to 1051 pg/ml), and IL-6 levels ranged from 37 to 57 pg/ml; on the contrary, IFNγ levels were borderline negative and TNF secretion was low (range between: 21 and 66 pg/ml).

The cytokine pattern observed upon stimulation of spleen cells taken from MSP3-1 LSP (a-d) immunized mice, corresponded clearly to a Th2 response: IL-10 and IL-6 levels were high, varying from 31 to 225pg/ml for IL-10 and from 32 to 145pg/ml for IL-6. In addition, IL-4 was detected in stimulated spleen cells supernatants of this group and ranged from 90 to 138pg/ml following the *in vitro* boost with the immunogen itself. We observed a high production of TNF (200 pg/ml to 1066 pg/ml) but very low levels of IFNγ (5 pg/ml to 26 pg/ml).

It is only in MSP3-1 LSP(b-d) immunized animals that a clear dominance of a Th1 profile of cytokines was observed (figure 3), as high concentrations of both TNF and IFNγ were detected following stimulation by each of the constructs or by MSP3-1 c peptide, whereas IL-10 and IL-6 levels remained low to moderate.

Taken together, these results confirm that immune responses towards the 69 amino acid target region are profoundly affected by the flanking sequences, as the immunogens including both the upstream and the downstream sequences of the 69 amino acid domain proved unable to induce T-cell responses towards the different peptides, and the deletion of both flanking regions was beneficial since the MSP3-1 LSP (b-d) construct was the unique construct having the property to elicit a dominant Th1 response.

### The isotype distribution of the antibody response confirms the Th1/Th2 balance observed

Since protective antibodies against *P.falciparum* blood stages have been shown to belong to cytophilic classes (Bouharoun-Tayoun and Druilhe, 1992a; Roussilhon et al., 2007), we analyzed the isotypes distribution of MSP3-1 antibodies induced in both mouse strains by each of the four MSP3-1 constructs, assuming that the non cytophilic IgG1 and IgG3 mouse isotypes would correspond to a Th2 response, whereas the cytophilic IgG2a and IgG2b would correspond to a Th1 response.

As shown in figure 4, non cytophilic antibodies (IgG1 and IgG3) are major components of the antibody response in sera of MSP3-1 (b-f) or MSP3-1 LSP (a-d) immunized mice. The same imbalance was observed in the BALB/c group immunized with MSP3-1 (a-f), whereas in the C57BL/6J mice there was rather an equilibrium between cytophilic and non cytophilic isotypes, which is consistent with the Th1/Th2 balance described above for the T cell response in these mice.

Interestingly, MSP3-1 LSP (b-d) is the only construct that induced a cytophilic antibody response, as more than 80% of the total specific IgGs consisted of cytophilic isotypes mainly belonging to the IgG2b subclass in C57BL/6J mice (figure 4), though no antibody response was induced in BALB/c mice (figure 1). This cytophilic response is in keeping with the Th1 cytokine profile described above.

### Immunogenicity of the five other C-terminal recombinant proteins

Prior to the construction of a new multigenic protein we investigated the immunogenicity of each of the five other members of the MSP3 protein family. We immunized both mouse strains with recombinant proteins corresponding to the five C-terminal regions (a-f) of MSP3-2, MSP3-3, MSP3-4, MSP3-7 and MSP3-8. As shown in figure 5-1, four of them induced antibodies in both mouse strains, which titers increased over repeated immunizations and ranged from one thousand to one million. Only MSP3-4 C-term, was somewhat less immunogenic than the remaining proteins in both mouse strains.

Due to structural homologies among the MSP3 protein family, particularly within peptides "b" and "d", antibodies elicited by one member cross-react with the remaining. This has been documented for antibodies elicited by MSP3-1 and MSP3-2 (Singh et al., 2005). In order to further document this phenomenon we tested the antibodies induced by the immunization with the C-terminal regions of MSP3-3, MSP3-4, MSP3-7 and MSP3-8 for their capacity to recognize all 6 MSP3 family members.

Antibody responses induced in BALB/c (figure 5-2) and in C57BL/6J (figure 5-3) immunized with each of the four MSP3 proteins cited above, were cross-reactive to the other six proteins though the degree of cross-reactivity varied. In general the highest titers were observed against the immunogen, and were lower but very substantial against the remaining 5 members of the family. MSP3-7 emerges as one generating the most homogeneous cross-reactive antibodies in BALB/c mice, whereas MSP3-3 and MSP3-8 generated rather homogeneous cross-reactivity in C57BL/6J mice.

### A multigenic construct elicits IgG reacting with the six MSP3 family proteins.

The first multigenic construct was designed on the basis of bioinformatics alignments determining in each MSP3 protein the cognate sequence to the 69 amino acid region defined on MSP3-1. The new protein is the combination of these six sequences, without addition of the upstream or the downstream sequences of the 69 amino acid domain, since immunogenicity studies reported above suggest that such addition might be deleterious. Its sequence is SEQ ID NO: 71. This molecule was used to immunize C567BL/6J and BALB/c mice. Specific IgG responses to the immunogen itself were induced in both mouse strains; titers ranged between 3 200 and 51 200 in C57BL/6J and 51 200 and 409 600 in BALB/c following the fourth immunization.

Sera from BALB/c mice immunized with the multigenic protein reacted in ELISA with all six MSP3 proteins (figure 6), whereas sera from C57BL/6J mice reacted more strongly with three of the six molecules: MSP3-1(a-f), MSP3-2 and MSP3-7; moderately with MSP3-3 and MSP3-4, and not with MSP3-8.

### Induced antibodies react with native parasite proteins.

As a vaccine should induce immune effectors that react with native proteins presented by the parasite, antibodies elicited in mice were assessed for reactivity in Western blot assays with *P.falciparum* blood stage extract and in Immunofluorescence assay upon mature blood forms.

Western blots using sera of C57BL/6J and BALB/c mice immunized with the multigenic protein show the recognition of parasitic proteins, notably of MSP3-1 (ca. 50Kd) and other MW corresponding to other members of the family. Sera from mice immunized with single proteins of the MSP-3 family were assessed too. The reactivity profile to native proteins of sera from MSP3-2 immunized mice was very similar to that observed with the sera of the MSP3-1 constructs immunized mice or of mice immunized with the multigenic protein; stressing out the high cross-reactivity between the MSP3-1 and the MSP3-2 proteins, possibly due to the identity among the two "b" peptide antigenic determinants (Singh et al., 2005). Sera collected from MSP3-3, MSP3-4, MSP3-7 or MSP3-8 immunized mice, did not cross-react with MSP3-1 or MSP3-2 and the MW they revealed corresponded to the respective native proteins.

The immunofluorescence assays also confirmed the recognition of native antigens on parasitized red blood cells or free merozoïtes by the sera collected from mice immunized with the new multigenic vaccine with titers reaching 6400.

### Isotypic distribution and functional activity of antibodies induced by the multigenic construct

The isotypic pattern of IgG elicited by the multigenic construct (SEQ ID NO: 71) was determined (figure 4). Results show that the isotypic distribution was well balanced between cytophilic (IgG2a and IgG2b) and non cytophilic (IgG1 and IgG3) subclasses in C57BL/6J mice, whereas an imbalance in favour of the IgG1 subclass was observed in BALB/c mice.

In order to assess their functional activity we tested the induced antibodies for their ability to inhibit parasite growth in the ADCI assay, carried out with additional controls using immunoglobulins from naïve mice to check for the specificity of the parasite growth inhibition obtained with antibodies prepared from sera of immunized mice.

For quantitative reasons, we analyzed the anti-parasitic activity of IgG prepared from a pool of sera collected from five BALB/c mice immunized four times with the multigenic construct. We assessed that this IgG preparation had kept its ability to cross-react with the six MSP3 recombinant proteins (OD values ranging from 2.5 to 4.3, similar to OD 4.2 recorded using the multigenic protein itself). In the ADCI assay, this IgG preparation was able to very efficiently inhibit the *in vitro* parasite growth, in cooperation with human monocytes. Indeed, the specific inhibition index (59,85% ± 1,62) was as high as that obtained with a pool of hyperimmune African sera IgG protective upon passive transfer in humans (63,36% ± 4,45).

### 1.C. Discussion

In the search for a malaria vaccine, we focused our studies on antigens targeted by the most potent immunity - that acquired over the years by individuals living in hyperendemic areas. This led us to characterize MSP3-1, and specially the highly conserved C-terminal part of it, as a most promising vaccine candidate. A first construct, a long synthetic peptide based on this C-terminal part, was tested in a phase I vaccine trial, and induced antibodies able to inhibit the parasite multiplication in vivo. However, a subset of the volunteers' sera failed to react with the native protein in Western blots (Audran et al., 2005; Druilhe et al., 2005). This occasional lack of recognition of parasite native proteins led us to re-examine, in the present work, the immunogenicity of this antigen. Our immunogenicity studies in two strains of mice led to the design of a multigenic vaccine, constructed from the six members of the MSP3 multi-gene family.

Our previous antigenicity studies, and functional assays of antibodies directed against the C-terminal region of MSP3, identified a 69 amino acids conserved domain, covered by the overlapping peptides MSP3-1 b, MSP3-1 c and MSP3-1d, as a target of biologically active antibodies to be included in MSP3-1 based vaccine constructs. Indeed, the MSP3-1 LSP tested in the phase I vaccine trial (Druilhe et al., 2005) corresponded to the region covered by peptides MSP3-1[a-d]. The present immunogenicity study in mice assessed the effect upon the response against the crucial region MSP3-1 [b-d] of the addition of the N-flanking sequence ("a") and of the C-flanking sequence ("e-f"). The addition of the sequence downstream from the 69 amino acid domain proved to be clearly deleterious. As, compared to the MSP3-1(a-f) construct, the MSP3-1 LSP(a-d) construct, induced markedly higher antibody and T-cell responses. Surprisingly, the deleterious effect of the C-flanking region was less intense in the absence of the MSP3-1 a region, as the antibody response induced by the MSP3-1 (b-f) construct was better than that induced by the MSP3-1 (a-f). These observations raise the question of the spatial conformation of these different constructs and of the possible interaction between the two sequences surrounding the sequence of interest, which both have a coiled structure, potentially allowing a coiled coil superstructure (Burgess et al., 2005; Mulhern et al., 1995) . The effect of the addition of MSP3-1 a was more complex. Whereas the comparison of the MSP3-1(a-f) vs the MSP3-1(b-f) construct suggests a deleterious effect related to the "a" region, the comparison of the MSP3-1 LSP(a-d) vs the MSP3-1 LSP(b-d) construct shows that the antibody response is somewhat better with the former. Indeed, the MSP3-1 LSP(a-d) construct, which has been conducted into clinics, was retro-validated as the only one able to induce a high and stable antibody response, in both mouse strains, against the three antigenic determinants (MSP3-1 b, MSP3-1 c, MSP3-1 d) targeted by biologically active antibodies. The MSP3-1 a peptide present in this construct defines a T cell epitope eliciting responses in mice, which is in accordance with the results of the human clinical trial (Audran et al., 2005). However, the MSP3-1 LSP(b-d) construct, in which the MSP3-1a sequence was eliminated, had the remarkable feature to induce a Th1 like cellular response in both mouse strains, and to induce mainly cytophilic antibodies in C57BL/6J. Thus, the deletion of the MSP3-1 a antigenic determinant, led to unvail a new T helper epitope located on the MSP3-1 c sequence which became immunodominant, in both BALB/c and C57BL/6J mice, though this improvement translated into a satisfactory humoral response in one mouse strain.

Taken together, these results indicated a deleterious effect of the sub-region downstream from the 69 amino acid sequence of interest, and shed light on the peculiarity of the MSP3-1 a sub-region: its sequence includes heptad repeats, with a corresponding coiled structure, that is unique to MSP3-1 (ie. not found in the other members of the MSP3 family); it contains an immunodominant T helper cell epitope, but was not the target of antibody responses neither in mice nor in humans immunized with the MSP3-1-LSP(a-d) construct (Audran et al., 2005). This is in accordance with the observation that in naturally exposed individuals, no substantial antibody response was detected against the MSP3-1a peptide (Singh et al., 2004). The flanking sequences thus profoundly affected the humoral and cellular immune responses towards the target [b-d] sequence. Activation of a B cell, implicates a direct recognition of the antigen in its native form by the B cell antigen receptor; as a consequence the humoral response is directly dependent on the structural context in which an antigenic determinant is presented, which could explain the differences we observed between the constructs in terms of antibody response directed against the various sub-regions. This may be the case also for T cell activation. Indeed, several groups have reported the influence of the antigen structure, ie. of the three-dimensional presentation of the epitopes, on the determination of the T helper cell epitope immunodominance (Mirano-Bascos et al., 2008; Nayak, 1999; Surman et al., 2001). They showed that the extent of antigen uptake by the antigen presenting cells, the rate of its processing and the affinity of the resulting peptides for MHC molecules were affected by its structure, which consequently should also affect the presentation to T cell receptor and the activation of T cells.

Antigenicity studies were conducted using similar peptides covering the 5 remaining proteins of the MSP3 family. These studies (Singh et al., 2005) have shown that each peptide was antigenic, a large proportion was the target of cytophilic antibody responses which also mediated the ADCI mechanism. The "b" peptide contains an 11 aminoacid stretch that is shared among 3 of the MSP3 proteins and generate full cross reactivity. The "d" peptide contains a 20 aminoacid sequence which is also present with limited sequence variation among the 6 members inducing cross-reactivity of antibody responses. Thus the 69 aminoacid region of interest is overall antigenically conserved across the MSP3 family. In contrast, the flanking regions are divergent from one gene to the other but are also fully conserved as MSP3-1 among diverse *P.falciparum* isolates. In contrast to other combined malaria vaccine candidates already in development, our approach was to group similar antigenic determinants from six proteins of the same family with the aim to increase immunogenicity towards cross-reactive sequences that are targets of protective immunity. However, the immunogenicity study of MSP3-1 considered as a model clearly demonstrated that the addition of various antigenic regions may profoundly affect the humoral and cellular responses to these targets. The results above finally led to a construct limited to the sub-regions homologous to MSP3-1 [b-d], that is excluding both the N-flanking regions (which markedly differed among the six proteins), and the C-flanking regions (with marked homologies, but suspected to be deleterious, as was established for the MSP3-1 [e-f] sub-region). This new construct (SEQ ID NO.:71) was immunogenic in mice, induced antibodies which recognized the parasitic native proteins and inhibited parasite growth in *in vitro* functional ADCI assays in cooperation with human monocytes, i.e. satisfied preclinical criteria.

### Example 2: Attempt to Rationally Design a Malaria Vaccine Construct; Example Of The MSP3 Family, Part II: Antigenicity Studies

*P.falciparum* MSP3 (MSP3-1), a main target of protective immunity against malaria belongs, together with MSP6 (MSP3-2), to a multi-gene family of six MSP3 proteins with similar structural organisation particularly in their highly conserved C-terminal regions. We performed a detailed antigenicity analysis of the other four proteins, similar to that already done for MSP3 and MSP6, using recombinant proteins and 24 peptides covering their C-terminal regions, and we evaluated the biological activity of the corresponding antibodies. A majority of malaria exposed individuals harbour antibodies to each MSP3 antigen, with a dominance of cytophilic IgGs. The 24 synthetic peptides were all antigenic, defining at least one epitope relevant of native proteins, with distinct IgG isotype pattern for each and an overall predominance of the IgG3 subclass. Human antibodies affinity-purified upon each of the 24 peptides exerted a *P.falciparum* killing effect in cooperation with blood monocytes (ADCI) in most cases as strong as that of IgG from protected African adults. Two regions "b" and "d" showing sequence homologies were found to generate in humans a broad network of cross reactive antibodies with various affinities. These findings suggest that all six members of the MSP3 family complement each other and play collectively an important role in protection. In addition, a first MSP3 multigenic construct (of SEQ ID NO: 71) designed in view of the data obtained from immunogenicity studies in mice was shown to have valuable immunological properties.

### 2. A. Materials and methods

### Antigens

The current Gene IDs in the data base PlasmoDB release 5.5 (www.plasmodb.org) of the *P. falciparum* MSP3 family proteins are:
PF10_0345, PF10_0346, PF10_0347, PF10_0348, PF10_0352 and PF10_0355 for MSP3.1, MSP3.2, MSP3.3, MSP3.4, MSP3.7 and MSP3.8, respectively. The set of six recombinant hexahistidine-tagged proteins that were designed to cover the related C-terminal regions of these MSP3 proteins are: MSP3.1₁₉₁₋₃₅₄, MSP3.2₁₆₁₋₃₇₁, MSP3.3₂₂₈₋₄₂₄, MSP3.4₅₀₈₋₆₉₇, MSP3.7₂₁₄₋₄₀₅ and MSP3.8₅₃₇₋₇₆₂. The overall percent homology for the MSP3 family sequences pairwise alignments obtained with EMBOSS (www.ebi.ac.uk/Tools/emboss/align/), which is between 32-49% for the full-length, increases to 43- 57% for the chosen C-terminal regions (figure 8-A).

Multiple sequence alignments for the MSP3 antigens were generated through ClustalW2 (www.ebi.ac.uk/Tools/clustalw2) and DIALIGN2 (mobyle.pasteur.fr). The scheme of alignments and further manual refinements were used to direct the design of 36 peptides i.e. 6 overlapping peptides covering the C-terminal part (excluding the glutamic acid rich region) for each of the 6 MSP3 antigens. The chemical peptides synthesis was carried out through standard stepwise solid phase peptide synthesis (GenScript Corp., Piscataway, NJ). The peptides sequences are detailed in Table 2 below.

### ELISA assays

The immunoepidemiological studies aimed at determining the prevalences and IgG subclasses distributions were performed using serum samples from the inhabitants of Dielmo, a village localised in an area of Senegal, West Africa, where malaria is holoendemic (Trape et al., 1994). These samples were collected during a cohort prospective follow-up study designed to identify and analyse all malaria episodes of morbidity (Trape et al., 1994). The detection of total IgG and IgG subclasses was performed by ELISA as described previously (Bouharoun-Tayoun and Druilhe, 1992b; Druilhe and Bouharoun-Tayoun, 2002), using secondary monoclonal antibodies originally selected as reacting faithfully with African subclass dominant allotypes. For IgG1 to IgG4 monoclonal mouse anti-human subclasses (clones NL16 = IgG1 [Boehringer Mannheim, Meylan, France], HP6002 = IgG2 [Sigma-Aldrich, Steinheim, Germany], Zg4 = IgG3, and RJ4 = IgG4 [both from Immunotech, Marseille, France]) were used, at a final dilution of 1/2,000; 1/10,000; 1/10,000; and 1/1,000, respectively. For the total IgG detection, the HRPO-conjugate goat anti-human (Caltag-Invitrogen, Carlsbad, CA) was used at a 1/3,000 dilution. For each antigen (peptides or recombinant proteins), the results were expressed as OD ratios, or Arbitrary Units, calculated by dividing the optical density (OD) of each serum sample over the mean OD + 3 standard deviations of 5 control serum samples (Roussilhon et al., 2007). These control sera were randomly selected among French blood donors without exposure to malaria.

### Affinity Purification of Specific Antibodies

The antibodies specific of the 24 overlapping peptides covering the C-terminal sequence of MSP3.3, MSP3.4, MSP3.7 and MSP3.8 were affinity-purified from a Pool of sera of 333 rural African adults from Ivory Coast, West Africa, having reached a state of premunition, similar to that employed for the IgG passive transfer experiment ( Sabchareon et al., 1991).

Antibodies specific to either peptides or recombinant antigens were prepared as described previously (Brahimi et al., 1993; Singh, et al., 2004). Briefly, plain polystyrene beads (mean diameter, 10 µm; Polysciences, Eppelheim, Germany) were coated with antigens in a 5% (w/v) PBS suspension, and washed thoroughly in PBS. The coated beads were incubated during 2 hours at room temperature with the PIAG diluted 1/10, then washed twice in PBS-0.01% Tween-20. The antibodies captured on the beads were eluted using a Glycine buffer 0.2M, pH 2.5, and immediately neutralised with the required volume of Tris buffer 2M, pH 11.2. The antibodies eluted were dialysed extensively against PBS, followed by RPMI, and then concentrated by centrifugation using the molecular sieve Vivaspin2 (30,000 MWCO PES, Sartorius Stedim, Göttingen, Germany). They were stored at 4°C after addition of 1% Albumaxl (Gibco Invitrogen, Carlsbad, CA). The specificity of affinity-purified antibodies was ascertained by ELISA on each peptide, using bovine serum albumin and the peptide SALSA-1 from the Sporozoite and Liver Stage Antigen-1 (Bottius et al., 1996), as negative controls. The purity of antibodies preparation was ascertained in SDS-PAGE using a 10% polyacrylamide gel.

### ImmunoFluorescence Assays (IFA)

Indirect Immunofluorescence assays were performed by previously described techniques in order to assess recognition of the native parasite proteins by affinity-purified antibodies (as disclosed for example in Singh et al., 2004). Air-dried acetone-fixed thin smears from the 3D7 P. falciparum clone at mature schizont stage were incubated with serial dilutions of affinity-purified antibodies, and using at a 1/200 dilution a fluorescein labelled Goat anti-human IgG (Alexa Fluor 488, Invitrogen, Carlsbad, CA) as second antibody.

### Functional ADCI assays

The *P. falciparum 3D7* clone (MRA-102, MR4, ATCC, Manassas, VA) was cultivated in RPMI-1640 supplemented with 0.5% Albumaxl (both from Gibco Invitrogen, Carlsbad, CA), as described earlier (Jensen, 2002). Schizonts enrichment by floatation on 1% (w/v) gelatin (Sigma-Aldrich, Steinheim, Germany) was performed 48 hours before initiating the ADCI-based functional assays.

IgG was extracted from the same pool of sera used for affinity purification, by ion exchange chromatography on DEAE Ceramic HyperD F (Pall BioSepra, Cergy-Saint-Christophe, France) and named PIAG (Pool of Immune African Globulins). In the ADCI assays performed to evaluate the biological activity of antibodies specific to each antigen, two positive controls were used: the PIAG, and antibodies affinity-purified on MSP3.1-Cterm. The negative control was IgG from a pool of French donors with no malaria history (N-IgG). Peripheral blood mononuclear cells (PBMC) from healthy French donors were prepared and cryopreserved as previously described (Jafarshad et al., 2007). For ADCI assay, PBMC were rapidly thawed at 37°C and assessed for viability by trypan blue exclusion. Cell suspensions were adjusted to a concentration of 10x10⁶ cells per ml with RPMI-1640, and 200 µl/well distributed in flat bottom 96-well plates (TPP, Trasadingen, Switzerland) were incubated for 1 hour at 37°C, 5% CO₂. The non-adherent cells were removed by two washing steps with RPMI-1640, then the cell appearance and the relative homogeneity of distribution of the adherent cells in the different wells, were controlled by observation under an inverted microscope. Antibodies affinity purified upon the different peptides or recombinant antigens were tested at a final concentration yielding a 1/20 IFA titre, the N-IgG and PIAG were used at 2mg/ml (about 10% the IgG concentration in African adults sera). To each well were added 50 µl of the test antibody in RPMI-1640 and 50 µl of an infected red blood cells suspension in RPMI-1640 with 1% Albumaxl at a final hematocrit of 2.5% and 0.5% parasitemia. The control wells consisted of either monocytes alone, or monocytes with N-IgG, or parasite culture with antibodies but without monocytes or parasite alone (Druilhe and Bouharoun-Tayoun, 2002a; Oeuvray et al., 1994). At 48 and 72h, 50µl of complete culture medium were added to each well. Following cultivation for 96 h, the level of parasitemia was determined on Giemsa-stained thin smears from each well by microscopic examination of at least 50,000 erythrocytes. The Specific Growth Inhibitory index (SGI), which takes into account the possible inhibition induced by monocytes or antibodies alone, was calculated as follows: SGI= 100x[1-(parasitemia with monocytes and test IgG/parasitemia with test IgG)/(parasitemia with monocytes and N-IgG/parasitemia with N-IgG)], where parasitemia are expressed as percentage of infected red blood cells. The ADCI assays were performed in duplicate for each affinity-purified antibody, using monocytes showing less than 10% direct non-specific inhibition of parasite growth.

### Cross-reactivity studies

In a first set of experiments, the cross-reactivity of antibodies was assessed by competition ELISA as described elsewhere (Friguet et al., 1995), with competing antigen at concentrations ranging from 0 to 800µg/ml. The results expressed as percentage reactivity were calculated as follows:
[(mean O.D values with the competing antigen at a given concentration)/ (mean O.D values without the competing antigen)]×100. To control the specificity of the competition between antigen and antibody, the non-relevant antigen SALSA-1 was added at the same concentration as the competing antigen.

In addition, the cross-reactivity network among MSP3 family within the peptide "d" sequence was dissected by analysing in ELISA the reactivity of antibodies specific to each peptide "d" upon the six recombinant MSP3-Cterm antigens.

### Avidity studies

The binding strength of antibodies induced in mice by a first MSP3 multigenic construct of sequence SEQ ID NO: 71 (see example 1), was evaluated by ELISA towards each MSP3 recombinant C-terminus protein as described elsewhere (Pullen et al., 1986.). Briefly, antibodies affinity purified upon the MSP3 multigenic construct from a pool sera of immunized mice were tested on microplates coated with each of the six MSP3 recombinant proteins. After allowing the formation of antigen-antibody complexes, the latter were dissociated with increasing concentrations of the chaotropic ion ammonium thiocyanate (NH₄SCN), from 0.25 to 4 molar. Then the remaining antibodies were quantified. The percentage reactivity was calculated as follows: [(mean O.D values with a given concentration of NH₄SCN)/ (mean O.D values without NH₄SCN)]×100. The affinity index representing the molar concentration of NH₄SCN required to reduce the initial optical density by 50% were estimated.

### 2. B. Results

Malaria vaccine development is both urgently needed and remarkably challenging in scientific terms, particularly in the choice of concepts leading to decide of a given path over others towards this end.

The criteria employed in the decision-making process affect obviously the choice of a candidate among the 5300 *P.falciparum* proteins, *i.e.* the vaccine discovery process, but they also affect markedly the type of construct, expression system or formulation chosen, as well as the need for combinations and obviously the selection of candidates to be included in such combinations (Genton and Reed, 2007).

Given the unknown and debatable relevance of experimental models, we have made the choice to base our research orientations on clinical observations, as we believe they constitute the sole reliable information. For vaccine discovery, passive transfer experiments of protective IgG into *P.falciparum* infected receivers (Bouharoun-Tayoun et al., 1990; Sabchareon et al., 1991) led us to compare non-protective with protective antibodies upon each receiver isolate, and to identify a novel defence mechanism which had not been foreseen by research in models, in which the effective antibodies act in a monocyte dependent manner, the ADCI mechanism (Bouharoun-Tayoun et al., 1990; Bouharoun-Tayoun et al., 1995). The latter, in turn led to identify MSP3 as a main target of protective antibodies (Oeuvray, et al., 1994).

MSP3 is thus an antigen that has been chosen by a defence mechanism validated by a clinical experiment, its C-terminal region is fully conserved across *P.falciparum* ( McColl and Anders, 1997; Roussilhon et al., 2007) in contrast to most other vaccine candidates, and studies in exposed populations show that it induces antibody responses that are strongly associated with clinical protection against disease ( Polley et al., 2007; Roussilhon et al., 2007; Soe et al., 2004).

A detailed antigenic analysis of the C-terminal part of MSP3 highlighted the importance of 3 antigenic determinants targeted by naturally occurring cytophilic antibodies that inhibit parasite growth in functional *in vitro* ADCI assays (Singh, et al., 2004). This information was instrumental in the design of the construct that was taken into the clinics, the MSP3-LSP (Audran et al., 2005). The latter induced in human volunteers antibodies that inhibited *P.falciparum* multiplication both *in vitro* and *in vivo* (Druilhe et al., 2005).

Meanwhile, we had identified that MSP3 belongs to a multigene family, differing in its features from all other *P. falciparum* gene families. The six msp3 genes are transcribed simultaneously, the corresponding proteins are expressed by parasites at late schizont stage and in all isolates investigated (Singh, Barnwell *et al.* manuscript in preparation). Their C-terminal regions share a similar structural organization, include regions with sequence homologies flanked by regions that markedly differ from one gene to the other, and yet are very well conserved among isolates. Thus the original MSP3 was renamed as MSP3.1, MSP6 (Trucco et al., 2001) as MSP3.2, and the remaining MSP3.3, MSP3.4, MSP3.7 and MSP3.8 (figure 14). In view of the unique features of this MSP3 family, particularly the high degree of conservation of homologous and divergent sequences implying that they had a role for parasite survival, we therefore thought it valuable to develop a multigene MSP3 construct combining chosen regions among the most immunologically relevant from these six genes in a manner which may allow the design of optimized constructs for the purpose of inducing immunogenic response in a human host.

We performed immunogenicity and antigenicity studies and and combined data generated in this manner to define novel vaccine constructs. Immunogenicity and antigenicity data are complementary: the former analyze the responses induced by a given vaccine formulation in experimental models, however have the great limitation to do so in animals whose relevance to humans is not granted. In contrast the latter have the paramount advantage to identify immune responses developed by the target population, human beings, although these responses have been induced by exposure to the whole parasite.

Here we characterized the immune responses directed to a series of peptides covering the C-terminal parts of MSP3.3, MSP3.4, MSP3.7 and MSP3.8, including the assessment of the biological anti-parasite activity of naturally acquired human antibodies, and compared them to similar studies previously performed with MSP3.1 and MSP3.2 (Singh, et al., 2004; Singh et al., 2005). The resulting data obtained with 36 peptides of the MSP3 family combined with the detailed study of the immunogenicity of various regions of the C-terminal part of MSP3.1, reported in Example 1 led to design a first MSP3 family-based multigenic vaccine construct, which was shown to have valuable immunological features.

### Antibodies specific of the six MSP3 antigens are highly prevalent in subjects from malaria endemic area.

IgG antibodies specific for all the six MSP3 antigens were detected in serum samples from adults living in the village of Dielmo, Senegal (Table 1). Prevalences were very high, ranging from 89% to 95%, even though IgG levels varied from one antigen to the other (Table 1).

**TABLE 1: Total IgG responses upon the C-terminal part of the six MSP3 family proteins in hyperimmune serum samples from adult individuals (n=45) from Dielmo, Senegal. To calculate the prevalence values, responses were considered positive when the net OD value of each serum sample obtained by subtracting the mean OD+3SD of control serum samples, was > 0. The mean OD represents the mean responses of the positive serum samples.**

| | **Mean OD Value** | **Prevalence (%)** |
|---|---|---|
| **MSP3.1-Cterm** | 2.23 | 91.1% |
| **MSP3.2-Cterm** | 2.47 | 88.9% |
| **MSP3.3-Cterm** | 1.87 | 93.3% |
| **MSP3.4-Cterm** | 2.26 | 95.6% |
| **MSP3.7-Cterm** | 1.27 | 91.1% |
| **MSP3.8-Cterm** | 1.75 | 91.1% |

The high antibody reactivity obtained with MSP3.2 was similar to that obtained with MSP3.4 or previously with MSP3.1. The lowest one was upon MSP3.7, and was in the same range as that of MSP3.8 and MSP3.3. In view of the role of cytophilic classes in parasite killing and of their association with protection, we further determined the IgG1 and IgG3 responses upon the six MSP3 antigens using a panel of 76 malaria exposed individuals of all ages from the same village (figure 7). The results confirm the high prevalence of responses, since for each antigen, except MSP3.8, the prevalences were ≥ 80% for IgG1 or IgG3. While IgG3 antibodies were dominant in the case of MSP3.2 and MSP3.4, the responses upon the four remaining antigens were balanced between IgG1 and IgG3 (figure 7).

### Peptides Design

Six overlapping peptides covering the C-terminal region of the proteins (figure 15) were designed for each of the four MSP3 proteins MSP3.3, MSP3.4, MSP3.7 and MSP3.8 in order to fit those previously designed for MSP3.1 and MSP3.2 (Singh, et al., 2004; Singh et al., 2005), and were named, as was the case for the latter, peptides "a, b, c, d, e, f'. In addition to their overall structural homology, the proteins of the MSP3 family share sequences similarities in their C-terminal parts (figure 8-A). The first conserved motif, made of eleven amino acids residues centred on three glycine residues (figure 8-B box I), is included in the peptides called "b" and the sequences upstream and downstream are named "peptides a" and "peptides c", respectively (see table 2 below). For each MSP3 antigen the "peptide d" located upstream of the glutamic acid rich region is characterised by a stretch of 20 amino acids with high position specific scoring *i.e.* identical or homologous residues being conserved for a given position (figure 8-B, box II). The leucine-zipper like pattern located downstream the Glu-rich region (figure 8-B box III) is displayed in the "peptides f", downstream those of "peptides e" (see table 2 below).

**TABLE 2: Single letter amino acids sequences of the overlapping peptides covering the C-terminal parts of the proteins of the MSP3 family.**

| **NAMES OF THE MOTIFS** | **POSITION IN MSP3 PROTEINS** | **SEQUENCE OF THE MOTIFS** | **SEQ ID NO.:** |
|---|---|---|---|
| **MSP3.1a** | MSP3.1₁₆₇₋₁₉₁ | YEKAKNAYQKANQAVLKAKEASSYD | 1 |
| **MSP3.1b** | MSP3.1₁₈₄₋₂₁₀ | AKEASSYDYILGWEFGGGVPEHKKEEN | 2 |
| **MSP3.1c** | MSP3.1₂₀₃₋₂₃₀ | PEHKKEENMLSHLYVSSKDKENISKEND | 3 |
| **MSP3.1d** | MSP3.1₂₁₁₋₂₅₂ | MLSHLYVSSKDKENISKENDDVLDEKEEEAEETEE EELEEK | 4 |
| **MSP3.1e** | MSP3.1₂₇₅₋₃₀₇ | EEENDKKKEQEKEQSNENNDQKKDMEAQNLISKN | 5 |
| **MSP3.1f** | MSP3.1₃₀₂₋₃₅₄ | NLISKNQNNNEKNVKEAAESIMKTLAGLIKGNNQID STLKDLVEELSKYFKNH | 6 |
| **MSP3.2a** | MSP3.2₁₆₁₋₁₈₂ | SETNKNPTSHSNSTTTSLNNN | 7 |
| **MSP3.2b** | MSP3.2₁₇₉₋₂₀₄ | LNNNILGWEFGGGAPQNGAAEDKKTEY | 8 |
| **MSP3.2c** | MSP3.2₁₉₂₋₂₂₄ | PQNGAAEDKKTEYLLEQIKIPSWDRNNIPDENE | 9 |
| **MSP3.2d** | MSP3.2₂₀₅₋₂₅₇ | LLEQIKIPSWDRNNIPDENEQVIEDPQEDNKDEDED EETETENLETEDDNNEE | 10 |
| **MSP3.2e** | MSP3.2₂₈₂₋₃₂₆ | EEEKKEEKKEEKKPDNEITNEVKEEQKYSSPSDINA QNLISN | 11 |
| **MSP3.2f** | MSP3.2₃₂₀₋₃₇₁ | NLISNKNKKNDETKKTAENIVKTLVGLFNEKNEIDST INNLVQEMIHLFSNN | 12 |
| **MSP3.3a** | MSP3.3₂₂₈₋₂₅₂ | YEKKNENKNVSNVDSKTKSNEKGR | 13 |
| **MSP3.3b** | MSP3.3₂₄₆₋₂₇₂ | SNEKGRPPTYSPILDDGIEFSGGLYF | 14 |
| **MSP3.3c** | MSP3.3₂₆₂₋₂₉₁ | GIEFSGGLYFNEKKSTEENKQKNVLESVN | 15 |
| **MSP3.3d** | MSP3.3₂₇₆₋₃₀₈ | STEENKQKNVLESVNLTSWDKEDIVKENEDVK | 16 |
| **MSP3.3e** | MSP3.3₃₅₂₋₃₈₅ | DTSDLEKKNIPDLSNDNNYYSLIYKNYKDNDKS | 17 |
| **MSP3.3f** | MSP3.3₃₇₂₋₄₂₄ | SLIYKNYKDNDKSEKTAQTLITALISLLNGKNELDATI RRLKHRFMEFFTYN | 18 |
| **MSP3.4a** | MSP3.4₅₀₈₋₅₃₃ | DNVNSVTQRGNNNYNNNLERGLGSG | 19 |
| **MSP3.4b** | MSP3.4₅₂₅₋₅₅₂ | LERGLGSGALPGTNIITEEKYSLELIK | 20 |
| **MSP3.4c** | MSP3.4₅₄₄₋₅₇₂ | KYSLELIKLTSKDEEDIIKHNEDVREEI | 21 |
| **MSP3.4d** | MSP3.4₅₅₂₋₅₈₀ | LTSKDEEDIIKHNEDVREEIEEQQEDIE | 22 |
| **MSP3.4e** | MSP3.4₆₀₅₋₆₃₉ | EENKEKELSNQQQSEKKSISKVDEDSYRILSVSY | 23 |
| **MSP3.4f** | MSP3.4₆₄₆₋₆₉₇ | RILSVSYKDNNEVKNVAESIVKKLFSLFNDNNNLETI FKGLTEDMTDLFQK | 24 |
| **MSP3.7a** | MSP3.7₂₁₄₋₂₄₀ | PEGPRANNRNENNQNTDPYNHYFAWE | 25 |
| **MSP3.7b** | MSP3.7₂₃₂₋₂₅₄ | YNHYFAWEIGGGAPTYKPENNK | 26 |
| **MSP3.7c** | MSP3.7₂₄₅₋₂₇₂ | PTYKPENNKNDNILLEHVKITSWDKED | 27 |
| **MSP3.7d** | MSP3.7₂₅₇₋₂₈₆ | ILLEHVKITSWDKEDIIKENEDTKREVQE | 28 |
| **MSP3.7e** | MSP3.7₃₂₄₋₃₅₉ | VNLEDINKNTRNDIFEEQIKLDSTQDDKAQKLISN | 29 |
| **MSP3.7f** | MSP3.7₃₅₃₋₄₀₅ | QKLISNEYKKTEEKKSLEDHVNLLFNFLQTNNQLDP SLKDLENELTFFLNNY | 30 |
| **MSP3.8a** | MSP3.8₅₃₇₅₇₀ | HESNVGSIQEVNQGSVSEESHSKTIDPSKIDDR | 31 |
| **MSP3.8b** | MSP3.8₅₆₄₋₅₉₃ | SKTIDPSKIDDRLELSSGSSSLEQHSKED | 32 |
| **MSP3.8c** | MSP3.8₅₈₆₋₆₁₅ | EQHSKEDVKKGCALELVPLSLSDIEQIAN | 33 |
| **MSP3.8d** | MSP3.8₅₉₈₋₆₃₁ | ALELVPLSLSDIEQIANESEDVLEEIEEEINTD | 34 |
| **MSP3.8e** | MSP3.8₆₆₉₋₇₁₀ | EIEDKPEQEIKNKSLEEKQIDKNTDTSEKKGFNNSE KDEKA | 35 |
| **MSP3.8f** | MSP3.8₇₀₁₋₇₆₂ | NNSEKDEKARNLISKNYKNYNELDKNVHTLVNSIISL LEEGNGSDSTLNSLSKDITNLFKN | 36 |

### The 24 peptides from MSP3.3, MSP3.4, MSP3.7 and MSP3.8 are all antigenic and predominantly targeted by cytophilic antibodies

The detailed analysis of the IgG subclasses distribution among a panel of malaria exposed individuals of all age groups, against the twenty-four peptides covering the C-terminal regions of these four antigens, showed that each of them was recognized by antibodies from endemic area sera, i.e. all were antigenic. The prevalences of subclasses specific antibodies were high, > 60% of the individuals (figure 9) for two thirds of the peptides.
For all of them the antibodies were predominantly of IgG1 and IgG3 subclasses, and only one (MSP3.8b) was targeted by a substantial non cytophilic IgG2 response. In most cases, the IgG1 and IgG3 levels were of similar magnitude, but a predominance of IgG3 was observed against some peptides (MSP3.3a, MSP3.4b and MSP3.7e), or of IgG1 for others (e.g. MSP3.3b and MSP3.7d) (figure 9). The present results together with those previously reported for MSP3.1 and MSP3.2 (Singh, et al., 2004; Singh et al., 2005), show that the subclass patterns of responses to the homologous peptides b, d or f from the six antigens of the MSP3 family differ markedly.
This suggests that the flanking regions may play an important role in the modulation of the IgG1 to IgG3 balance. Additional differences emerge for peptide "a": in contrast to the low prevalence and low levels of antibodies previously observed to peptide MSP3-1a, high prevalences and levels are observed for MSP3-3a and MSP3-8a. Conversely responses to the various peptides "b", which is a major target of ADCI for MSP3-1, are high in 5 out of 6 proteins (i.e. all except MSP3-7).

### Multiple targets of antibodies triggering ADCI are found throughout the MSP3 family

Human antibodies affinity purified upon each of the 24 peptides of MSP3.3, MSP3.4, MSP3.7 and MSP3.8, recognized efficiently both the corresponding peptide and the recombinant C-terminus region (not shown). More importantly, these affinity purified antibodies were all found able to recognize the parasite native proteins in ImmunoFluorescence Assays (IFA), indicating that each synthetic peptide mimicked at least one natural parasite epitope.

In order to allow comparisons in ADCI assays, these antibodies were adjusted according to their IFA titre. As shown in figure 10, each of the 24 peptides from the four antigens was found to be a target of human antibodies with potent Monocyte-dependent antiplasmodial activity in the ADCI assay. For few of them (anti-3.3c, anti-3.3f, anti-3.4c and anti-3.7c) the ADCI effect was only marginal, yet significant, whereas for the majority it was comparable to that of the PIAG, i.e. IgG of protected African adults. The anti-plasmodial effect mediated by some of them, such as anti-3.3e, anti-3.4b and anti-3.8d antibodies was even higher than that observed using the PIAG. This strong effect is remarkable as each peptide specific antibody corresponds to a very minor subset of total malarial antibodies and a small subset of antibodies directed to the 6 MSP3 family proteins. Although antibodies specific to the recombinant antigens were all efficient in ADCI (figure 10), the antibodies specific to several peptides (MSP3.3a, MSP3.3e, MSP3.4b, MSP3.4b, MSP3.7a, MSP3.8a, MSP3.8b, MSP3.8d, MSP3.8e, MSP3.3f) mediated an effect stronger than that observed with the corresponding recombinant antigen. These results differ from those obtained previously with MSP3.1 where only 3 of the 6 peptides were the target of biologically active antibodies (Singh, et al., 2004). They are in keeping with results obtained using MSP3.2 peptide-specific antibodies which all had a strong inhibitory effect (Singh et al., 2005). Thus, the present data, complement those obtained in our two previous studies, to characterize a very large series of targets for antibodies mediating the killing of *P. falciparum* parasite through the ADCI mechanism.

### Cross-reactions among MSP3 family members

As mentioned above, a stretch of eleven amino acids is quasi-identical in the peptides "b" derived from MSP3.1 and MSP3.2, and highly homologous in MSP3.7b, which suggests that antibodies specific to these peptides may be cross-reactive. Indeed, in competition ELISA assays, the MSP3.2b peptide at 200µg/ml concentration was able to decrease by 80% the binding of anti-MSP3.1b antibodies to MSP3.1-Cterm. Similarly, the binding of anti-MSP3.7b was decreased by 60% by the competing MSP3.1 b peptide (figure 11). These findings extend the cross-reactivity observed previously between MSP3.1 b and MSP3.2b (Singh et al., 2005). The competition was less efficient between MSP3.1 b and MSP3.7b than between MSP3.1 b and MSP3.2b (figure 11), most probably due to the fact that, within the highly conserved stretch of eleven amino acids of MSP3.7b, there are substitutions in the first three positions: YFA vs. ILG, and in the 6th position F/I, whereas MSP3.1 b and MSP3.2b only differ by a V/A substitution in the 10^{th} position.

The cross reactivity was also assessed among the second group of holomogous peptides, i.e. peptides "d" (figure 8-B, box II), using antibodies immunopurified on each peptide. The cross-reactivity network summarized in figure 12 indicates that naturally occurring human antibodies specific to "peptide d" of each MSP3 protein cross-reacted with at least one other antigen of the MSP3 family. Anti-MSP3-8d presented cross-reactivity with only one other member of the gene family, whereas anti-MSP3.1 d and anti-MSP3.2d presented cross-reactivities with each of the remaining five proteins, *i.e.* reacted with all 6 MSP3 proteins. On the other hand, MSP3.4-Cterm and MSP3.8-Cterm appeared as the "most antigenic", as they were recognized by antibodies immuno-purified on each of the six "d" peptides. Conversely, these results indicate that the limited point mutations occurring in the highly conserved motif of the peptides "d" can also induce true antigenic divergence despite a high level of sequence homology. In this manner the various homologous sequences generate a large number of distinct antibody species with a wide pattern of reactivity to the 6 proteins.

### A MSP3 multigenic construct induces in mice antibodies cross-reacting with each antigen of the MSP3 family

The above studies show that sequence homologies among MSP3 proteins generate a large network of cross-reactions, despite sequence divergences particularly among the flanking regions. Based on the combination of data from immunogenicity studies reported in example 1 and the present antigenicity data, a MSP3-multigene construct covering the "b-c-d" peptide sequences was designed and used to immunize mice. The mice antibodies affinity-purified upon the synthetic MSP3s-multigenic protein reacted with each of the six antigens of the MSP3 family. However they differently recognized each MSP3 antigen as affinity indexes were estimated as 0.4M, 0.5M, 0.7M, 0.9M, 2.6M and 2.8M for MSP3.1, MSP3.4, MSP3.7, MSP3.8, MSP3.3 and MSP3.2 respectively. Therefore avidity of these antibodies was high for MSP3.3 and MSP3.2, medium for MSP3.8, MSP3.7 and low for MSP3.4 and MSP3.1 (figure 13).

These results, and those above, indicate that the antigens of the MSP3 family are the targets of a family of cross-reactive antibodies, with wide differences in terms of avidity and specificity.

### 2. C. Discussion

In order to design in rational manner a multi-subunit MSP3 vaccine, we performed a detailed immunological study of the remaining four members of the MSP3 family of proteins, to complement the data previously obtained on the other two, MSP3.1 and MSP3.2 (Singh et al., 200; Singh et al., 2005). As a complement to the immunogenicity study reported in example 1, we performed in the present work a detailed antigenicity analysis of the homologous C-terminal parts of these proteins, and evaluated the biological activity of the corresponding antibodies. As in our previous studies with MSP3.1 and MSP3.2, we used both recombinant proteins and peptides corresponding to homologous or divergent regions, in order to select, based on immune responses from exposed individuals, those to include in vaccine constructs.

Using sera from subjects covering all ages living in a hyperendemic region of Senegal, we found that the conserved C-terminal regions of the six MSP3 proteins were all antigenic, and that a large majority of adults harboured antibodies to each of them. A more detailed analysis of MSP3.3, MSP3.4, MSP3.7 and MSP3.8, performed using for each of them six overlapping peptides covering their C-terminal part, showed in addition that each of these 24 peptides was antigenic, in accordance with what had previously been observed for the 12 peptides from MSP3.1 and MSP3.2. This indicates that each synthetic peptide defines at least one B-cell epitope properly conformed. The recognition of the parasite native protein by antipeptides antibodies, demonstrated by IFA assays, and indirectly confirmed by ADCI assays, is an additional proof of the relevance of peptides to the native parasite epitopes.

Besides their overall structural homology, the six C-terminal MSP3 proteins share highly homologous sequences displayed on peptides "b", "d" (and "f"). The sequence "ILGWEFGGGAP" from peptides "b" is well conserved among MSP3.1, MSP3.2 and MSP3.7, and was therefore found to be targeted by antibodies that are fully cross-reactive to these 3 antigens. The antibodies directed to the peptides "d" presented a broader range of cross-reactivities on each of the six MSP3 proteins, consistent with the occurrence of single point amino acid changes among the homologous sequences. In addition to the resulting arithmetic increase in the number of targets for the ADCI-effective antibodies, a malaria vaccine combining these antigens would thus presumably have the advantage to broaden the immune responses elicited in humans to these epitopes.

Twenty of the twenty-four peptides analysed displayed an IgG subclass pattern with dominant cytophilic responses. All twenty-four peptides were found to define antigenic determinants able to trigger the antiparasitic ADCI mechanism. The biological activity of affinity-purified antibodies upon these peptides was found for more than half of them to be as much or more effective against *P.falciparum* in ADCI as total IgG from protected African adults. Therefore they constitute a large series of novel valuable targets able to trigger the ADCI mechanism in addition to the nine targets previously described in the other two MSP3 family (Singh, et al., 2004; Singh, et al., 2005), and also add to epitopes identified in unrelated proteins such as in GLURP, SERP ( Soe et al., 2002; Theisen et al., 2000) and recently in MSP1-Block2.

These findings bring new perspectives on the implication of MSP3 antigens in the natural host-parasite interaction suggesting that they have the function to maintain the homeostasis between *P. falciparum* and human beings, by contributing to control parasite densities.

Our findings have also practical consequences for vaccine development. Indeed, they pave the way towards the rational design of a multigenic vaccine construct based on a family of proteins having both homologous and divergent sequences that are highly conserved across isolates. This vaccine combination strategy markedly differs in its concept from constructs combining different and polymorphic antigens, such as AMA1, RESA, MSP1 and MSP2 ( Fluck et al., 2007; Genton et al., 2002; Hu et al., 2008; Malkin et al., 2008; Miller, et al., 1993; Polley et al., 2003; Takala et al., 2007). A multigenic vaccine based on combinations of the C-terminus of the MSP3 proteins has in addition the major benefit to circumvent the polymorphism problem, which is a recognized bottleneck for vaccine development (Genton and Reed, 2007; Lalitha et al., 1999; Pan et al., 2004; Stowers et al., 2001), since their sequences are highly conserved.

However antigenicity data have also to be combined with results from immunogenicty studies reported in example 1, which suggest that some regions are involved in the regulation of the intensity of immune responses. This was expectable. Indeed, one of the remarkable characteristics of malaria infection in African children is the overwhelming number of parasites children harbour over childhood and adolescence. These high parasite loads corresponds to outstandingly high amounts of malarial antigens, which stimulate daily the immune system for years. Since the antibody titres to MSP3 and other antigens, observed in children as in adults, remain very moderate, it implies that malarial antigens must contain regulatory regions to prevent the induction of high immune responses that could otherwise eradicate the parasite. Such regions were identified in the immunogenicity study (example 1) where it was found that removing the "e-f" region from MSP3.1 resulted in an increase by two orders of magnitude of antibody responses to the ADCI-relevant "b-d" region. In this manner artificial immunisation may potentially become more effective than natural exposure to the parasite.

Our first multigenic construct therefore includes only the regions "b-d" of the six MSP3 antigens. The resulting multi-component fusion antigen was shown to have strong immunogenicity in mice, inducing biologically active and cross-reactive antibodies of various avidities upon the 6 antigens of the MSP3 family (the present study, and example 1).

### Example 3: Combinations of MSP3 antigens in polyprotein constructs for a subunit vaccine against malaria - Immunogenicity studies of 8 new constructs

### 3. A. Materials and methods

### Recombinant proteins production

The nucleic acid sequences coding for the MSP3 polypeptides were chemically synthesised (GenScript, USA) and are provided in figure 18A to H along with their corresponding amino acid (AA) sequences. For comprehensive reasons, the sequences are colour-coded in function of the molecule of origin as red for MSP3-1, light green for MSP3-2, orange for MSP3-3, brown for MSP3-4, blue for MSP3-7 and green for MSP3-8. The sequences coding for the C-terminal histidine tag and the coil-coiled linkers between the MSP3 moieties in some polypeptides constructions (figure 18C and D) are left in black. The amino acid sequences included in the constructions comprise AA 184 to 251 in MSP3-1 (PlasmoDB Acc. number; PF10_0345), AA 161 to 257 in MSP3-2 (PlasmoDB Acc. number; PF10_0346), AA 228 to 307 in MSP3-3 (PlasmoDB Acc. number; PF10_0347), AA 508 to 579 in MSP3-4 (PlasmoDB Acc. number; PF10_0348), AA 214 to 285 in MSP3-7 (PlasmoDB Acc. number; PF10_0352), AA 537 to 624 in MSP3-8 (PlasmoDB Acc. number; PF10_0355). The sequence corresponding to MSP3-2 in the last construction (figure 18H) was modified to shorten a glutamic acid-rich region embedded between peptides d and e, by linking the regions corresponding to peptides a,b,c and d (AA 161 to 257) to peptides d and f (AA 293 to 371) (Table 2). The sequences corresponding to the coil-coiled peptide linkers are derived from the molecules Ag27 (figure 18C) (AA 845 to 871) (PlasmoDB Acc. number; PFF0165c) and a combination of tetrapeptide repeated units from LSA3 in the region comprised between AA 223 to AA 586 (figure 18D) (PlasmoDB Acc. number; PFB0915w).

These nucleotide sequences were incorporated in the prokaryotic expression plasmid vector pTcrHis2A (Invitrogen) at the Bgl II restriction site. Chemically competent Escherichia coli bacteria (strain BL21 DE3) (Invitrogen) were transformed with the corresponding plasmids under selective culture conditions (Luria Bertani medium with 100µg/ml ampicillin).

The production of recombinant protein was induced by the adjunction of 1 mM of IPTG in bacteria culture reaching an optical density of 0,6 measured at 600 nm. Recombinant proteins were purified by affinity of the histidine tag for Ni ions fixed on agarose beads according to the manufacturer instructions (Qiagen). The purified proteins were further cleared of bacterial endotoxin with an endotoxin removing gel kit (Thermo scientific). The recombinant proteins were stored at minus 20°C in endotoxin-free phosphate buffered saline until use.

### Animals and immunisation

Four weeks old female mice of two inbred strains, C57BL/6 (H-2b) and BALB/c (H-2d) and one outbred strain, Swiss, were obtained from Elevage Janvier (Le Genest Saint Isle, France). The animals were handled according to the Institut Pasteur guidelines. They were immunized at three weeks intervals by six subcutaneous injections at the base of the tail with 20 µg of *Escherichia coli* recombinant polypeptides emulsified in montanide ISA 720 (Seppic, France) at a ratio of three parts of antigen for seven parts of montanide in a volume of 100 µL. The immune sera were collected by tail bleeding two weeks after the second up to the sixth injections and kept at - 20°C until use.

### Western blotting

Total blood stage parasite material was obtained from a culture of *P. falciparum* strain 3D7 on human erythrocytes and utilised as a source of antigen for the Western blot assay. The percentage of infected erythrocytes was elevated to 70% by centrifugation through a plasmagel gradient. The proteins corresponding to 60 × 106 erythrocytes were separated by electrophoresis in a 75 × 1 mm well of a 10% acrylamide gel under denaturing conditions. The proteins were electro transferred on a membrane of nitrocellulose (Amersham Biosciences). The membrane was saturated in PBS with 5% of skimmed milk 15 minutes at room temperature and cut into two to three millimetres large bands across the migration front. Each band was incubated with a mix of immunesera from animal groups immunised by the same polyprotein construction (n=7) at a 1:1000 dilution in PBS / 5% skimmed milk one hour at room temperature on a rocking tray. In competition experiments with soluble antigens, the first antibodies were preincubated one hour at room temperature with antigens at a concentration of 2 µg / mL before the incubation with the nitrocellulose membrane. After three washes in PBS, the bands were incubated with the secondary antibody, a goat anti-mouse IgG conjugated with alkaline phosphatase (GAMAP) at a dilution of 1:7500 (Promega, USA). After three washes in PBS, the bands were incubated in BCIP/NBT color substrate solution (Promega, USA) according to the manufacturer instructions. Uninfected erythrocytes and sera from naïve animals were used for controls.

### 3. B. Results

*P. falciparum* MSP3-1 protein is the target of cytophilic subclasses of immunoglobulins (IgG1 and IgG3 in humans and IgG2a and IgG2b in mice) that inhibit the asexual blood stages development in association with monocytes (Antibody Cellular Dependant Inhibition (ACDI); Khusmith and Druilhe, 1983; Lunel and Druilhe, 1989; Bouharoun-Tayoun et al., 1990; Jafarshad et al., 2007). In addition, specific anti-MSP3-1 antibodies are more effective than total IgG from African hyperimmune sera at clearing a *P.falciparum* parasitemia in a humanised model of malaria in immunocompromised mice (Badell et al., 2000).

Six members of the MSP3 family on the chromosome 10, MSP3-1, MSP3-2, MSP3-3, MSP3-4, MSP3-7 and MSP3-8, share a similar C-terminal extremity organisation (Singh et al., 2004). Moreover, these proteins show very limited polymorphism.

Family members of *P. falciparum* MSP3 antigens trigger a network of cross-reactive antibodies involved in protective ADCI responses against the asexual blood stages of the parasite. In addition, as for MSP3-1, the C-terminal extremities of the molecules are conserved in different clinical isolates.

The aim of theis study was to develop a multigenic vaccine in order to:
- take advantage of the network of protective cross-reactive antibody response against the structurally related *P. falciparum* MSP3 antigens along with their genetic stability;
- minimize the apparition of parasite variants following the vaccination with a subunit vaccine containing a limited number of protective epitopes (peptides or single recombinant antigens);
- increase the potency of the vaccine in a wide range of individuals with different immunogenetic backgrounds; and
- choose the vaccine formulation that elicits an optimal immune response, i.e. an immune response which is well balanced toward each gene, well balanced in mice with different genetic backgrounds, and constituted of dominant cytophilic classes.

A third generation of MSP3-based vaccines was generated, which is multiepitopic, immunogenic, and stable : novel vaccine constructs derived from the MSP3 gene family were designed, based on the data on antigenicity and immunogenicity shown in example 1 and 2; the detailed analysis of the antigenicity in humans exposed from endemic areas of six proteins belonging to the MSP3 family (MSP3-1, MSP3-2, MSP3-3, MSP3-4, MSP3-7 and MSP3-8), using six recombinant proteins, as well as immunogenicity studies with various constructs derived from the MSP-1 protein, led us to design chimeric polypeptides.

Height different polyproteins consisting of particular motifs derived from one or several *P. falciparum* MSP3 proteins were designed (see figures 16-18): one monovalent, one bivalent, one trivalent, two quadrivalent and one hexavalent multigene constructs, as well as two quadrivalent constructs which incorporate regions from the LSA3 vaccine candidate and the p27 vaccine candidate, using regions that form coiled-coiled structures in the protein from which they derive, so as to mimic the coiled region from the MSP3 vaccines.

### Production of the multigene constructs in E. Coli

The recombinant polypeptides were produced in the BL21 strain of *E. Coli.* 1 µg of each of these recombinant polypeptides was then run on SDS Page 10%.

As shown in figure 19, the height recombinant constructs could be expressed, produced and purified from *E*. *coli.* Thus expressed and purified recombinant polypeptides retain their antigenicity. Indeed, figure 20 shows that these recombinant polypeptides are recognized by a rat immune serum directed against the C-terminal region of MSP3-1.

### Immunogenicity studies

Immune responses were analysed in experimentals models with different genetic backgrounds; two inbred mice strains (BALB/c (H-2^{d}) and C57/BL6 (H-2^{b})) and one outbred mice strain (Swiss) were used.

For each of the 8 recombinant polypeptides, 7 mice of each of the 3 strains were immunized. The schedule of immunizations and samples harvesting is illustrated on figure 21.

### Western blotting analysis on the parasite blood stages

Red blood cells infected with the 3D7 strain of *P. falciparum* were analyzed by western blot using pools of immune sera harvested from the three strains of mice (BALB/c(H-2d), C57/BL6 (H-2b) and Swiss) immunized with the MSP3 recombinant polypeptides after the second injection. As shown in figure 22, all the recombinant polypeptides induced a similar immune response in the three strains of mice and the induced antibodies that are able to recognize the native proteins in the three strains of mice.

The kinetic of the immune response in mice immunised with the 8 MSP3 recombinant polypeptides was then analyzed *by Western blotting* on *P. falciparum* (3D7 strain). Infected red blood cells were analyzed after the second, the third and the fourth injections. As shown in figure 23, kinetic of the antibody response was stable after the second injection.

A *Western blot* analysis was also performed on red blood cells infected with *P. falciparum* (3D7) using pools of immune sera harvested from BALB/c mice after the sixth immunization; competition with single antigens (the C-terminal end of MSP3-1, MSP3-2, MSP3-3, MSP3-4, MSP3-7 and MSP3-8) was assessed. As shown in figure 24, the recognition profile is different depending on the construct which was used for immunization. This suggests participation of cross-reacting antibodies.

### ELISA analysis - analysis of the antibodies isotypes

An ELISA analysis was performed to evaluate the antibody response against 6 MSP3 family members (MSP3, MSP3-2, MSP3-3, MSP3-4, MSP3-7 and MSP3-8) after immunization of the 3 mice strains with the multigenics vaccines constructs shown in figures 16-18. Total IgG and IgM responses were measured after the third injection, as well as the profile of IgG subclasses responses (IgG1, IgG2a, IgG2b, IgG3).

Three strains with different genetic background *(Balbc, SWISS, C57BL*/*6)* were used. 56 mices per strain were dispatched in 8 groups of 7 mices. Each group of mice was immunized with one immunogen (one of the multigenic constructs). Sampling was performed every 19 days after the third immunization. The sera were diluted at 1/1000 *(i.e., 56x3= 168 sera).* The immune response was then analysed against the C-terminal end of each of the 6 members of MSP3 family: MSP3, MSP3-2, MSP3-3, MSP3-4, MSP3-7 and MSP3-8 or against p27 or LSA-3 (see figures 28-36).

Figure 28 shows that there are few differences in total IgG response between the 3 mice strains (except for the C-terminal end of MSP3-7 in C57BL/6) and a good immunogenicity of each of the 8 MSP3-polyproteins constructs in the 3 strains of mice. In addition, the immunogenicity is improved compared to the first MSP3 polyprotein construct (the poly-bcd construct used in examples 1 and 2, of sequence SEQ ID NO: 71). Furthermore, a good tolerance of the MSP3-polyproteins constructs adjuvanted with this formulation was observed (no Delayed Type Hypersensitivity signs or undesirable reactions observed in the 3 mice strains).

However, a good immunogenicity is not enough; what matters most is not the quantity of the antibody response but its quality:
- According to epidemiological data, a positive correlation has been made between the IgG3 levels against MSP3-1 and a long term protection to *falciparum* Malaria (Roussilhon *et al.,* 2007);
- Implication of cytophilics antibodies (IgG1 and IgG3) have been shown in the reduction of parasite growth through a mechanism involving their cooperation with blood monocytes observed in both *in vitro* models (Bouharoun-Tayoun et *al*., 1995; Jafarshad *et al.,* 2007) and *in vivo* model (Badell *et al.,* 2000);
- One mean to evaluate the quality of this response is the cytophilicity ratio. In humans, this ratio is measured by: (IgG1 + IgG3) / (IgG2 + IgG4 + IgM). In mice, this ratio is measured by: (IgG2a/c + IgG2b) / (IgG1 + IgG3 + lgm).

Figure 29 shows that IgG1 and IgM responses are homogenous between each of the three strains of mice.

In addition, as shown on figure 31, with respect to IgG2a responses, in Balbc and SWISS mice, immunization with MSP3 polyproteins constructs adjuvanted in Montanide induced high levels of IgG2a against the C-terminal end of six members of the MSP3 family. In contrast, in C57BL/6 mice, general low levels of IgG2a were induced against these MSP3-CT related antigens (see figure 31A). This is likely because mice strains with B6 background lack the allele Igh-1a enabling the expression of IgG2a and therefore these mice strains express rather IgG2c encoded by Igh-1b allele (Martin *et al.,* 1997 ; Martin *et al.,* 1998). With respect to IgG2b responses, there is indirect evidence of this genetic polymorphism bias over the antibody response because the pattern of the IgG2b subclass is similar between the 3 mice strains (see figure 31B). This is likely because this allele expressing this subclass is identically induced in the 3 strains of mice. It seems that the MSP3 polyproteins constructs more strongly induce an IgG2b subclass response directed against some of the antigens (i.e., against the C-terminal end of MSP3-1, -2 and -3 in C57BL/6 mice and against the C-terminal end of MSP3-1, -2, -3 and -7 in Balbc and Swiss mice). The vaccine constructs elicited very low IgG2b responses directed against the C-terminal end of MSP3-4 or MSP3-8 in the three strains of mice or against the C-terminal end of MSP3-7 in C57BL/6 mice.

Altogether these data suggest combined influences upon the cytophilic IgG pattern of a) the genetic background of the host, b) the nature of the immunogen(s) and c) the target antigen(s). In addition, some constructs produced more homogeneous results in different strains.

### 3. C. Conclusion

The results presented above show that each of the eight polypeptidic synthetic constructs are expressed in *Escherichia coli.*

Very detailed immunogenicity studies performed in 3 species of rodents indicates a satisfactory immunogenicity of each of these eight novel constructs, with an overall dominance of the cytophilic classes IgG2a and IgG2b in rodents, which cytophilic classes are efficient in the monocyte-dependent antibody-mediated effect of the ADCI mechanism effective against *P. Falciparum* asexual blood stages. The eight constructs are able to induce a similar antibody response in the three strains of mice, which response is mainly directed against MSP3-1 and MSP3-2. In addition, the immunogenicity is improved compared to the one obtained with the poly-bcd construct disclosed in examples 1 and 2.

The high levels of IgG2a elicited are particularly valuable in view of a likely Th-1 response driven by IFN-γ production and in view of the capacity of IFN-γ to enhance on the monocytes expression of some rFc-γ (analogous between mice and human) able to bind the IgG2a subclass (Snapper *et al.,* 1987).

Four constructs appeared, at first glance, as having possibly better features based on results from preclinical experimental models, namely: MSP3-1-2 (construct 7; SEQ ID NOs: 67 and 68), MSP3-1-2-3-8 (construct 5; SEQ ID NOs: 63 and 64), MSP3-1-2-4-7 (construct 2; SEQ ID NO: 57 and 58), and MSP3-1-2-3-4-7-8 (construct 1; SEQ ID NOs: 55 and 56).

In addition, p27 and LSA3 repeat peptides generate antibodies against specific proteins in asexual blood stages.

The magnitude of the antibody response is stable even after up to five successive boosts, in contrast to what was observed in the case of immunization with the full length C-terminal region of MSP3.1.

These combined vaccine candidates have several attractive features: they combine highly conserved B-lymphocyte targets of effective antibodies in the ADCI assays, T-helper epitopes from each of the 6 genes that ensure a better and more consistent immune response on various human genetic backgrounds (as a vaccine would have to be effective in European, Asian as well as African populations with various HLA molecules), they are fully conserved across different *P. Falciparum* isolates, and they proved to be safe and highly immunogenic in preclinical models. Moreover, the antibodies elicited by those constructs display a large network of cross-reactivity across all 6 members of the MSP3 family. The simultaneous targeting of 6 different proteins, each simultaneously expressed by each parasite, is an important progress as compared to previous single gene constructs.

### Example 4: analysis of the in vitro inhibitory effect upon the parasite growth of mice sera in presence of blood monocytes.

Antibody dependent cellular inhibition (ADCI) assays were performed essentially as described previously (Bouharoun-Tayoun *et al.,* 1990; Druilhe and Bouharoun-Tayoun, 2002a; Jafarshad *et al.,* 2007) and as described herein (see *"Functional ADCI assays",* pages 47-48).

### Example 5: evaluation of the induction of T cells responses to MSP3-polyproteins by assaying the spleen cells cytokine profile (IFN-γ, TNF-α, IL-10, IL-4) after immunization.

Cytokine quantification was analysed as described previously (for example in Boeuf *et al.,* 2005) and as described herein (see page 32, first paragraph).

### REFERENCES

Audran, R., M. Cachat, F. Lurati, S. Soe, O. Leroy, G. Corradin, P. Druilhe, and F. Spertini. 2005. Phase I malaria vaccine trial with a long synthetic peptide derived from the merozoite surface protein 3 antigen. Infect Immun 73:8017-8026.
Badell, E., C. Oeuvray, et al. 2000. Human malaria in immunocompromised mice: an in vivo model to study defense mechanisms against Plasmodium falciparum. J Exp Med 192(11): 1653-60.
BenMohamed, L., Y. Belkaid, et al. 2002. Systemic immune responses induced by mucosal administration of lipopeptides without adjuvant. Eur J Immunol 32(8): 2274-81.
Boeuf P, Vigan-Womas I, Jublot D, Loizon S, Barale JC, Akanmori BD, Mercereau-Puijalon O, Behr C. 2005. CyProQuant-PCR: a real time RT-PCR technique for profiling human cytokines, based on external RNA standards, readily automatable for clinical use. BMC Immunology. 6(1):5. doi: 10.1186/1471-2172-6-5.
Bottius, E., L. BenMohamed, K. Brahimi, H. Gras, J. P. Lepers, L. Raharimalala, M. Aikawa, J. Meis, B. Slierendregt, A. Tartar, A. Thomas, and P. Druilhe. 1996. A novel Plasmodium falciparum sporozoite and liver stage antigen (SALSA) defines major B, T helper, and CTL epitopes. J Immunol 156:2874-2884.
Bouharoun-Tayoun, H., P. Attanath, A. Sabchareon, T. Chongsuphajaisiddhi, and P. Druilhe. 1990. Antibodies that protect humans against Plasmodium falciparum blood stages do not on their own inhibit parasite growth and invasion in vitro, but act in cooperation with monocytes. The Journal of experimental medicine 172:1633-1641.
Bouharoun-Tayoun, H., and P. Druilhe. 1992a. Plasmodium falciparum malaria: evidence for an isotype imbalance which may be responsible for delayed acquisition of protective immunity. Infection and immunity 60:1473-1481.
Bouharoun-Tayoun, H., and P. Druilhe. 1992b. Antibodies in falciparum malaria: what matters most, quantity or quality? Mem Inst Oswaldo Cruz 87 Suppl 3:229-234.
Bouharoun-Tayoun, H., C. Oeuvray, F. Lunel, and P. Druilhe. 1995. Mechanisms underlying the monocyte-mediated antibody-dependent killing of Plasmodium falciparum asexual blood stages. The Journal of experimental medicine 182:409-418.
Brahimi, K., J. L. Perignon, M. Bossus, H. Gras, A. Tartar, and P. Druilhe. 1993. Fast immunopurification of small amounts of specific antibodies on peptides bound to ELISA plates. Journal of immunological methods 162:69-75.
Burgess, B. R., P. Schuck, and D. N. Garboczi. 2005. Dissection of merozoite surface protein 3, a representative of a family of Plasmodium falciparum surface proteins, reveals an oligomeric and highly elongated molecule. J Biol Chem 280:37236-37245.
Carvalho LJ, Oliveira SG, Theisen M, Alves FA, Andrade MC, Zanini GM, Brigido MC, Oeuvray C, Póvoa MM, Muniz JA, Druilhe P, Daniel-Ribeiro CT. 2004. Immunization of Saimiri sciureus Monkeys with Plasmodium falciparum Merozoite Surface Protein-3 and Glutamate-Rich Protein Suggests that Protection is Related to Antibody Levels. Scandinavian Journal of Immunology.Volume 59, Issue 4, Pages 363-372.
Dame, J. B., J. L. Williams, et al. 1984. Structure of the gene encoding the immunodominant surface antigen on the sporozoite of the human malaria parasite Plasmodium falciparum. Science 225(4662): 593-9.
Daubersies, P., A. W. Thomas, et al. 2000. Protection against Plasmodium falciparum malaria in chimpanzees by immunization with the conserved preerythrocytic liver-stage antigen 3. Nat Med 6(11): 1258-63.
Druilhe, P., and H. Bouharoun-Tayoun. 2002a. Antibody-dependent cellular inhibition assay. Methods Mol Med 72:529-534.
Druilhe, P., and H. Bouharoun-Tayoun. 2002b. Human antibody subclass ELISA. Methods in molecular medicine 72:457-459.
Druilhe, P., F. Spertini, D. Soesoe, G. Corradin, P. Mejia, S. Singh, R. Audran, A. Bouzidi, C. Oeuvray, and C. Roussilhon. 2005. A malaria vaccine that elicits in humans antibodies able to kill Plasmodium falciparum. PLoS Med 2:e344.
Escalante, A. A., H. M. Grebert, et al. 2001. Polymorphism in the gene encoding the apical membrane antigen-1 (AMA-1) of Plasmodium falciparum. X. Asembo Bay Cohort Project. Mol Biochem Parasitol 113(2): 279-87.
Fidock, D. A., E. Bottius, et al. 1994. Cloning and characterization of a novel Plasmodium falciparum sporozoite surface antigen, STARP. Mol Biochem Parasitol 64(2): 219-32.
Fluck, C., S. Schopflin, T. Smith, B. Genton, M. P. Alpers, H. P. Beck, and 1. Felger. 2007. Effect of the malaria vaccine Combination B on merozoite surface antigen 2 diversity. Infect Genet Evol 7:44-51.
Friguet, B., A. F. Chaffotte, L. Djavadi-Ohaniance, and M. E. Goldberg. 1995. Under proper experimental conditions the solid-phase antigen does not disrupt the liquid phase equilibrium when measuring dissociation constants by competition ELISA. Journal of immunological methods 182:145-150.
Garraud, O., S. Mahanty, and R. Perraut. 2003. Malaria-specific antibody subclasses in immune individuals: a key source of information for vaccine design. Trends in immunology 24:30-35.
Genton, B., I. Betuela, I. Felger, F. Al-Yaman, R. F. Anders, A. Saul, L. Rare, M. Baisor, K. Lorry, G. V. Brown, D. Pye, D. O. Irving, T. A. Smith, H. P. Beck, and M. P. Alpers. 2002. A recombinant blood-stage malaria vaccine reduces Plasmodium falciparum density and exerts selective pressure on parasite populations in a phase 1-2b trial in Papua New Guinea. The Journal of infectious diseases 185:820-827.
Genton, B., and Z. H. Reed. 2007. Asexual blood-stage malaria vaccine development: facing the challenges. Current opinion in infectious diseases 20:467-475.
Guerin-Marchand, C., P. Druilhe, et al. 1987. A liver-stage-specific antigen of Plasmodium falciparum characterized by gene cloning. Nature 329(6135): 164-7.
Hu, J., Z. Chen, J. Gu, M. Wan, Q. Shen, M. P. Kieny, J. He, Z. Li, Q. Zhang, Z. H. Reed, Y. Zhu, W. Li, Y. Cao, L. Qu, Z. Cao, Q. Wang, H. Liu, X. Pan, X. Huang, D. Zhang, X. Xue, and W. Pan. 2008. Safety and immunogenicity of a malaria vaccine, Plasmodium falciparum AMA-1/MSP-1 chimeric protein formulated in montanide ISA 720 in healthy adults. PLoS ONE 3:e1952.
Huber, W., I. Felger, H. Matile, H. J. Lipps, S. Steiger, and H. P. Beck. 1997. Limited sequence polymorphism in the Plasmodium falciparum merozoite surface protein 3. Mol Biochem Parasitol 87:231-234.
Jafarshad, A., M. H. Dziegiel, R. Lundquist, L. K. Nielsen, S. Singh, and P. L. Druilhe. 2007. A novel antibody-dependent cellular cytotoxicity mechanism involved in defense against malaria requires costimulation of monocytes FcgammaRll and FcgammaRlll. J Immunol 178:3099-3106.
Jensen, J. B. 2002. In vitro culture of Plasmodium parasites. Methods in molecular medicine 72:477-488.
Khusmith, S. and P. Druilhe. 1983. Cooperation between antibodies and monocytes that inhibit in vitro proliferation of Plasmodium falciparum. Infect Immun 41(1): 219-23.
Knapp, B., E. Hundt, et al. 1989. Molecular cloning, genomic structure and localization in a blood stage antigen of Plasmodium falciparum characterized by a serine stretch. Mol Biochem Parasitol 32(1): 73-83.
Lalitha, P. V., P. Malhotra, R. Chattopadhyay, and V. S. Chauhan. 1999. Plasmodium falciparum: variations in the C-terminal cysteine-rich region of the merozoite surface protein-1 in field samples among Indian isolates. Experimental parasitology 92:12-18.
Lunel, F. and P. Druilhe. 1989. Effector cells involved in nonspecific and antibody-dependent mechanisms directed against Plasmodium falciparum blood stages in vitro. Infect Immun 57(7): 2043-9.
Lupas, A. 1996. Coiled coils: new structures and new functions. TIBS 21 (10):375-382.
Malkin, E., J. Hu, Z. Li, Z. Chen, X. Bi, Z. Reed, F. Dubovsky, J. Liu, Q. Wang, X. Pan, T. Chen, B. Giersing, Y. Xu, X. Kang, J. Gu, Q. Shen, K. Tucker, E. Tierney, W. Pan, C. Long, and Z. Cao. 2008. A Phase 1 trial of PfCP2.9: An AMA1/MSP1 chimeric recombinant protein vaccine for Plasmodium falciparum malaria. Vaccine.
Marshall, V. M., W. Tieqiao, et al. 1998. Close linkage of three merozoite surface protein genes on chromosome 2 of Plasmodium falciparum. Mol Biochem Parasitol 94(1): 13-25.
Martin RM, Silva A, Lew AM. 1997. The lgh-1 sequence of the non-obese diabetic (NOD) mouse assigns it to the IgG2c isotype. Immunogenetics. 46(2):167-8.
Martin, RM, Brady, JL., Lew AM. 1998. The need for IgG2c specific antiserum when isotyping antibodies from C57BL/6 and NOD mice. Journal of Immunological Methods, Volume 212, Issue 2; Pages 187-192.
McColl, D. J., and R. F. Anders. 1997. Conservation of structural motifs and antigenic diversity in the Plasmodium falciparum merozoite surface protein-3 (MSP-3). Mol Biochem Parasitol 90:21-31.
Miller, L. H., T. Roberts, M. Shahabuddin, and T. F. McCutchan. 1993. Analysis of sequence diversity in the Plasmodium falciparum merozoite surface protein-1 (MSP-1). Molecular and biochemical parasitology 59:1-14.
Mirano-Bascos, D., M. Tary-Lehmann, and S. J. Landry. 2008. Antigen structure influences helper T-cell epitope dominance in the human immune response to HIV envelope glycoprotein gp120. Eur J Immunol 38:1231-1237.
Moorthy, V. S., M. F. Good, and A. V. Hill. 2004. Malaria vaccine developments. Lancet 363:150-156.
Mulhern, T. D., G. J. Howlett, G. E. Reid, R. J. Simpson, D. J. McColl, R. F. Anders, and R. S. Norton. 1995. Solution structure of a polypeptide containing four heptad repeat units from a merozoite surface antigen of Plasmodium falciparum. Biochemistry 34:3479-3491.
Nayak, B. P. 1999. Differential sensitivities of primary and secondary T cell responses to antigen structure. FEBS Lett 443:159-162.
Oakley, MG, Hollenbeck, JJ. 2001. The design of antiparallel coiled coils. Curr Opin Struct Biol. 11 (4):450-457.
Oeuvray, C., H. Bouharoun-Tayoun, H. Gras-Masse, E. Bottius, T. Kaidoh, M. Aikawa, M. C. Filgueira, A. Tartar, and P. Druilhe. 1994. Merozoite surface protein-3: a malaria protein inducing antibodies that promote Plasmodium falciparum killing by cooperation with blood monocytes. Blood 84:1594-1602.
Pan, W., D. Huang, Q. Zhang, L. Qu, D. Zhang, X. Zhang, X. Xue, and F. Qian. 2004. Fusion of two malaria vaccine candidate antigens enhances product yield, immunogenicity, and antibody-mediated inhibition of parasite growth in vitro. J Immunol 172:6167-6174.
Peterson, M. G., V. M. Marshall, et al. 1989. Integral membrane protein located in the apical complex of Plasmodium falciparum. Mol Cell Biol 9(7): 3151-4.
Pleass, R. J. and J. M. Woof. 2001. Fc receptors and immunity to parasites. Trends Parasitol 17(11): 545-51.
Polley, S. D., W. Chokejindachai, and D. J. Conway. 2003. Allele frequency-based analyses robustly map sequence sites under balancing selection in a malaria vaccine candidate antigen. Genetics 165:555-561.
Polley, S. D., K. K. Tetteh, J. M. Lloyd, O. J. Akpogheneta, B. M. Greenwood, K. A. Bojang, and D. J. Conway. 2007. Plasmodium falciparum merozoite surface protein 3 is a target of allele-specific immunity and alleles are maintained by natural selection. The Journal of infectious diseases 195:279-287.
Pullen, G. R., M. G. Fitzgerald, and C. S. Hosking. 1986. Antibody avidity determination by ELISA using thiocyanate elution. Journal of immunological methods 86:83-87.
Reichelt, P., C. Schwarz, and M. Donzeau. 2006. Single step protocol to purify recombinant proteins with low endotoxin contents. Protein Expr Purif 46:483-488.
Robson, K. J., J. R. Hall, et al. 1988. A highly conserved amino-acid sequence in thrombospondin, properdin and in proteins from sporozoites and blood stages of a human malaria parasite. Nature 335(6185): 79-82.
Roggero, M. A., C. Servis, and G. Corradin. 1997. A simple and rapid procedure for the purification of synthetic polypeptides by a combination of affinity chromatography and methionine chemistry. FEBS Lett 408:285-288.
Roussilhon, C., C. Oeuvray, C. Muller-Graf, A. Tall, C. Rogier, J. F. Trape, M. Theisen, A. Balde, J. L. Perignon, and P. Druilhe. 2007. Longterm clinical protection from falciparum malaria is strongly associated with IgG3 antibodies to merozoite surface protein 3. PLoS Med 4:e320.
Sabchareon, A., T. Burnouf, D. Ouattara, P. Attanath, H. Bouharoun-Tayoun, P. Chantavanich, C. Foucault, T. Chongsuphajaisiddhi, and P. Druilhe. 1991. Parasitologic and clinical human response to immunoglobulin administration in falciparum malaria. The American journal of tropical medicine and hygiene 45:297-308.
Simmons, D., G. Woollett, et al. 1987. A malaria protein exported into a new compartment within the host erythrocyte. Embo J 6(2): 485-91.
Singh, S. 2004. Caractérisation des réponses immunes protectrices dirigées contre les protéines de la famille MSP3 et d'autres antigènes de Plasmodium falciparum. PhD thesis. 292p. University of Rouen, France.
Singh, S., S. Soe, J. P. Mejia, C. Roussilhon, M. Theisen, G. Corradin, and P. Druilhe. 2004. Identification of a conserved region of Plasmodium falciparum MSP3 targeted by biologically active antibodies to improve vaccine design. The Journal of infectious diseases 190:1010-1018.
Singh, S., S. Soe, C. Roussilhon, G. Corradin, and P. Druilhe. 2005. Plasmodium falciparum merozoite surface protein 6 displays multiple targets for naturally occurring antibodies that mediate monocyte-dependent parasite killing. Infect Immun 73:1235-1238.
Snapper CM, Paul WE. 1987. Interferon-gamma and B cell stimulatory factor-1 reciprocally regulate lg isotype production. Science. 236(4804):944-7.
Soe, S., A. Khin Saw, et al. 2001. Premunition against Plasmodium falciparum in a malaria hyperendemic village in Myanmar. Trans R Soc Trop Med Hyg 95(1): 81-4.
Soe, S., S. Singh, D. Camus, T. Horii, and P. Druilhe. 2002. Plasmodium falciparum serine repeat protein, a new target of monocyte-dependent antibody-mediated parasite killing. Infection and immunity 70:7182-7184.
Soe, S., M. Theisen, C. Roussilhon, K. S. Aye, and P. Druilhe. 2004. Association between protection against clinical malaria and antibodies to merozoite surface antigens in an area of hyperendemicity in Myanmar: complementarity between responses to merozoite surface protein 3 and the 220-kilodalton glutamate-rich protein. Infect Immun 72:247-252.
Stowers, A. W., V. Cioce, R. L. Shimp, M. Lawson, G. Hui, O. Muratova, D. C. Kaslow, R. Robinson, C. A. Long, and L. H. Miller. 2001. Efficacy of two alternate vaccines based on Plasmodium falciparum merozoite surface protein 1 in an Aotus challenge trial. Infection and immunity 69:1536-1546.
Surman, S., T. D. Lockey, K. S. Slobod, B. Jones, J. M. Riberdy, S. W. White, P. C. Doherty, and J. L. Hurwitz. 2001. Localization of CD4+ T cell epitope hotspots to exposed strands of HIV envelope glycoprotein suggests structural influences on antigen processing. Proc Natl Acad Sci U S A 98:4587-4592.
Takala, S. L., D. Coulibaly, M. A. Thera, A. Dicko, D. L. Smith, A. B. Guindo, A. K. Kone, K. Traore, A. Ouattara, A. A. Djimde, P. S. Sehdev, K. E. Lyke, D. A. Diallo, O. K. Doumbo, and C. V. Plowe. 2007. Dynamics of polymorphism in a malaria vaccine antigen at a vaccine-testing site in Mali. PLoS medicine 4:e93.
Theisen, M., J. Vuust, A. Gottschau, S. Jepsen, and B. Hogh. 1995. Antigenicity and immunogenicity of recombinant glutamate-rich protein of Plasmodium falciparum expressed in Escherichia coli. Clin Diagn Lab Immunol 2:30-34.
Theisen, M., S. Soe, et al. 1998. The glutamate-rich protein (GLURP) of Plasmodium falciparum is a target for antibody-dependent monocyte-mediated inhibition of parasite growth in vitro. Infect Immun 66(1): 11-7.
Theisen, M., S. Soe, S. G. Jessing, L. M. Okkels, S. Danielsen, C. Oeuvray, P. Druilhe, and S. Jepsen. 2000. Identification of a major B-cell epitope of the Plasmodium falciparum glutamate-rich protein (GLURP), targeted by human antibodies mediating parasite killing. Vaccine 19:204-212.
Theisen, M., D. Dodoo, et al. 2001. Selection of glutamate-rich protein long synthetic peptides for vaccine development: antigenicity and relationship with clinical protection and immunogenicity. Infect Immun 69(9): 5223-9.
Theisen, M., S. Soe, K. Brunstedt, F. Follmann, L. Bredmose, H. Israelsen, S. M. Madsen, and P. Druilhe. 2004. A Plasmodium falciparum GLURP-MSP3 chimeric protein; expression in Lactococcus lactis, immunogenicity and induction of biologically active antibodies. Vaccine 22:1188-1198.
Thomas, A. W., D. A. Carr, et al. 1990. Sequence comparison of allelic forms of the Plasmodium falciparum merozoite surface antigen MSA2. Mol Biochem Parasitol 43(2): 211-20.
Trape, J. F., C. Rogier, L. Konate, N. Diagne, H. Bouganali, B. Canque, F. Legros, A. Badji, G. Ndiaye, P. Ndiaye, and et al. 1994. The Dielmo project: a longitudinal study of natural malaria infection and the mechanisms of protective immunity in a community living in a holoendemic area of Senegal. The American journal of tropical medicine and hygiene 51:123-137.
Trucco, C., D. Fernandez-Reyes, et al. 2001. The merozoite surface protein 6 gene codes for a 36 kDa protein associated with the Plasmodium falciparum merozoite surface protein-1 complex. Mol Biochem Parasitol 112(1): 91-101.

## Claims

1. A polypeptide, especially a chimeric polypeptide, which is not a *Plasmodium* MSP3 native protein, and which comprises or consists of at least one polypeptide fragment encompassing motifs a, b, c and d of the C-terminal region of a *Plasmodium* MSP3 protein chosen among MSP3-2, MSP3-3, MSP3-4, MSP3-7, and MSP3-8.

2. The polypeptide according to claim 1, which is a polyprotein and which further comprises a polypeptide fragment consisting of motifs b, c and d of the C-terminal region of a *Plasmodium* MSP3-1 protein, and in particular which further comprises the polypeptide of sequence SEQ ID NO: 38.

3. The polypeptide according to claim 1 or 2, wherein the polypeptide fragment or at least one of the polypeptide fragments encompassing motifs a, b, c and d of the C-terminal region of a *Plasmodium* MSP3 protein is a polypeptide fragment encompassing
- motifs a, b, c and d of the C-terminal region of a *Plasmodium* MSP3-2 protein, and in particular a polypeptide fragment encompassing sequence SEQ ID NO: 40, and,
- optionally, motif e or f or motifs e and f of the C-terminal region of a *Plasmodium* MSP3 protein chosen among MSP3-1, MSP3-2, MSP3-3, MSP3-4, MSP3-7, and MSP3-8.

4. The polypeptide according to any one of claims 1 to 3, which results from a combination, in a polyprotein of:
- at least two polypeptide fragments from the C-terminal region of a *Plasmodium* MSP-3 protein chosen among MSP3-2, MSP3-3, MSP3-4, MSP3-7 and MSP3-8, wherein each polypeptide fragment consists of the succession of motifs a, b, c, and d, and optionally,
- at the C-terminal end of one or more of said polypeptide fragments the succession of motifs e or e and f of a protein providing motifs a, b, c and d.

5. The polypeptide according to any one of claims 1 to 4, wherein motifs a, b, c, d, e and f, when present, are ordered as follows, from the N-terminal to the C-terminal end of the polypeptide: MSP3-1 (b - c - d - e - f) - MSP3-2 (a - b - c - d - e - f) - MSP3-3 (a - b - c - d - e - f) - MSP3-4 (a - b - c - d - e - f) - MSP3-7 (a - b - c - d - e - f) - MSP3-8 (a - b - c - d - e - f).

6. The polypeptide of claim 5, which is devoid from further antigenic and/or immunogenic fragments of MSP3 proteins of *Plasmodium falciparum.*

7. The polypeptide according to any one of claims 1 to 5, which further comprises one or several coil-coiled linker(s), in particular one or several linker(s) comprising or consisting of sequence SEQ ID NO: 52 or SEQ ID NO: 54.

8. The polypeptide according to any one of claims 1 to 7, which comprises or consists of a polypeptide chosen among:
- polypeptides wherein the motifs a, b, c and d are organized in a sequence chosen among sequence SEQ ID NO: 42, SEQ ID NO: 44, SEQ ID NO: 46, SEQ ID NO: 48 and SEQ ID NO: 50.
- polypeptides having an amino acid sequence chosen among sequence SEQ ID NO: 56, SEQ ID NO: 58, SEQ ID NO: 60, SEQ ID NO: 62, SEQ ID NO: 64, SEQ ID NO: 66, SEQ ID NO: 68 and SEQ ID NO: 70.

9. A polynucleotide comprising or consisting of a sequence coding for the polypeptide according to any one of claims 1 to 8.

10. The polynucleotide according to claim 9, **characterized in that** it comprises or consists in a sequence chosen in the group consisting of of SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67 and SEQ ID NO: 69.

11. A recombinant cloning and/or expression vector, comprising a polynucleotide insert consisting of a polynucleotide according to claim 9 or 10.

12. A recombinant host cell, in particular a recombinant bacterium, yeast, insect cell or mammalian cell, which is transformed by the recombinant cloning and/or expression of claim 11.

13. An immunogenic composition comprising as an immunogen one or several polypeptide(s) according to any of claims 1 to 8 or recombinant cloning and/or expression vector(s) according to claim 11, and optionally, one or several pharmaceutically acceptable carrier(s), vehicle(s), diluent(s), excipient(s) or adjuvant(s) or any combination thereof.

14. A vaccine against malaria comprising as an immunogen one or several polypeptide(s) according to any one of claims 1 to 8, in association with a suitable pharmaceutical vehicle.

15. A synthetic or recombinant antibody or fragment of antibody (antibodies) which cross-reacts with one or several polypeptide(s) according to any one of claims 1 to 8, and which mediates *Plasmodium* blood stage growth inhibition or killing, in the monocyte-dependent, antibody-mediated ADCI mechanism, under *in vitro* conditions.

16. A pool of antibodies or biologically active fragments of antibodies prepared directed against several polypeptides according to any one of claims 1 to 8.

17. A medicament for passive immunotherapy of malaria, comprising an antibody or a pool of antibodies according to claim 15 or 16.

18. A polypeptide according to claims 1 to 8, an expression vector according to claim 10 or a composition comprising an antibody or a pool of antibodies according to claim 15 or 16, for use for preventing and/or treating malaria.

19. An expression vector according to claim 11, for use for genetic immunisation against *Plasmodium falciparum.*

20. A method for the *in vitro* diagnosis of malaria in an individual likely to be infected by *Plasmodium falciparum,* which comprises:
a) the bringing of a biological sample from said individual into contact with
- either a polypeptide of any one of claims 1 to 8, under conditions enabling the formation of antigen/antibody complexes between said polypeptide and the antibodies possibly present in the biological sample;
- or antibodies according to claim 15 or 16, under conditions enabling the formation of antigen/antibody complexes between said antibodies and the antigens specific for *Plasmodium falciparum* possibly present in the biological sample; and
b) the *in vitro* detection of the antigen/antibody complexes possibly formed.
